# EUROPÄISCHE PATENTSCHRIFT

(11) **EP 1 392 693 B1**
(45) Veröffentlichungstag und Bekanntmachung des Hinweises auf die Patenterteilung: **07.02.2007**
(21) Anmeldenummer: 02732637.0
(22) Anmeldetag: 18.04.2002
(51) Int. Cl.: C07D 487/04, A01N 43/90

(54) **TRIAZOLOPYRIMIDINE FUNGIZIDE**
TRIAZOLOPYRIMIDINES FUNGICIDES
TRIAZOLOPYRIMIDINES FONGICIDES

(30) Priorität: 30.04.2001 DE 10121162
(43) Veröffentlichungstag der Anmeldung: 03.03.2004
(73) Patentinhaber: Bayer CropScience AG, 40789 Monheim (DE)
(72) Erfinder: GEBAUER, Olaf, 50737 Köln (DE); GREUL, Jörg, Nico, 42799 Leichlingen (DE); HEINEMANN, Ulrich, 42799 Leichlingen (DE); ELBE, Hans, Ludwig, 42329 Wuppertal (DE); KRÜGER, Bernd, Wieland, 51467 Bergisch Gladbach (DE); MAURER, Fritz, 40789 Monheim (DE); DUNKEL, Ralf, 40789 Monheim (DE); VOERSTE, Arnd, 50677 Köln (DE); EBBERT, Ronald, 40789 Monheim (DE); WACHENDORFF-NEUMANN, Ulrike, 56566 Neuwied (DE); KITAGAWA, Yoshinori, Tochigi, 321-4305 (JP); MAULER-MACHNIK, Astrid, 42799 Leichlingen (DE); KUCK, Karl, Heinz, 40764 Langenfeld (DE)
(86) Internationale Anmeldenummer: PCT/EP2002/004287
(87) Internationale Veröffentlichungsnummer: WO 2002/088126

(56) Entgegenhaltungen:
- EP-A- 0 550 113
- EP-A- 0 613 900
- EP-A- 0 834 513
- WO-A-00/09508
- WO-A-98/46607
- WO-A-98/46608
- FR-A- 2 765 875
- FR-A- 2 784 991
- GB-A- 2 355 261
- US-A- 5 612 345
- US-A- 5 854 252
- WERMUTH ET AL: "The Practise of Medicinal Chemistry" , PRACTICE OF MEDICINAL CHEMISTRY, XX, XX, PAGE(S) 203-237 XP002190259 das ganze Dokument

## Beschreibung

Die vorliegende Erfindung betrifft neue Triazolopyrimidine, mehrere Verfahren zu deren Herstellung und deren Verwendung zur Bekämpfung von unerwünschten Mikroorganismen. Die Erfindung betrifft außerdem neue Zwischenprodukte sowie Verfahren zu deren Herstellung.

Es ist bereits bekannt geworden, dass bestimmte Triazolopyrimidine fungizide Eigenschaften besitzen (vgl. EP-A 0 550 113, WO 94-20 501, EP-A 0 613 900, US-A 5 612 345, EP-A 0 834 513, WO 98-46 607 und WO 98-46 608). Die Wirksamkeit dieser Stoffe ist gut, lässt aber bei niedrigen Aufwandmengen in manchen Fällen zu wünschen übrig.

Es wurden nun Triazolopyrimidine der nachfolgenden Formel 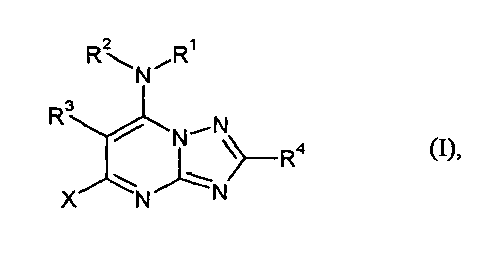 in welcher
- R¹: für Amino oder Hydroxy,
für gegebenenfalls durch Halogen, Cyano, Hydroxy, Amino, Phenyl, Heterocyclyl, Alkoxy mit 1 bis 4 Kohlenstoffatomen, Alkoxycarbonyl mit 1 bis 4 Kohlenstoffatomen, Alkylamino mit 1 bis 4 Kohlenstoffatomen, Dialkylamino mit 2 bis 8 Kohlenstoffatomen, Cycloalkyl mit 3 bis 6 Kohlenstoffatomen, Halogencycloalkyl mit 3 bis 6 Kohlenstoffatomen und 1 bis 5 Halogenatomen, Alkylthio mit 1 bis 4 Kohlenstoffatomen, Oxo, Hydroxyimino und/oder Alkoximino mit 1 bis 4 Kohlenstoffatomen substituiertes Alkyl mit 1 bis 6 Kohlenstoffatomen,
für gegebenenfalls durch Halogen, Cycloalkyl, Cyano, Phenyl und/oder Heterocyclyl substituiertes Alkenyl mit 2 bis 6 Kohlenstoffatomen,
für gegebenenfalls durch Halogen, Cycloalkyl, Cyano, Phenyl und/oder Heterocyclyl substituiertes Alkinyl mit 2 bis 6 Kohlenstoffatomen,
für gegebenenfalls durch Halogen, Cycloalkyl, Cyano, Halogenalkyl mit 1 bis 2 Kohlenstoffatomen und 1 bis 5 Halogenatomen, Phenyl und/oder Heterocyclyl substituiertes Cycloalkyl mit 3 bis 7 Kohlenstoffatomen,
für gegebenenfalls durch Halogen, Cycloalkyl, Cyano, Phenyl und/oder Heterocyclyl substituiertes Alkoxy mit 1 bis 7 Kohlenstoffatomen,
für gegebenenfalls durch Halogen, Cycloalkyl, Cyano, Phenyl und/oder Heterocyclyl substituiertes Alkenyloxy mit 2 bis 6 Kohlenstoffatomen,
für gegebenenfalls durch Halogen, Cycloalkyl, Cyano, Phenyl und/oder Heterocyclyl substituiertes Alkinyloxy mit 2 bis 6 Kohlenstoffatomen,
für gegebenenfalls durch Halogen, Cycloalkyl, Cyano, Phenyl und/oder Heterocyclyl substituiertes Cycloalkyloxy mit 3 bis 7 Kohlenstoffatomen,
für gegebenenfalls durch Halogen, Cycloalkyl, Cyano, Phenyl und/oder Heterocyclyl substituiertes Alkylamino mit 1 bis 7 Kohlenstoffatomen,
für gegebenenfalls durch Halogen, Cycloalkyl, Cyano, Phenyl und/oder Heterocyclyl substituiertes Dialkylamino mit 1 bis 7 Kohlenstoffatomen in jedem der Alkylreste,
für gegebenenfalls durch Halogen, Cycloalkyl, Cyano, Phenyl und/oder Heterocyclyl substituiertes Alkenylamino mit 2 bis 6 Kohlenstoffatomen,
für gegebenenfalls durch Halogen, Cycloalkyl, Cyano, Phenyl und/oder Heterocyclyl substituiertes Alkinylamino mit 2 bis 6 Kohlenstoffatomen,
für gegebenenfalls durch Halogen, Cycloalkyl, Cyano, Phenyl und/oder Heterocyclyl substituiertes Cycloalkylamino mit 3 bis 7 Kohlenstoffatomen,
für gegebenenfalls durch Halogen, Cyano, Phenyl und/oder Heterocyclyl substituiertes N-Alkyl-N-Alkenylamino mit 1 bis 6 Kohlenstoffatomen im Alkylteil und 2 bis 6 Kohlenstoffatomen im Alkenylteil,
für gegebenenfalls durch Halogen, Cycloalkyl, Cyano, Phenyl und/oder Heterocyclyl substituiertes N-Cycloalkyl-N-alkyl-amino mit 3 bis 7 Kohlenstoffatomen im Cycloalkylteil und 1 bis 7 Kohlenstoffatomen in Alkylteil,
für gegebenenfalls durch Halogen, Cycloalkyl, Cyano, Phenyl und/oder Heterocyclyl substituiertes Alkylidenamino mit 2 bis 6 Kohlenstoffatomen,
für gegebenenfalls durch Halogen, Cycloalkyl, Cyano, Phenyl und/oder Heterocyclyl substituiertes Phenyl, für gegebenenfalls durch Halogen, Alkyl, Cycloalkyl, Cyano, Phenyl und/oder Heterocyclyl substituiertes Heterocyclyl mit 5 oder 6 Ringgliedem,
für gegebenenfalls durch Halogen, Alkyl, Cycloalkyl, Cyano, Phenyl und/oder Heterocyclyl substituiertes Heterocyclyloxy mit 5 oder 6 Ringgliedern, oder
für einen Rest der Formel -SR⁵ steht, worin
R⁵ für gegebenenfalls durch Halogen, Cycloalkyl, Cyano, Phenyl und/oder Heterocyclyl substituiertes Alkyl mit 1 bis 6 Kohlenstoffatomen,
für gegebenenfalls durch Halogen, Cycloalkyl, Cyano, Phenyl und/oder Heterocyclyl substituiertes Alkenyl mit 2 bis 6 Kohlenstoffatomen,
für gegebenenfalls durch Halogen, Cycloalkyl, Cyano, Phenyl und/oder Heterocyclyl substituiertes Alkinyl mit 2 bis 6 Kohlenstoffatomen, oder
für gegebenenfalls durch Halogen, Cycloalkyl, Cyano, Phenyl und/oder Heterocyclyl substituiertes Cycloalkyl mit 3 bis 7 Kohlenstoffatomen steht, und wobei
die zuvor genannten Heterocyclyl-Reste einfach bis dreifach, gleichartig oder verschieden substituiert sein können durch Halogen, Hydroxy, Phenyl, 1,2-Dioxyethylen, Alkyl mit 1 bis 4 Kohlenstoffatomen, Halogenalkyl mit 1 oder 2 Kohlenstoffatomen und 1 bis 5 Halogenatomen, Alkoxy mit 1 bis 4 Kohlenstoffatomen, Alkylthio mit 1 bis 4 Kohlenstoffatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, und
die zuvor genannten Heterocyclyl-Reste gesättigt oder teilweise ungesättigt sind,
die zuvor genannten Phenyl-Reste einfach bis dreifach, gleichartig oder verschieden substituiert sein können durch
Halogen, Cyano, Nitro, Amino, Hydroxy, Formyl, Carboxy, Carbamoyl, Thiocarbamoyl,
jeweils geradkettiges oder verzweigtes Alkyl, Alkoxy, Alkylthio, Alkylsulfinyl oder Alkylsulfonyl mit jeweils 1 bis 6 Kohlenstoffatomen,
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen,
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen,
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen,
Cycloalkyl mit 3 bis 6 Kohlenstoffatomen,
in 2,3-Position verknüpftes 1,3-Propandiyl, 1,4-Butandiyl, Methylendioxy (-O-CH₂-O-), 1,2-Ethylendioxy (-O-CH₂-CH₂-O-), wobei diese Reste einfach oder mehrfach, gleichartig oder verschieden substituiert sein können durch Halogen, Alkyl mit 1 bis 4 Kohlenstoffatomen und/oder Halogenalkyl mit 1 bis 4 Kohlenstoffatomen und 1 bis 9 gleichen oder verschiedenen Halogenatomen;
- R²: für Wasserstoff,
für gegebenenfalls durch Halogen, Cycloalkyl mit 3 bis 6 Kohlenstoffatomen Alkoxy mit 1 bis 4 Kohlenstoffatomen, Alkylthio mit 1 bis 4 Kohlenstoffatomen, Oxo, Hydroximino und/oder Alkoximino mit 1 bis 4 Kohlenstoffatomen substituiertes Alkyl mit 1 bis 4 Kohlenstoffatomen,
für gegebenenfalls durch Halogen und/oder Cycloalkyl mit 3 bis 6 Kohlenstoffatomen substituiertes Alkenyl mit 2 bis 4 Kohlenstoffatomen,
für gegebenenfalls durch Halogen und/oder Cycloalkyl mit 3 bis 6 Kohlenstoffatomen substituiertes Alkinyl mit 2 bis 4 Kohlenstoffatomen, oder
für gegebenenfalls durch Halogen und/oder Cycloalkyl mit 3 bis 6 Kohlenstoffatomen substituiertes Cycloalkyl mit 3 bis 6 Kohlenstoffatomen steht; oder
- R¹ und R²: gemeinsam mit dem Stickstoffatom, an das sie gebunden sind, für einen 3- bis 6-gliedrigen heterocyclischen Ring stehen, der gesättigt oder teilweise gesättigt ist, der neben dem bereits erwähnten Stickstoffatom noch ein weiteres Heteroatom aus der Reihe Stickstoff, Sauerstoff und Schwefel enthalten kann, und der einfach bis dreifach, gleichartig oder verschieden substituiert sein kann durch
Halogen, Hydroxy, Cyano, Morpholinyl, Amino, einen annelierten Phenylring, eine Methylen- oder Ethylenbrücke, Alkyl mit 1 bis 4 Kohlenstoffatomen,
Halogenalkyl mit 1 bis 4 Kohlenstoffatomen und 1 bis 9 gleichen oder verschiedenen Halogenatomen,
Alkylcarbonylamino mit 1 bis 4 Kohlenstoffatomen, Dialkylamino mit 2 bis 8 Kohlenstoffatomen,
Alkoxycarbonylamino mit 1 bis 4 Kohlenstoffatomen,
Di(alkoxycarbonyl)amino mit 2 bis 8 Kohlenstoffatomen,
Hydroxyalkyl mit 1 bis 4 Kohlenstoffatomen, Alkoxycarbonyl mit 1 bis 4 Kohlenstoffatomen, und/oder
Alkylcarbonyl mit 1 bis 4 Kohlenstoffatomen;
- R³: für Phenyl steht, das einfach bis vierfach, gleichartig oder verschieden substituiert sein kann durch
Halogen, Cyano, Nitro, Amino, Hydroxy, Formyl, Carboxy, Carbamoyl, Thiocarbamoyl,
jeweils geradkettiges oder verzweigtes Alkyl, Alkoxy, Alkylthio, Alkylsulfinyl oder Alkylsulfonyl mit jeweils 1 bis 6 Kohlenstoffatomen,
jeweils geradkettiges oder verzweigtes Alkenyl oder Alkenyloxy mit jeweils 2 bis 6 Kohlenstoffatomen,
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen,
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen,
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen,
Cycloalkyl mit 3 bis 6 Kohlenstoffatomen,
in 2,3-Position verknüpftes 1,3-Propandiyl, 1,4-Butandiyl, Methylendioxy (-O-CH₂-O-), 1,2-Ethylendioxy (-O-CH₂-CH₂-O-), wobei diese Reste einfach oder mehrfach, gleichartig oder verschieden substituiert sein können durch Halogen, Alkyl mit 1 bis 4 Kohlenstoffatomen und/oder Halogenalkyl mit 1 bis 4 Kohlenstoffatomen und 1 bis 9 gleichen oder verschiedenen Halogenatomen;
- R⁴: für gegebenenfalls durch 1 bis 9 Halogenatome substituiertes Alkyl mit 1 bis 4 Kohlenstoffatomen oder für gegebenenfalls durch 1 bis 9 Halogenatome substituiertes Cycloalkyl mit 3 bis 6 Kohlenstoffatomen steht; und
- X: für Fluor, Chlor oder Brom steht;
sowie Säure-Additionssalze von denjenigen Verbindungen der Formel (I), in denen R¹ für Amino steht,
gefunden.

Die erfindungsgemäßen Verbindungen können je nach Substitutionsmuster gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Ist R³ an beiden Atomen, die der Bindungsstelle benachbart sind, ungleich substituiert, können die betreffenden Verbindungen in einer besonderen Form der Stereoisometrie, als Atropisomere, vorliegen.

Weiterhin wurde gefunden, dass sich Triazolopyrimidine der Formel (I) herstellen lassen, indem man
a) Dihalogen-triazolopyrimidine der Formel 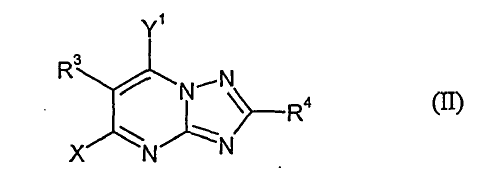 in welcher
   R³, R⁴ und X die oben angegebenen Bedeutungen haben und
   Y¹ für Halogen steht,
   mit Aminen der Formel 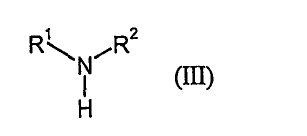 in welcher
   - R¹ und R²: die oben angegebenen Bedeutungen haben,
   gegebenenfalls in Gegenwart eines Verdünnungsmittels und gegebenenfalls in Gegenwart eines Säureakzeptors umsetzt,
   oder
b) Triazolopyrimidine der Formel 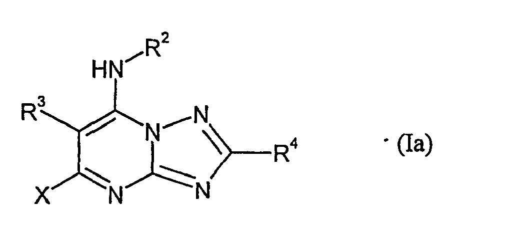 in welcher
   - R², R³, R⁴ und X: die oben angegebenen Bedeutungen haben,
   mit Sulfensäurehalogeniden der Formel

   Y²-S-R⁵ (IV),

   in welcher
   - R⁵: die oben angegebenen Bedeutungen hat und
   - Y²: für Halogen steht, gegebenenfalls in Gegenwart eines Verdünnungsmittels und gegebenenfalls in Gegenwart eines Säureakzeptors umsetzt,
   und gegebenenfalls an die so erhaltenen Verbindungen der Formel (I), in denen
   - R¹: für Amino steht,
   eine Säure addiert.

Schließlich wurde gefunden, dass sich die neuen Triazolopyrimidine der Formel (I) bzw. deren Säureadditions-Salze sehr gut zur Bekämpfung von unerwünschten Mikroorganismen eignen. Sie zeigen vor allem eine starke fungizide Wirksamkeit und lassen sich sowohl im Pflanzenschutz als auch im Materialschutz verwenden.

Überraschenderweise besitzen die erfindungsgemäßen Triazolopyrimidine der Formel (I) eine wesentlich bessere mikrobizide Wirksamkeit als die konstitutionell ähnlichsten, vorbekannten Stoffe gleicher Wirkungsrichtung.
- R¹: steht bevorzugt für Hydroxy, Amino, für gegebenenfalls durch Halogen, Cyano, Hydroxy, Amino, Phenyl, Heterocyclyl, Alkoxy mit 1 bis 4 Kohlenstoffatomen, Alkoxycarbonyl mit 1 bis 4 Kohlenstoffatomen, Alkylamino mit 1 bis 4 Kohlenstoffatomen, Dialkylamino mit 2 bis 8 Kohlenstoffatomen, Cycloalkyl mit 3 bis 6 Kohlenstoffatomen, Halogencycloalkyl mit 3 bis 6 Kohlenstoffatomen und 1 bis 5 Halogenatomen, Alkylthio mit 1 bis 4 Kohlen- / stoffatomen, Oxo, Hydroxyimino und/oder Alkoximino mit 1 bis 4 Kohlenstoffatomen substituiertes Alkyl mit 1 bis 6 Kohlenstoffatomen,
für gegebenenfalls durch Halogen, Cycloalkyl, Cyano, Phenyl und/oder Heterocyclyl substituiertes Alkenyl mit 2 bis 6 Kohlenstoffatomen,
für gegebenenfalls durch Halogen, Cycloalkyl, Cyano, Phenyl und/oder Heterocyclyl substituiertes Alkinyl mit 2 bis 6 Kohlenstoffatomen,
für gegebenenfalls durch Halogen, Cycloalkyl, Cyano, Halogenalkyl mit 1 bis 2 Kohlenstoffatomen und 1 bis 5 Halogenatomen, Phenyl und/oder Heterocyclyl substituiertes Cycloalkyl mit 3 bis 7 Kohlenstoffatomen,
für gegebenenfalls durch Halogen, Cycloalkyl, Cyano, Phenyl und/oder Heterocyclyl substituiertes Alkoxy mit 1 bis 7 Kohlenstoffatomen,
für gegebenenfalls durch Halogen, Cycloalkyl, Cyano, Phenyl und/oder Heterocyclyl substituiertes Alkenyloxy mit 2 bis 6 Kohlenstoffatomen,
für gegebenenfalls durch Halogen, Cycloalkyl, Cyano, Phenyl und/oder Heterocyclyl substituiertes Alkinyloxy mit 2 bis 6 Kohlenstoffatomen,
für gegebenenfalls durch Halogen, Cycloalkyl, Cyano, Phenyl und/oder Heterocyclyl substituiertes Cycloalkyloxy mit 3 bis 7 Kohlenstoffatomen,
für gegebenenfalls durch Halogen, Cycloalkyl, Cyano, Phenyl und/oder Heterocyclyl substituiertes Alkylamino mit 1 bis 7 Kohlenstoffatomen,
für gegebenenfalls durch Halogen, Cycloalkyl, Cyano, Phenyl und/oder Heterocyclyl substituiertes Dialkylamino mit 1 bis 7 Kohlenstoffatomen in jedem der Alkylreste,
für gegebenenfalls durch Halogen, Cycloalkyl, Cyano, Phenyl und/oder Heterocyclyl substituiertes Alkenylamino mit 2 bis 6 Kohlenstoffatomen,
für gegebenenfalls durch Halogen, Cycloalkyl, Cyano, Phenyl und/oder Heterocyclyl substituiertes Alkinylamino mit 2 bis 6 Kohlenstoffatomen,
für gegebenenfalls durch Halogen, Cyano, Phenyl und/oder Heterocyclyl substituiertes N-Alkyl-N-Alkenylamino mit 1 bis 6 Kohlenstoffatomen im Alkylteil und 2 bis 6 Kohlenstoffatomen im Alkenylteil,
für gegebenenfalls durch Halogen, Cycloalkyl, Cyano, Phenyl und/oder Heterocyclyl substituiertes Cycloalkylamino mit 3 bis 7 Kohlenstoffatomen,
für gegebenenfalls durch Halogen, Cycloalkyl, Cyano, Phenyl und/oder Heterocyclyl substituiertes N-Cycloalkyl-N-alkyl-amino mit 3 bis 7 Kohlenstoffatomen im Cycloalkylteil und 1 bis 7 Kohlenstoffatomen in Alkylteil,
für gegebenenfalls durch Halogen, Cycloalkyl, Cyano, Phenyl und/oder Heterocyclyl substituiertes Alkylidenamino mit 2 bis 6 Kohlenstoffatomen,
für gegebenenfalls durch Halogen, Cycloalkyl, Cyano, Phenyl und/oder Heterocyclyl substituiertes Phenyl,
für gegebenenfalls durch Halogen, Alkyl, Cycloalkyl, Cyano, Phenyl und/oder Heterocyclyl substituiertes Heterocyclyl mit 5 oder 6 Ringgliedern,
für gegebenenfalls durch Halogen, Alkyl, Cycloalkyl, Cyano, Phenyl und/oder Heterocyclyl substituiertes Heterocyclyloxy mit 5 oder 6 Ringgliedem,
für -SR⁵, worin
R⁵ für gegebenenfalls durch Halogen, Cycloalkyl, Cyano, Phenyl und/oder Heterocyclyl substituiertes Alkyl mit 1 bis 6 Kohlenstoffatomen,
für gegebenenfalls durch Halogen, Cycloalkyl, Cyano, Phenyl und/oder Heterocyclyl substituiertes Alkenyl mit 2 bis 6 Kohlenstoffatomen,
für gegebenenfalls durch Halogen, Cycloalkyl, Cyano, Phenyl und/oder Heterocyclyl substituiertes Alkinyl mit 2 bis 6 Kohlenstoffatomen oder
für gegebenenfalls durch Halogen, Cycloalkyl, Cyano, Phenyl und/oder Heterocyclyl substituiertes Cycloalkyl mit 3 bis 7 Kohlenstoffatomen steht, wobei die zuvor genannten Heterocyclyl-Reste einfach bis dreifach, gleichartig oder verschieden substituiert sein können durch
Halogen, Hydroxy, Phenyl, 1,2-Dioxyethylen, Alkyl mit 1 bis 4 Kohlenstoffatomen, Halogenalkyl mit 1 oder 2 Kohlenstoffatomen und 1 bis 5 Halogenatomen, Alkoxy mit 1 bis 4 Kohlenstoffatomen, Alkylthio mit 1 bis 4 Kohlenstoffatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen und wobei die zuvor genannten Heterocyclylreste gesättigt oder teilweise ungesättigt sind,
und wobei die zuvor genannten Phenyl-Reste einfach bis dreifach, gleichartig oder verschieden substituiert sein können durch
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen,
in 2,3-Position verknüpftes 1,3-Propandiyl, 1,4-Butandiyl, Methylendioxy (-O-CH₂-O-), 1,2-Ethylendioxy (-O-CH₂-CH₂-O-), wobei diese Reste einfach oder mehrfach, gleichartig oder verschieden substituiert sein können durch Halogen, Alkyl mit 1 bis 4 Kohlenstoffatomen und/oder Halogenalkyl mit 1 bis 4 Kohlenstoffatomen und 1 bis 9 gleichen oder verschiedenen Halogenatomen.
- R²: steht bevorzugt für Wasserstoff, für gegebenenfalls durch Halogen, Cycloalkyl mit 3 bis 6 Kohlenstoffatomen Alkoxy mit 1 bis 4 Kohlenstoffatomen, Alkylthio mit 1 bis 4 Kohlenstoffatomen, Oxo, Hydroximino und/oder Alkoximino mit 1 bis 4 Kohlenstoffatomen substituiertes Alkyl mit 1 bis 4 Kohlenstoffatomen.
für gegebenenfalls durch Halogen und/oder Cycloalkyl mit 3 bis 6 Kohlenstoffatomen substituiertes Alkenyl mit 2 bis 4 Kohlenstoffatomen,
für gegebenenfalls durch Halogen und/oder Cycloalkyl mit 3 bis 6 Kohlenstoffatomen substituiertes Alkinyl mit 2 bis 4 Kohlenstoffatomen oder
für gegebenenfalls durch Halogen und/oder Cycloalkyl mit 3 bis 6 Kohlenstoffatomen substituiertes Cycloalkyl mit 3 bis 6 Kohlenstoffatomen.
- R¹ und R²: stehen bevorzugt gemeinsam mit dem Stickstoffatom, an das sie gebunden sind, für einen 3- bis 6-gliedrigen heterocyclischen Ring, der gesättigt oder teilweise gesättigt ist, der neben dem bereits erwähnten Stickstoffatom noch ein weiteres Heteroatom aus der Reihe Stickstoff, Sauerstoff und Schwefel enthalten kann und der einfach bis dreifach, gleichartig oder verschieden substituiert sein kann durch
Halogen, Hydroxy, Cyano, Morpholinyl, Amino, einen annelierten Phenyl-ring, eine Methylen- oder Ethylenbrücke,
Alkyl mit 1 bis 4 Kohlenstoffatomen,
Halogenalkyl mit 1 bis 4 Kohlenstoffatomen und 1 bis 9 gleichen oder verschiedenen Halogenatomen;
Alkylcarbonalamino mit 1 bis 4 Kohlenstoffatomen,
Dialkylamino mit 2 bis 8 Kohlenstoffatomen,
Alkoxycarbonylamino mit 1 bis 4 Kohlenstoffatomen,
Di(alkoxycarbonyl)amino mit 2 bis 8 Kohlenstoffatomen,
Hydroxyalkyl mit 1 bis 4 Kohlenstoffatomen,
Alkoxycarbonyl mit 1 bis 4 Kohlenstoffatomen und/oder
Alkylcarbonyl mit 1 bis 4 Kohlenstoffatomen.
- R³: steht bevorzugt für Phenyl, das einfach bis vierfach, gleichartig oder verschieden substituiert sein kann durch
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen,
in 2,3-Position verknüpftes 1,3-Propandiyl, 1,4-Butandiyl, Methylendioxy (-O-CH₂-O-), 1,2-Ethylendioxy (-O-CH₂-CH₂-O-), wobei diese Reste einfach oder mehrfach, gleichartig oder verschieden substituiert sein können durch Halogen, Alkyl mit 1 bis 4 Kohlenstoffatomen und/oder Halogenalkyl mit 1 bis 4 Kohlenstoffatomen und 1 bis 9 gleichen oder verschiedenen Halogenatomen.
- R⁴: steht bevorzugt für gegebenenfalls durch 1 bis 9 Halogenatome substituiertes Alkyl mit 1 bis 4 Kohlenstoffatomen oder für gegebenenfalls durch 1 bis 9 Halogenatome substituiertes Cycloalkyl mit 3 bis 6 Kohlenstoffatomen.
- X: steht bevorzugt für Fluor, Chlor oder Brom.
- R¹: steht besonders bevorzugt für Hydroxy, Amino, für Methyl, Ethyl, n-Propyl, i-Propyl, n-, i-, s- oder t-Butyl, Methoxymethyl, 2-Methoxy-ethyl, Methylthiomethyl, 2-Methylthio-ethyl, Hydroximinomethyl, Methoximinomethyl, Acetylmethyl, 2-Hydroximino-propyl, 2-Methoximino-propyl, Allyl, 2-Methyl-prop-2-enyl, Propargyl, 2,2,2-Trifluorethyl, 1-(Trifluormethyl)-ethyl, 3,3,3-Trifluorpropyl, Cyclopentyl, Cyclohexyl,
Cyclopropyloxy, Cyclobutyloxy, Cyclopentyloxy, Cyclohexyloxy, Difluormethoxy, Trifluormethoxy, Difluorchlormethoxy, Trifluorethoxy,
Methylamino, Ethylamino, n- oder i-Propylamino, n-, i-, s- oder t-Butylamino, Dimethylamino, Diethylamino, Trifluorethylamino, Cyclohexylmethylamino, 2-Cyanethylamino, Allylamino, 1-Cyclopropylethylamino, Cyclopropylamino, Cyclobutylamino, Cyclopentylamino, Cyclohexylamino, 1-Methylethylidenamino, Phenyl, Benzyloxy, Piperidinyl, Morpholinyl, Pyridylmethoxy, Thiazolylmethoxy, oder für -S-R⁵, worin
R⁵ für Methyl, Ethyl, n- oder i-Propyl, Difluormethyl, Difluorchlormethyl, Dichlorfluormethyl oder Trifluormethyl steht,
oder
- R¹: steht für (2,2-Dichlorcyclopropyl)methyl, (2-Furyl)methyl, (2-Tetrahydrofuryl)methyl, (2-Tetrahydropyranyl)methyl, 1,2-Dimethylpropyl, 1,3-Dioxolan-2-ylmethyl, 1-Cyclopropylethyl, 1-Cyclopropylethylamino, 1-Methylethylidenamino, 2,2,2-Trifluor-1-methylethyl, 2,4-Dichlorbenzyloxy, 2,6-Dichlorbenzyloxy, 2-Butyl, 2-Chlorbenzyloxy, 2-Fluorcyclopropyl, 2-Hexahydropyranyloxy, 2-Methoxyethyl, 2-Thienylmethyl, 2-Tolyl, 2-Trifluormethylcyclohexyl, 3-(Dimethylamino)-propyl, 3,5-bis-Trifluormethylcyclohexyl, 3,5-Dichlorbenzyloxy, 3-Aminopropyl, 3-Chlorbenzyloxy, 3-Tolyl, 3-Trifluormethylbenzyloxy, 3-Trifluormethylcyclohexyl, 3,5-(Bis-trifluormethyl)-cyclohexyl, 2-Trifluormethyl-cyclohexyl, 4-Trifluormethyl-cyclohexyl, 4-Chlorbenzyloxy, 4-Fluorbenzyloxy, 4-Fluorphenyl, 4-Tolyl, 4-Trifluormethylbenzyloxy, 4-Trifluormethylcyclohexyl, Allyl, Allylamino, Allyloxy, Benzyloxy, -C(CH₃)₂-CF₃, -C(CH₃)₂-CH₂-COCH₃, -C₂H₅, -CH(CH₂OH)-COOCH₃, -CH(CH₃)-C(CH₃)₃-, -CH(CH₃)-CH(O-CH₃)₂-, -CH(CH₃)-CH=CH₂-, -CH(CH₃)-CH₂-CH(CH₃)₂-, -CH(CH₃)-CH₂-O-CH₃-, -CH(CH₃)-CH₂-OH, -CH(CH₃)-COOCH₃, -CH(CH₃)-COO-t-butyl, -CH₂-C(CH₃)=CH₂, -CH₂-C(CH₃)₃, -CH₂-CF₃, -CH₂-CH(OCH₃)₂,-CH₂-CH₂-CF₃, -CH₂-CH₂-Cl, -CH₂-CH₂-CN, -CH₂-CH₂-N(CH₃)₂, -CH₂-CH₂-N(CH₃)₂, -CH₂-CH₂-NH₂, -CH₂-CHF₂, -CH₂-CN, -CH₂-COOC₂H₅, -CH₂-COOC₂H₅, -CH₂-COOCH₃, -CH₃, Cyclohexyl, Cyclopentyl, Cyclopropyl, Cyclopropylmethyl, Dimethylamino, i-Butoxy, i-Butyl, i-Propylamino, n-Butoxy, n-Butyl, n-Butylamino, -NH₂, -NH-CH₂-CF₂-CHF₂, -NH-CH₂-CF₃, -NH-CH₂-CH(CH₃)₂, -O-C₂H₅, -O-CH(CH₃)-CH₂-CH₃, -O-CH₃, -OH, O-i-Propyl, Propargyl, t-Butoxy, t-Butyl, t-Butylamino, oder für eine Gruppierung 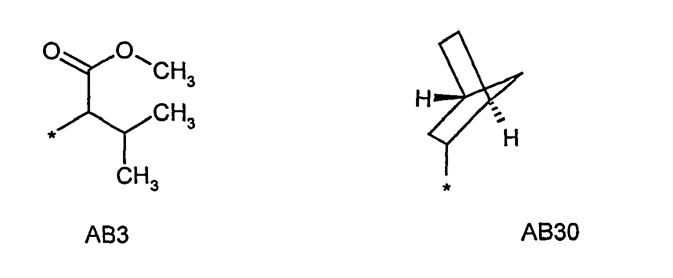 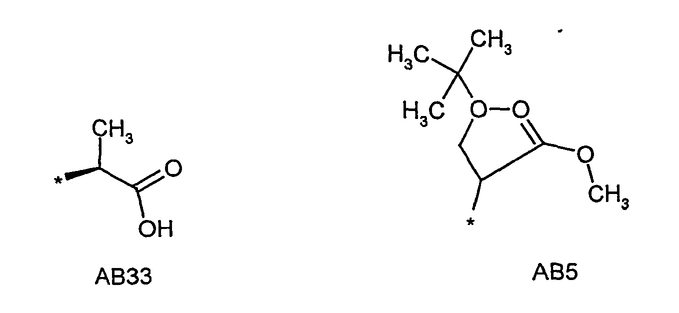 (* markiert jeweils die Bindungsstelle)
wobei die zuvor genannten Thiazolyl- und Pyridyl-Reste im Falle von Thiazolyl einfach oder zweifach und im Falle von Pyridyl einfach bis dreifach, jeweils gleichartig oder verschieden substituiert sein können durch Fluor, Chlor Brom, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n-oder i-Propylthio, Difluormethoxy, Trifluormethoxy, Difluorchlormethoxy, Trifluorethoxy, Difluormethylthio, Difluorchlormethylthio, Dichlorfluormethylthio, Trifluormethylthio und/oder Phenyl, und wobei die zuvor genannten Phenyl- und Benzyloxy-Reste im Phenylteil einfach bis dreifach, gleichartig oder verschieden substituiert sein können durch
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl oder Cyclohexyl,
in 2,3-Position verknüpftes 1,3-Propandiyl, Methylendioxy (-O-CH₂-O-), 1,2-Ethylendioxy (-O-CH₂-CH₂-O-), wobei diese Reste einfach oder mehrfach, gleichartig oder verschieden substituiert sein können durch Fluor, Chlor, Methyl, Ethyl, n-Propyl, i-Propyl und/oder Trifluormethyl.
- R²: steht besonders bevorzugt für Wasserstoff, Methyl, Ethyl, n- oder i-Propyl, n-, i-, s- oder t-Butyl, Methoxymethyl, 2-Methoxy-ethyl, Methylthiomethyl, 2-Methylthio-ethyl, Hydroximinomethyl, Methoximinomethyl, Acetylmethyl, 2-Hydroxyimino-propyl, 2-Methoxyimino-propyl, Allyl, Propargyl, 2,2,2-Trifluorethyl, 1-(1,1,1-Trifluormethyl)ethyl, Cyclopropylmethyl, Cyclobutyl-methyl, Cyclopentylmethyl oder Cyclohexylmethyl.
- R¹ und R²: stehen besonders bevorzugt gemeinsam mit dem Stickstoffatom, an welches sie gebunden sind, für 1-Pyrrolinyl, 3-Pyrrolinyl, Pyrrolidinyl, Dihydropyridinyl, Piperidinyl, Pyrazolinyl, Pyrazolidinyl, Imidazolinyl, Imidiazolidinyl, 1,2-Diazinan-yl, 1,3-Diazinan-yl, Piperazinyl, Oxazolinyl, Oxazolidinyl, Isoxazolyl, Isoxazolidinyl, Dihydrooxazinyl, Morpholinyl, Thiazolinyl, Thiazolidinyl, Thiomorpholinyl, wobei die genannten Heterocyclen substituiert sein können durch
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulf-onyl, Trifluormethyl, Trifluorethyl, Difluormethoxy, Trifluormethoxy, Difluorchlormethoxy, Trifluorethoxy, Difluormethylthio, Difluorchlor-methylthio, Trifluormethylthio, Trifluormethylsulfinyl, Trifluormethylsulf-onyl, Methylamino, Ethylamino, n- oder i-Propylamino, Dimethylamino, Diethylamino, Acetyl, Propionyl, Acetyloxa, Methoxycarbonyl, Ethoxycarb-onyl, Methylsulfonyloxy, Ethylsulfonyloxy, Hydroximinomethyl, Hydroximi-noethyl, Methoximinomethyl, Ethoximinomethyl, Methoximinoethyl, Eth-.oximinoethyl, Cyclopropyl, Cyclobutyl, Cyclopentyl, Cyclohexyl, durch einen annelierten Phenylring oder
durch eine Methandiyl- oder Ethandiyl-Brücke.
- R³: steht besonders bevorzugt für Phenyl, das einfach bis dreifach, gleichartig oder verschieden substituiert sein kann durch
Fluor, Chlor, Brom, Cyano, Nitro, Formyl, Methyl, Ethyl, n- oder i-Propyl, n-, i-, s- oder t-Butyl, Allyl, Propargyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Allyloxy, Propargyloxy, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Trichlorethinyloxy, Trifluorethinyloxy, Chlorallyloxy, Iodpropargyloxy, Methylamino, Ethylamino, n- oder i-Propylamino, Dimethylamino, Diethylamino, Acetyl, Propionyl, Acetyloxy, Methoxycarbonyl, Ethoxycarbonyl, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl oder Cyclohexyl,
in 2,3-Position verknüpftes 1,3-Propandiyl, Methylendioxy (-O-CH₂-O-), 1,2-Ethylendioxy (-O-CH₂-CH₂-O-), wobei diese Reste einfach oder mehrfach, gleichartig oder verschieden substituiert sein können durch Fluor, Chlor, Methyl, Ethyl, n-Propyl, i-Propyl und/oder Trifluormethyl.
- R⁴: steht besonders bevorzugt für Methyl, Ethyl, n-Propyl, i-Propyl, n-, i-, s- oder t-Butyl, Trifluormethyl, Trifluorethyl oder Cyclopropyl.
- X: steht besonders bevorzugt für Fluor oder Chlor.
- R¹ und R²: stehen ganz besonders bevorzugt gemeinsam für eine der folgenden Gruppierungen

-CH(CF₃)-CH₂-CH₂-CH₂-, -CH(CF₃)-CH₂-CH₂-CH₂-CH₂-,

-CH(CH₃)-CH=CH-CH(CH₃)-, -CH(CH₃)-CH₂-CH₂-O-,

-CH(COOCH₃)-, -CH₂-CH(CH₃)-CH₂-CH(CH₃)-CH₂-,

-CH₂-CH(CH₃)-O-CH(CH₃)-CH₂-, -CH₂-CH(NH₂)-CH₂-CH₂-

CH₂-C-CH₂-CH(OH)-CH₂-CH₂-, -CH₂-CH(OH)-CH₂-CH₂-CH₂-

-CH₂-CH=C(C₂H₅)-CH₂-CH₂-, -CH₂-CH₂-C(CH₃)₂-CH₂-CH₂-

-CH₂-CH₂-CH₂-, -CH₂-CH₂-CH(CF₃)-CH₂-CH₂-

-CH₂-CH₂-CH(CH₃)-CH₂-CH₂-, -CH₂-CH₂-CH(CH₃)-CH₂-CH₂-

-CH₂-CH₂-CH(COCH₃)-CH₂-CH₂-, -CH₂-CH₂-CH(COOCH₃)-CH₂-CH₂-

-CH₂-CH₂-CH(NH-COCH₃)-CH₂-CH₂-, -CH₂-CH₂-CH(OH)-CH₂-CH₂-

-CH₂-CH₂-CH=C(CH₃)-CH₂-, -CH₂-CH₂-CH=CH-CH₂-

-CH₂-CH₂-CH₂-CH(CH₃)-, -CH₂-CH₂-CH₂-CH(CH₃)-CH₂-

-CH₂-CH₂-CH₂-CH₂-, -CH₂-CH₂-CH₂-CH₂-CH(CH₃)-

-CH₂-CH₂-CH₂-CH₂-CH₂-, -CH₂-CH₂-CHBr-CH₂-CH₂-

-CH₂-CH₂-CHF-CH₂-CH₂-, -CH₂-CH₂-N(CH₃)-CH₂-CH₂-

-CH₂-CH₂-O-CH₂-CH₂-, -CH₂-CH₂-S-CH₂-CH₂-

-CH₂-S-CH₂-CH₂-, -NH-CH₂-CH₂-CH₂-CH₂-

-O-CH₂-CH₂-CH₂-CH(CH₃)-, -O-CH₂-CH₂-CH₂-CH₂-,

oder für eine der folgenden Gruppierungen, in denen X₂ für das Stickstoffatom steht, an welches die Reste R¹, R² gebunden sind 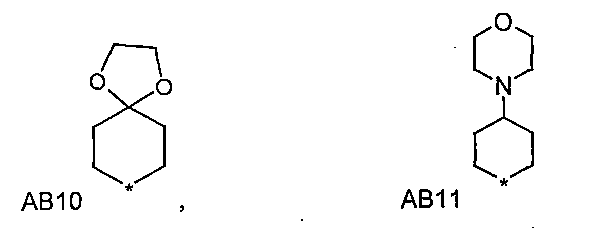 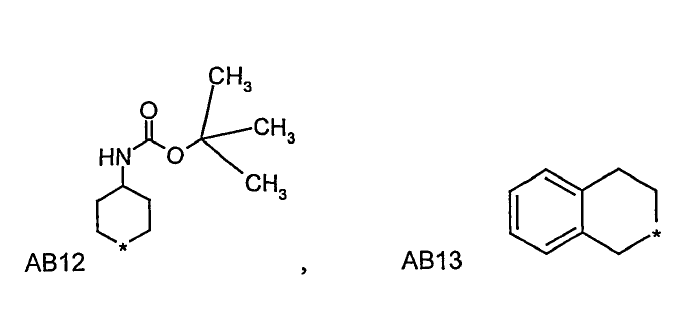 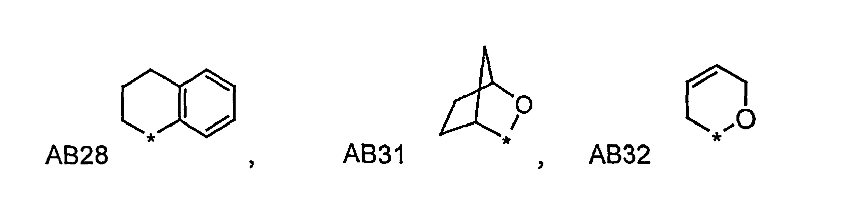 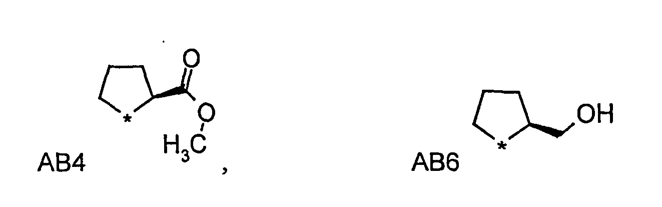 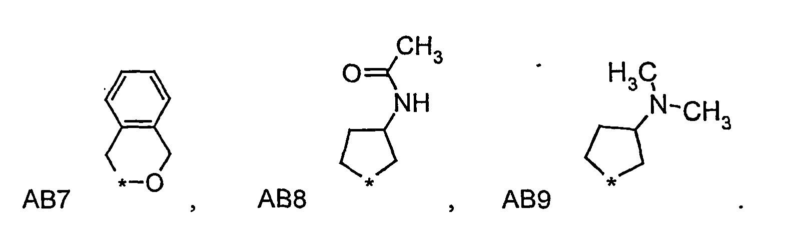 (* markiert jeweils die Bindungsstelle)
- R²: steht ganz besonders bevorzugt für Wasserstoff, Methyl, Ethyl, n-, i-Propyl, n-, i-, s- oder t-Butyl.
- R³: steht ganz besonders bevorzugt für Phenyl, welches einfach bis dreifach, gleich oder verschieden durch Fluor und/oder Chlor in den Positionen 2, 4 und 6 substituiert ist.
- X: steht ganz besonders bevorzugt für Chlor.

Ganz besonders bevorzugt sind weiterhin diejenigen Verbindungen der Formel (I), in denen
- R¹, R², R⁴ und X: die zuvor genannten bevorzugten Bedeutungen haben und
- R³: für 2,4-disubstituiertes, 2,6-disubstituiertes oder 2,4,6-trisubstituiertes Phenyl steht.

Ganz besonders bevorzugt sind weiterhin diejenigen Verbindungen der Formel (I), in denen
- R¹, R², R⁴ und X: die zuvor genannten Bedeutungen haben und
- R³: für 2-Chlor-4-fluorphenyl, 2-Chlor-6-fluorphenyl oder 2-Chlor-4,6-difluorphenyl steht.

Ganz besonders bevorzugt sind weiterhin diejenigen Verbindungen der Formel (I), in denen
- R¹, R², R³: und X die zuvor genannten Bedeutungen haben und
- R⁴: für Cyclopropyl steht.

Ganz besonders bevorzugt sind weiterhin diejenigen Verbindungen der Formel (I), in denen
X, R³ und R⁴ die zuvor genannten Bedeutungen haben und
- R¹: für Wasserstoff und
- R²: für -CH(CH₃)CF₃ steht.

Eine weitere ganz besonders bevorzugte Gruppe von Verbindungen sind diejenigen Triazolopyrimidine der Formel (I), in denen
- R4: für Cyclopropyl steht und

R¹, R², R³ und X die zuvor als bevorzugt genannten Bedeutungen haben.

Die zuvor genannten Reste-Definitionen können untereinander in beliebiger Weise kombiniert werden. Außerdem können auch einzelne Bedeutungen entfallen.

Bevorzugte erfindungsgemäße Verbindungen sind auch Additionsprodukte aus Säuren und denjenigen Triazolopyrimidinen der Formel (I), in denen
- R¹: für Amino steht und
- R², R³, R⁴ und X: diejenigen Bedeutungen haben, die für diese Reste als bevorzugt genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure, 1,5-Naphthalindisulfonsäure, Saccharin und Thiosaccharin.

Verwendet man 5,7-Dichlor-2-(trifluormethyl)-6-(2,4,6-trifluorphenyl)-[1,2,4]-triazolo[1,5-a]-pyrimidin und 4-Trifluormethylpiperidin als Ausgangsstoffe, so kann der Verlauf des erfindungsgemäßen Verfahrens (a) durch das folgende Formelschema veranschaulicht werden.

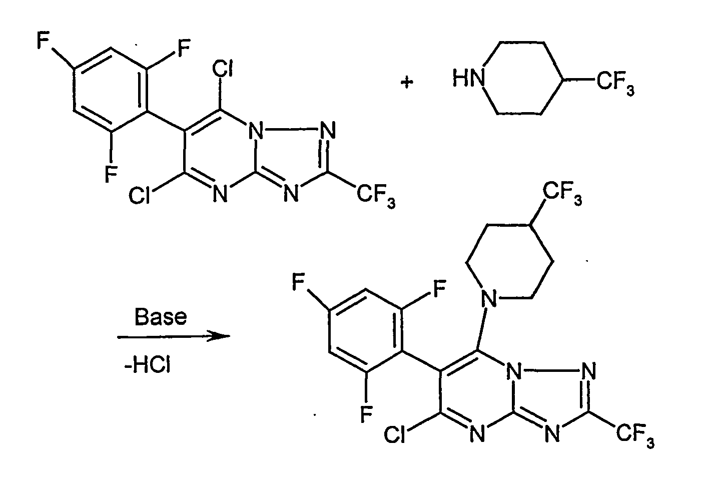

Verwendet man 5-Chlor-2-(trifluormethyl)-N-[(1S)-2,2,2-trifluor-1-methyl-ethyl]-6-(2,4,6-trifluorphenyl)-[1,2,4]-triazolo-[1,5-a]pyrimidin-7-amin und Dichlorfluormethan-sulfenylchlorid als Ausgangsstoffe, so kann der Verlauf des erfindungsgemäßen Verfahrens (b) durch das folgende Formelschema veranschaulicht werden.

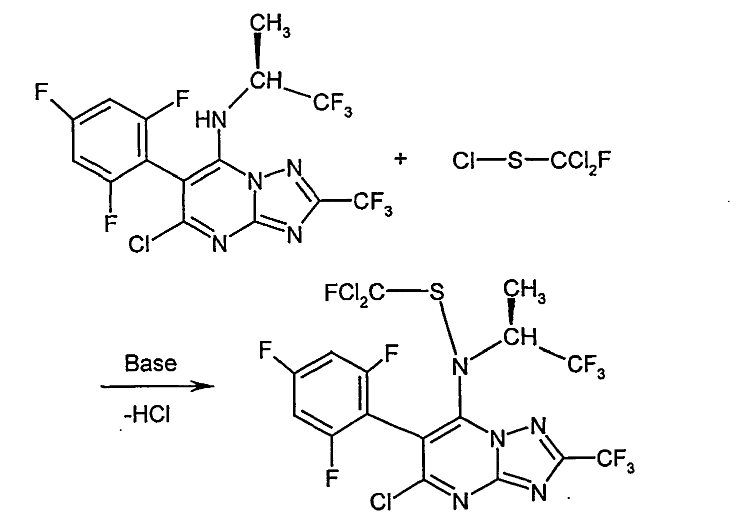

Die bei der Durchführung des erfindungsgemäßen Verfahrens (a) als Ausgangsstoffe benötigten Dihalogen-triazolo-pyrimidine sind durch die Formel (II) allgemein definiert. In dieser Formel haben R³, R⁴ und X vorzugsweise diejenigen Bedeutungen, die bereits im Zusammenhang mit der Beschreibung der erfindungsgemäßen Stoffe der Formel (I) für diese Reste als bevorzugt genannt wurden. Y¹ steht vorzugsweise für Fluor, Chlor oder Brom, besonders bevorzugt für Fluor oder Chlor.

Die Dihalogen-triazolopyrimidine der Formel (II) sind neu. Auch diese Stoffe eignen sich zur Bekämpfung von unerwünschten Mikroorganismen.

Die Dihalogen-triazolopyrimidine lassen sich herstellen, indem man
c) Dihydroxy-triazolo-pyrimidine der Formel 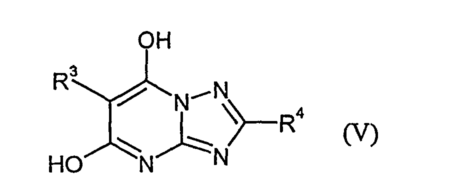 in welcher
   - R³ und R⁴: die oben angegebenen Bedeutungen haben,
mit Halogenierungsmitteln, gegebenenfalls in Gegenwart eines Verdünnungsmittels umsetzt.

Die bei der Durchführung des Verfahrens (c) als Ausgangsstoffe benötigten Dihydroxy-triazolopyrimidine sind durch die Formel (V) allgemein definiert. In dieser Formel haben R³ und R⁴ vorzugsweise diejenigen Bedeutungen, die bereits im Zusammenhang mit der Beschreibung der erfindungsgemäßen Stoffe der Formel (I) für diese Reste als bevorzugt genannt wurden.

Auch die Dihydroxy-triazolopyrimidine der Formel (V) sind bisher noch nicht bekannt. Sie lassen sich herstellen, indem man
d) Arylmalonester der Formel 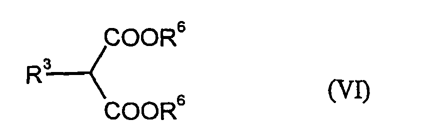 in welcher
   - R³: die oben angegebenen Bedeutungen hat und
   - R⁶: für Alkyl mit 1 bis 4 Kohenstoffatomen steht,
   mit Aminotriazolen der Formel 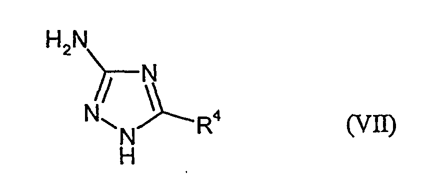
in welcher
- R⁴: die oben angegebenen Bedeutungen hat,
gegebenenfalls in Gegenwart eines Verdünnungsmittels und gegebenenfalls in Gegenwart eines Säurebindemittels umsetzt.

Die bei der Durchführung des Verfahrens (d) als Ausgangsstoffe benötigten Arylmalonester sind durch die Formel (VI) allgemein definiert. In dieser Formel hat R³ vorzugsweise diejenigen Bedeutungen, die bereits im Zusammenhang mit der Beschreibung der erfindungsgemäßen Stoffe der Formel (I) für diesen Rest als bevorzugt genannt wurden. R⁶ steht vorzugsweise für Methyl oder Ethyl.

Die Arylmalonester der Formel (VI) sind bekannt oder lassen sich nach bekannten Methoden herstellen (vgl. US-A 6 156 925).

Die bei der Durchführung des Verfahrens (d) weiterhin als Ausgangsstoffe benötigten Aminotriazole sind durch die Formel (VII) allgemein definiert. In dieser Formel hat R⁴ vorzugsweise diejenigen Bedeutungen, die bereits im Zusammenhang mit der Beschreibung der erfindungsgemäßen Stoffe der Formel (I) für diesen Rest als bevorzugt genannt wurden.

Die Aminotriazole der Formel (VII) sind bekannt oder können nach bekannten Methoden hergestellt werden (vergl. J. Org. Chem. (1974), 39(11), Khim. Geterotsikl. Soedin. (1989), (2), 278 oder Zh. Obshch. Khim. (1969), 39(11)).

Als Verdünnungsmittel kommen bei der Durchführung des Verfahrens (d) alle für derartige Umsetzungen üblichen, inerten organischen Solventien in Frage. Vorzugsweise verwendbar sind Alkohole, wie Methanol, Ethanol, n-Propanol, i-Propanol, n-Butanol und tert.-Butanol.

Als Säurebindemittel kommen bei der Durchführung des Verfahrens (d) alle für derartige Umsetzungen üblichen anorganischen und organischen Basen in Betracht. Vorzugsweise verwendbar sind tertiäre Amine, wie Tributylamin oder Pyridin. Im Überschuss eingesetztes Amin kann auch als Verdünnungsmittel fungieren.

Die Temperaturen können bei der Durchführung des Verfahrens (d) in einem größeren Bereich variiert werden. Im allgemeinen arbeitet man bei Temperaturen zwischen 20°C und 200°C, vorzugsweise zwischen 50°C und 180°C.

Bei der Durchführung des Verfahrens (d) arbeitet man im allgemeinen unter Atmosphärendruck. Es ist allerdings auch möglich, unter erhöhtem oder vermindertem Druck zu arbeiten.

Bei der Durchführung des Verfahrens (d) setzt man Arylmalonester der Formel (VI) und Aminotriazol der Formel (VII) im allgemeinen in äquivalenten Mengen um. Es ist aber auch möglich, die eine oder andere Komponente in einem Überschuss zu verwenden. Die Aufarbeitung erfolgt nach üblichen Methoden.

Als Halogenierungsmittel kommen bei der Durchführung des Verfahrens (c) alle für den Ersatz von Hydroxygruppen durch Halogen üblichen Komponenten in Betracht. Vorzugsweise verwendbar sind Phosphortrichlorid, Phosphortribromid, Phosphorpentachlorid, Phosphoroxychlorid, Thionylchlorid, Thionylbromid oder deren Gemische. Die entsprechenden Fluor-Verbindungen der Formel (II) lassen sich aus den Chlor- oder Brom-Verbindunngen durch Umsetzung mit Kaliumfluorid herstellen.

Als Verdünnungsmittel kommen bei der Durchführung des Verfahrens (c) alle für derartige Halogenierungen üblichen Solventien in Frage. Vorzugsweise verwendbar sind halogenierte aliphatische oder aromatische Kohlenwasserstoffe, wie Chlorbenzol. Als Verdünnungsmittel kann aber auch das Halogenierungsmittel selbst, z.B. Phosphoroxychlorid oder ein Gemisch von Halogenierungsmitteln fungieren.

Die Temperaturen können auch bei der Durchführung des Verfahrens (c) in einem größeren Bereich variiert werden. Im allgemeinen arbeitet man bei Temperaturen zwischen 0°C und 150°C, vorzugsweise zwischen 10°C und 120°C.

Bei der Durchführung des Verfahrens (c) arbeitet man im allgemeinen unter Atmosphärendruck. Es ist aber auch möglich, unter erhöhtem Druck zu arbeiten.

Bei der Durchführung des Verfahrens (d) setzt man Dihydroxy-triazolpyrimidin der Formel (V) im allgemeinen mit einem Überschuss an Halogenierunngsmittel um. Die Aufarbeitung erfolgt nach üblichen Methoden.

Die weiterhin zur Durchführung des erfindungsgemäßen Verfahrens (a) als Ausgangsstoffe benötigten Amine sind durch die Formel (III) allgemein definiert. In dieser Formel haben R¹ und R² vorzugsweise diejenigen Bedeutungen, die bereits im Zusammenhang mit der Beschreibung der erfindungsgemäßen Verbindungen der Formel (I) für R¹ und R² als bevorzugt angegeben wurden.

Die Amine der Formel (III) sind teilweise bekannt.

Neu sind Amine der Formel (IIIa), 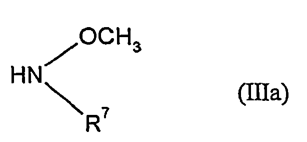 in welcher R⁷ für Isobutyl, 2-Methoxyethyl oder für 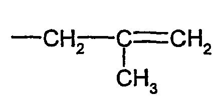 steht.

Die Amine der Formel (IIIa) lassen sich herstellen, indem man
e) in einer ersten Stufe N-Methoxycarbaminsäure-ethylester der Formel (VIII) 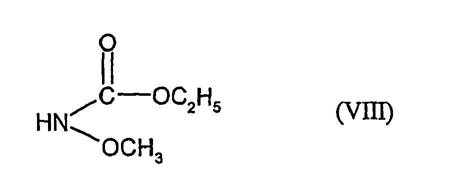 mit Halogenverbindungen der Formel (IX),

   R⁷-X¹ (IX)

   in welcher
   - R⁷: die oben angegebenen Bedeutungen hat und
   - X¹: für Brom oder Iod steht,
   in Gegenwart einer Base und in Gegenwart eines Verdünnungsmittels umsetzt und die entstehenden Carbamate der Formel (X), 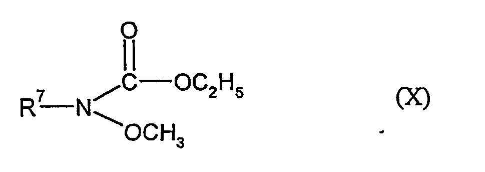 in welcher
   - R⁷: die oben angegebenen Bedeutungen hat,
   in einer zweiten Stufe mit Kaliumhydroxid in Gegenwart von Ethanol und Wasser umsetzt.

Neu sind auch Amine der Formel (IIIb), 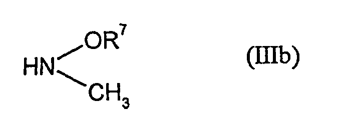 in welcher
- R⁷: die oben angegebenen Bedeutungen hat.

Die Amine der Formel (IIIb) lassen sich herstellen, indem man
f) in einer ersten Stufe N-Hydroxy-N-methyl-carbaminsäure-ethylester der Formel (XI), 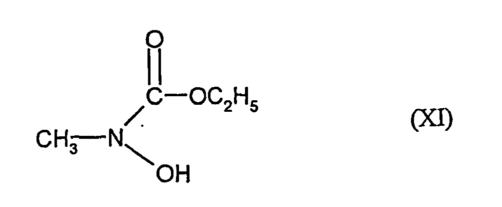 mit Halogenverbindungen der Formel (IX),

   R⁷-X¹ (IX)

   in welcher
   - R⁷ und X¹: die oben angegebenen Bedeutungen haben,
   in Gegenwart einer Base und in Gegenwart eines Verdünnungsmittels umsetzt und die entstehenden Carbamate der Formel (XII), 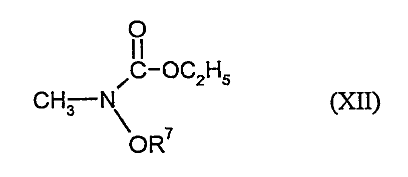 in welcher
   - R⁷: die oben angegebenen Bedeutungen hat,
   in einer zweiten Stufe mit Kaliumhydroxid in Gegenwart von Ethanol und Wasser umsetzt.

Neu sind auch Trifluorisopropylamine der Formel (IIIc), 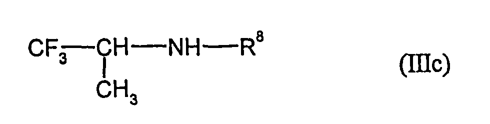 in welcher
- R⁸: für Methyl, Ethyl oder Propyl steht.

Die Trifluorisopropylamine der Formel (IIIc) lassen sich herstellen, indem man .
g) in einer ersten Stufe N-Trifluorisopropyl-carbaminsäure-ethylester der Formel (XIII), 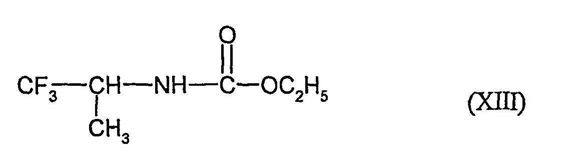 mit Halogenverbindungen der Formel (XIV),

   R⁸-X¹ (XIV)

   in welcher
   - R⁸ und X¹: die oben angegebenen Bedeutungen haben,
   in Gegenwart einer Base und in Gegenwart eines Verdünnungsmittels umsetzt und die entstehenden Carbamate der Formel (XV), 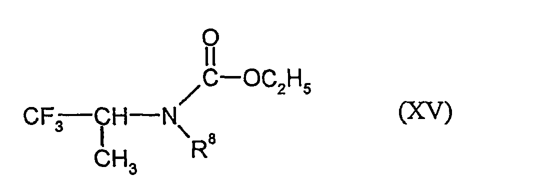 in welcher
   - R⁸: die oben angegebenen Bedeutungen hat,
   in einer zweiten Stufe mit Kaliumhydroxid in Gegenwart von Ethanol und Wasser umsetzt.

Neu ist schließlich auch das 3-Trifluor-methyl-3-amino-propen der Formel (III-4)

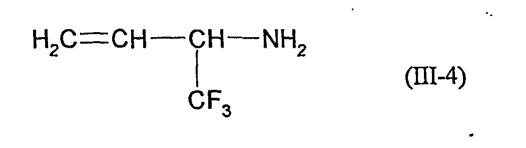

Das 3-Trifluormethyl-3-amino-propen der Formel (IIId) lässt sich herstellen, indem man
h) das Carbamat der Formel (XVI) 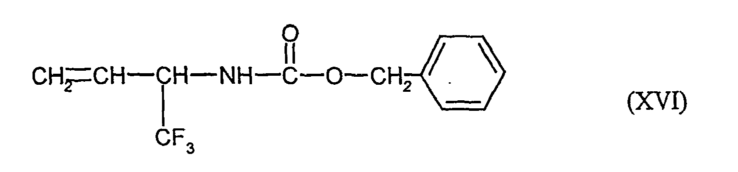 mit wässriger Salzsäure umsetzt.

Die bei der Durchführung der erfindungsgemäßen Verfahren (e)-(g) als Ausgangsstoffe benötigten Verbindungen der Formeln (VIII), (IX), (XI), (XIII), (XIV) und (XVI) sind bekannt oder lassen sich nach bekannten Methoden herstellen.

Bei der Durchführung der ersten Stufe der erfindungsgemäßen Verfahren (e), (f) und (g) kommen jeweils alle für derartige Umsetzungen üblichen anorganischen und organischen Säureakzeptoren in Frage.

Vorzugsweise verwendbar sind Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat und Natriumhydrogencarbonat, und außerdem Ammonium-Verbindungen, wie Ammoniumhydroxid, Ammoniumacetat und Ammoniumcarbonat.Als organische Basen seien genannt: tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel kommen bei der Durchführung der ersten Stufe der erfindungsgemäßen Verfahren (e), (f) und (g) jeweils alle üblichen inerten organischen Solventien in Betracht. Vorzugsweise verwendbar sind Ether, wie Diethylether, Dioxan, Tetrahydrofuran, 1,2-Dimethoxyethan, 1,2-Diethoxyethan oder Anisol; Amide, wie N,N-Dimethylformamid, N,N-Dimethylacetamid, N-Methylformanilid oder N-Methylpyrrolidon; Sulfone, wie Sulfolan; Alkohole wie Methanol, Ethanol, Isopropanol, tert.Butanol, n-Butanol.

Die Reaktionstemperaturen können bei der Durchführung der ersten Stufe der erfindungsgemäßen Verfahren (e) (f), und (g) jeweils innerhalb eines größeren Bereiches variiert werden. Im Allgemeinen arbeitet man bei Temperaturen zwischen 0°C und 150°C, vorzugsweise zwischen 10°C und 100°C.

Bei der Durchführung der ersten Stufe der erfindungsgemäßen Verfahren (e), (f) und (g) arbeitet man im allgemeinen jeweils unter Atmosphärendruck. Es ist jedoch auch möglich, unter erhöhtem Druck oder, sofern keine niedrig siedenden Komponenten an der Umsetzung beteiligt sind, unter vermindertem Druck zu arbeiten.

Bei der Durchführung der ersten Stufe der erfindungsgemäßen Verfahren (e), (f) und (g) setzt man
- auf 1 mol an N-Methoxy-carbaminsäure-ethylester der Formel (VIII) im Allgemeinen 0,5 bis 1,5 mol, vorzugsweise 1 bis 5 mol an Halogenverbindung der Formel (IX) ein, bzw.
- auf 1 mol an N-Hydroxy-N-methyl-carbaminsäure-ethylester der Formel (XI) im Allgemeinen 0,5 bis 15 mol, vorzugsweise 1 bis 5 mol an Halogenverbindung der Formel (IX) ein, bzw.
- auf 1 mol an N-Trifluorisopropyl-carbaminsäure-ethylester der Formel (XIII) im Allgemeinen 0,5 bis 15 mol, vorzugsweise 1 bis 5 mol an Halogenverbindung der Formel (XIV) ein.

Die Aufarbeitung erfolgt jeweils nach üblichen Methoden, beispielsweise durch Extraktion und anschließende Trocknung oder durch Fällung mit anschließender Filtration und Trocknung. Gegebenenfalls noch vorhandene Verunreinigungen können nach üblichen Methoden entfernt werden.

Die bei der Durchführung der ersten Stufe der erfindungsgemäßen Verfahren (e), (t) und (g) als Zwischenprodukte erhaltenen Verbindungen der Formeln (X), (XII) und (XV) sind neu.

Auch bei der Durchführung der zweiten Stufe der erfindungsgemäßen Verfahren (e), (f) und (g) können die Reaktionstemperaturen jeweils innerhalb eines größeren Bereiches variiert werden. Im Allgemeinen arbeitet man bei Temperaturen zwischen 0°C und 100°C, vorzugsweise zwischen 10°C und 80°C.

Auch bei der Durchführung der zweiten Stufe der erfindungsgemäßen Verfahren (e), (f) und (g) arbeitet man im Allgemeinen jeweils unter Atmosphärendruck. Es ist jedoch wiederum möglich, jeweils auch unter erhöhtem Druck oder, sofern die zu isolierenden Produkte keine sehr niedrigen Siedepunkte aufweisen, unter vermindertem Druck zu arbeiten.

Bei der Durchführung der zweiten Stufe der erfindungsgemäßen Verfahren (e), (f) und (g) setzt man auf 1 mol an einer Verbindung der Formel (X), (XII) der (XV) jeweils bis zu 10 mol an Kaliumhydroxid ein. Die Aufarbeitung erfolgt nach üblichen Methoden. Dabei werden die Amine zweckmäßigerweise im Allgemeinen durch Hinzufügen von Säure, vorzugsweise wässriger Salzsäure, in Form ihrer Salze isoliert.

Bei der Durchführung des erfindungsgemäßen Verfahrens (h) können die Reaktionstemperaturen ebenfalls in einem größeren Bereich variiert werden. Im allgemeinen arbeitet man bei Temperaturen zwischen 10°C und 150°C, vorzugsweise bei Rückflusstemperatur.

Im Allgemeinen arbeitet man bei der Durchführung des erfindungsgemäßen Verfahrens (h) unter Atmosphärendruck. Es ist aber auch möglich, unter erhöhtem Druck zu arbeiten.

Bei der Durchführung des erfindungsgemäßen Verfahrens (h) setzt man auf 1 mol an Carbamat der Formel (XVI) einem Überschuss, vorzugsweise bis zu 10 mol an wässriger Salzsäure ein. Die Aufarbeitung erfolgt wiederum nach üblichen Methoden.

Die bei der Durchführung des erfindungsgemäßen Verfahrens (b) als Ausgangsstoffe benötigten Triazolopyrimidine sind durch die Formel (Ia) allgemein definiert. In dieser Formel haben R², R³, R⁴ und X vorzugsweise diejenigen Bedeutungen, die bereits im Zusammenhang mit der Beschreibung der erfindungsgemäßen Stoffe der Formel (I) für diese Reste als bevorzugt genannt wurden.

Bei den Triazolopyrimidinen der Formel (Ia) handelt es sich um erfindungsgemäße Stoffe. Sie lassen sich nach dem erfindungsgemäßen Verfahren (a) herstellen.

Die weiterhin zur Durchführung des erfindungsgemäßen Verfahrens (b) als Ausgangsstoffe benötigten Sulfensäurehalogenide sind durch die Formel (IV) allgemein definiert. In dieser Formel hat R⁵ vorzugsweise diejenigen Bedeutungen, die bereits im Zusammenhang mit der Beschreibung der erfindungsgemäßen Verbindungen der Formel (I) für diesen Rest als bevorzugt genannt wurden.

Y² steht vorzugsweise für Fluor, Chlor oder Brom, besonders bevorzugt für Chlor.

Die Sulfensäurehalogenide der Formel (IV) sind bekannt oder lassen sich nach bekannten Methoden herstellen.

Als Verdünnungsmittel kommen bei der Durchführung des erfindungsgemäßen Verfahrens (a) alle üblichen, inerten organischen Solventien in Betracht. Vorzugsweise verwendbar sind aliphatische, alicyclische oder aromatische Kohlenwasserstoffe, wie Petrolether, Hexan, Heptan, Cyclohexan, Methylcyclohexan, Benzol, Toluol, Xylol oder Decalin; halogenierte Kohlenwasserstoffe, wie Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Dioxan, Tetrahydrofuran, 1,2- Dimethoxyethan oder 1,2-Diethoxyethan; Amide, wie N,N-Dimethylformamid, N,N-Dimethylacetamid oder N-Methylpyrrolidon; Ester wie Essigsäuremethylester oder Essigsäureethylester; Sulfoxide, wie Dimethylsulfoxid; Sulfone, wie Sulfolan.

Als Säureakzeptoren kommen bei der Durchführung des erfindungsgemäßen Verfahrens (a) alle für derartige Umsetzungen üblichen Säurebindemittel in Frage. Vorzugsweise verwendbar sind tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und 150°C, vorzugsweise bei Temperaturen zwischen 0°C und 80°C.

Sowohl bei der Durchführung des erfindungsgemäßen Verfahrens (a) als auch des Verfahrens (b) arbeitet man im allgemeinen unter Atmosphärendruck. Es ist aber auch möglich, unter erhöhtem oder vermindertem Druck, im allgemeinen zwischen 0,1 und 10 bar, zu arbeiten.

Bei der Durchführung des erfindungsgemäßen Verfahrens (a) setzt man auf 1 mol an Dihalogen-triazolo-pyrimidin der Formel (II) im allgemeinen 0,5 bis 10 mol, vorzugsweise 0,8 bis 2 mol an Amin der Formel (III) ein. Die Aufarbeitung erfolgt nach üblichen Methoden.

Zur Herstellung von Säureadditions-Salzen von Triazolopyrimidinen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B.

Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die erfindungsgemäßen Wirkstoffe weisen auch eine sehr gute stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokolation mit unerwünschten Mikroorgansimen weitgehende Resistenz gegen diese Mirkroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe eignen sich auch zur Steigerung des Emteertrages. Sie sind außerdem mindertoxisch und weisen eine gute Pflanzenverträglichkeit auf.

Die erfindungsgemäßen Wirkstoffe können in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich auch als Zwischen- und Vorprodukte für die Synthese weiterer Wirkstoffe einsetzen.

Mit den erfindungsgemäßen Wirkstoffen können Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die Behandlung der Pflanzen und Pflanzenteile mit den erfindungsgemäßen Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram, Carpropamid,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox, Fenhexamid,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione, Iprovalicarb,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Picoxystrobin, Pimaricin, Piperalin, Polyoxin, Polyoxorim, Probenazol, Prochloraz, Procymidon, Propamocarb, Propanosine-Natrium, Propiconazol, Propineb, Pyraclostrobin, Pyrazophos, Pyrifenox, Pyrimethanil, Pyroquilon, Pyroxyfur,
Quinconazol, Quintozen (PCNB), Quinoxyfen,
Schwefel und Schwefel-Zubereitungen, Spiroxamine,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Trifloxystrobin, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,

α-(1,1-Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-β-methoxy-a-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5,(1H-1,2,4-triazol-1-yl)-3-octanon,
(E)-a-(Methoxyimino)-N-methyl-2-phenoxy-phenylacetamid,
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-eyan-N,N-dimethyl-5-(4-methylphenyl)-1H-imidazol-l-sulfonamid,
4-Methyl-tetrazolo[1,5-a]quinazolin-5(4H)-on,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholin-hydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H,inden,1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioate,
S-Methyl-1,2,3-benzothiadiazol-7-carbothioat,
spiro[2H]-1-Benzopyran-2,1'(3'H)-isobenzofuran]-3'-on,
4-[3,4-Dimethoxyphenyl)-3-(4-fluorphenyl)-acryloyl]-morpholin

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, Bistrifluron, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Chromafenozide, Cis-Resmethrin, Cispermethrin, Clocythrin, Cloethocarb, Clofentezine, Clothianidine, Cyanophos, Cycloprene, Cycloprothrin, Cyfluthrin, Cyhalothrin, Cyhexatin, Cypermethrin, Cyromazine,
Deltamethrin, Demeton M, Demeton S, Demeton-S-methyl, Diafenthiuron, Diazinon, Dichlorvos, Dicofol, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Esfenvalerate, Ethiofencarb, Ethion, Ethoprophos, Etofenprox, Etoxazole, Etrimfos,
Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazuron, Flubrocythrinate, Flucycloxuron, Flucythrinate, Flufenoxuron, Flumethrin, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene,
Imidacloprid, Indoxacarb, Isazofos, Isofenphos, Isoxathion, Ivermectin,
Kernpolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Methoprene, Methomyl, Methoxyfenozide, Metolcarb, Metoxadiazone, Mevinphos, Milbemectin, Milbemycin, Monocrotophos,
Naled, Nitenpyram, Nithiazine, Novaluron
Omethoat, Oxamyl, Oxydemethon M
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propargite, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin
Salithion, Sebufos, Silafluofen, Spinosad, Spirodiclofen, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Tetradifon, Thetacypermethrin, Thiacloprid, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii
YI 5302
Zeta-cypermethrin, Zolaprofos

(1R-cis)-[5-(Phenylmethyl)-3-furanyl]-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat
1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol
2-(Acetlyoxy)-3-dodecyl-1,4-naphthalindion
2-Chlor-N-[[[4-(1-phenylethoxy)-phenyl]-amino]-carbonyl]-benzamid
2-Chlor-N-[[[4-(2,2-dichlor-1,1-difluorethoxy)-phenyl]-amino]-carbonyl]-benzamid
3-Methylphenyl-propylcarbamat
4-[4-(4-Ethoxyphenyl)-4-methylpentyl]-1-fluor-2-phenoxy-benzol
4-Chlor-2-(1,1-dimethylethyl)-5-[[2-(2,6-dimethyl-4-phenoxyphenoxy)ethyl]thio]-3(2H)-pyridazinon
4-Chlor-2-(2-chlor-2-methylpropyl)-5-[(6-iod-3-pyridinyl)methoxy]-3(2H)-pyridazinon
4-Chlor-5-[(6-chlor-3-pyridinyl)methoxy]-2-(3,4-dichlorphenyl)-3(2H)-pyridazinon Bacillus thuringiensis strain EG-2348
Benzoesäure [2-benzoyl-1-(1,1-dimethylethyl)-hydrazid
Butansäure 2,2-dimethyl-3-(2,4-dichlorphenyl)-2-oxo-1-oxaspiro[4.5]dec-3-en-4-yl-ester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylmethyl)-4-pyridazinyl]oxy]ethyl]-carbamat
N-(3,4,4-Trifluor-1-oxo-3-butenyl)-glycin
N-(4-Chlorphenyl)-3-[4-(difluormethoxy)phenyl]-4,5-dihydro-4-phenyl-1H-pyrazol-1-carboxamid
N-[(2-Chlor-5-thiazolyl)methyl]-N'-methyl-N"-nitro-guanidin
N-Methyl-N'-( 1-methyl-2-propenyl)-1,2-hydrazindicarbothioamid
N-Methyl-N'-2-propenyl-1,2-hydrazindicarbothioamid
O,O-Diethyl-[2-(dipropylamino)-2-oxoethyl]-ethylphosphoramidothioat
N-Cyanomethyl-4-trifluormethyl-nicotinamid
3,5-Dichlor-1-(3,3-dichlor-2-propenyloxy)-4-[3-(5-trifluormethylpyridin-2-yloxy)-propoxy]-benzol

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können mit erfindungsgemäßen Wirkstoffen alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft mit den erfindungsgemäßen Verbindungen der Formel (I) behandelt werden. Die bei den Wirkstoffen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen.

Die Erfindung wird durch die folgenden Beispiele veranschaulicht.

### Herstellungsbeispiele

### Beispiel 1

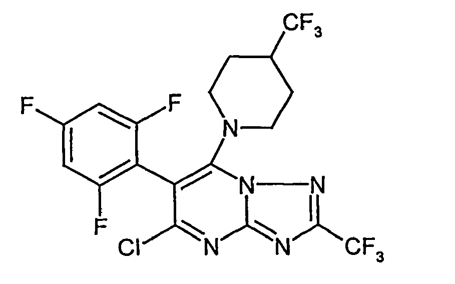

### Verfahren (a)

In eine Lösung von 0,7 g (181 mMol) 5,7-Dichlor-2-(trifluormethyl)-6-(2,4,6-trifluorphenyl)[1,2,4]triazolo[1,5-a]pyrimidin und 0,28 g (1,81 mMol) 4-Trifluormethylpiperidin in 20 ml Dichlormethan werden 0,18 g Triethylamin gegeben. Das Gemisch wird 18 Stunden bei Raumtemperatur gerührt. Danach wird das Reaktionsgemisch mit soviel 1N Salzsäure versetzt und gerührt, dass der pH-Wert der Mischung bei 1 - 2 liegt (ca. 50 ml). Die organische Phase wird abgetrennt, über Natriumsulfat getrocknet und unter vermindertem Druck eingeengt. Der Rückstand wird mit Petrolether verrührt und abgesaugt. Man erhält 0,3 g (30,3 % der Theorie) 5-Chlor-2-(trifluormethyl)-7-[4-trifluormethyl)-1-piperidinyl]-6-(2,4,6-trifluorphenyl)[1,2,4]-triazolo[1,5-a]pyrimidin.
HPLC: logP = 4,43

Nach den zuvor angegebenen Methoden werden auch die in der nachstehenden Tabelle 1 aufgeführten Verbindungen der Formel (I) erhalten.

**Tabelle 1**

| | | | | | | | | |
|---|---|---|---|---|---|---|---|---|
| 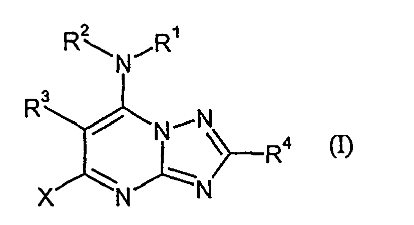 | | | | | | | | |

| Bsp. Nr. | R¹ | R² | R³ | R⁴ | X | Isomer | logP | Fp.: (°C) |
|---|---|---|---|---|---|---|---|---|
| 1 | -CH₂-CH₂-CH(CF₃)-CH₂-CH₂- | | 2,4,6-Trifluorphenyl | -CF₃ | Cl | | 4,43 | |
| 2 | -CH₂-CH₂-CH(CF₃)-CH₂-CH₂- | | 2,4,6-Trifluorphenyl | -C₂H₅ | Cl | | 3,99 | |
| 3 | 2,2,2-Trifluor-1-methylethyl | -H | 2,4,6-Trifluorphenyl | -CF₃ | Cl | | 3,94 | |
| 4 | 2,2,2-Trifluor-1-methylethyl | -H | 2,4,6-Trifluorphenyl | -C₂H₅ | Cl | | 3,39 | |
| 5 | -CH₂-CF₃ | -H | 2,4,6-Trifluorphenyl | -C₂H₅ | Cl | | 3,06 | |
| 6 | -CH₂-CH₂-CF₃ | -H | 2,4,6-Trifluorphenyl | -C₂H₅ | Cl | | 3,18 | |
| 7 | -CH₂-CH₂-CF₃ | -H | 2,4,6-Trifluorphenyl | -CF₃ | Cl | | 3,76 | |
| 8 | -CH₂-CF₃ | -H | 2,4,6-Trifluorphenyl | -CF₃ | Cl | | 3,64 | |
| 9 | 2,2,2-Trifluor-1-methylethyl | -H | 2,4,6-Trifluorphenyl | t-Butyl | Cl | | 4,24 | |
| 10 | -CH₂-C(CH₃)=CH₂ | -C₂H₅ | 2,4,6-Trifluorphenyl | -CF₃ | Cl | | 4,73 | |
| 11 | -i-Propyl | -H | 2,4,6-Trifluorphenyl | -CF₃ | Cl | | 3,8 | |
| 12 | 2,2,2-Trifluor-1-methylethyl | -H | 2,4,6-Trifluorphenyl | -CH₃ | Cl | | 3,01 | |
| 13 | -CH₂-CF₃ | -H | 2,4,6-Trifluorphenyl | -CH₃ | Cl | | 2,69 | |
| 14 | -CH₂-C(CH₃)=CH₂ | -C₂H₅ | 2,4,6-Trifluorphenyl | -CH₃ | Cl | | 3,83 | |
| 15 | -CH₂-C(CH₃)=CH₂ | -C₂H₅ | 2,4,6-Trifluorphenyl | -C₂H₅ | Cl | | 4,34 | |
| 16 | -CH₂-C(CH₃)=CH₂ | -H | 2,4,6-Trifluorphenyl | -CH₃ | Cl | | 2,76 | |
| 17 | -i-Propyl | -H | 2-Chlorphenyl | Cyclopropyl | Cl | | 3,39 | |
| 18 | -CH₂-CN | -H | 2,4,6-Trifluorphenyl | -CH₃ | Cl | | 2,02 | |
| 19 | 2,2,2-Trifluor-1-methylethyl | -H | 2-Chlorphenyl | Cyclopropyl | Cl | AS | 3,59 | |
| 20 | 2,2,2-Trifluor-1-methylethyl | -H | 2-Chlorphenyl | Cyclopropyl | Cl | BS | 3,61 | |
| 21 | -CH₂-C(CH₃)=CH₂ | -H | 2,4,6-Trifluorphenyl | -C₂H₅ | Cl | | 3,15 | |
| 22 | -CH₂-C(CH₃)=CH₂ | -H | 2,4,6-Trifluorphenyl | -CF₃ | Cl | | 3,82 | |
| 23 | -CH₂-CN | -H | 2,4,6-Trifluorphenyl | -C₂H₅ | Cl | | 2,27 | |
| 24 | -CH₂-CN | -H | 2,4,6-Trifluorphenyl | -CF₃ | Cl | | 2,91 | |
| 25 | 2,2,2-Trifluor-1-methylethyl | -H | 2,4,6-Trifluorphenyl | -CF₃ | Br | | 3,99 | |
| 26 | -i-Propyl | -H | 2,4,6-Trifluorphenyl | -CH₃ | Cl | | 2,71 | |
| 27 | -NH₂ | -i-Propyl | 2,4,6-Trifluorphenyl | -CH₃ | Cl | | 2,77 | |
| 28 | i-Propylamino | -H | 2,4,6-Trifluorphenyl | -CH₃ | Cl | | 2,7 | |
| 29 | 2-Methoxyethyl | -C₂H₅ | 2,4,6-Trifluorphenyl | -CH₃ | Cl | | 2,93 | |
| 30 | 2-Methoxyethyl | -C₂H₅ | 2,4,6-Trifluorphenyl | -C₂H₅ | Cl | | 3,37 | |
| 31 | 2-Methoxyethyl | -C₂H₅ | 2,4,6-Trifluorphenyl | -CF₃ | Cl | | 4,04 | |
| 32 | Cyclopentyl | -H | 2,4,6-Trifluorphenyl | -CF₃ | Cl | | 4,25 | |
| 33 | -C₂H₅ | -C₂H₅ | 2,4,6-Trifluorphenyl | -CF₃ | Cl | | 4,25 | |
| 34 | -CH₂-CH₂-O-CH₂-CH₂- | | 2,4,6-Trifluorphenyl | -CF₃ | Cl | | 3,22 | |
| 35 | Cyclopropyl | -H | 2,4,6-Trifluorphenyl | -CF₃ | Cl | | 3,53 | |
| 36 | -CH₃ | -CH₃ | 2-Chlorphenyl | Cyclopropyl | Cl | | 3,13 | |
| 37 | -C₂H₅ | -H | 2-Chlorphenyl | Cyclopropyl | Cl | | | |
| 38 | -CH₂-CH₂-CH₂-CH₂- | | 2-Chlorphenyl | Cyclo-propyl | Cl | | | |
| 39 | -CH₂-CN -H | | 2-Chlorphenyl | Cyclopropyl | Cl | | | |
| 40 | Cyclopentyl | -H | 2-Chlorphenyl | Cyclopropyl | Cl | | | |
| 41 | -C₂H₅ | -C₂H₅ | 2-Chlorphenyl | Cyclopropyl | Cl | | | |
| 42 | -CH₂-CH₂-O-CH₂-CH₂- | | 2-Chlorphenyl | Cyclopropyl | Cl | | | |
| 43 | 2-Methoxyethyl | -H | 2-Chlorphenyl | Cyclopropyl | Cl | | | |
| 44 | -CH₃ | -H | 2-Chlorphenyl | Cyclopropyl | Cl | | | |
| 45 | -CH₂-CH₂-S-CH₂-CH₂- | | 2-Chlorphenyl | Cyclopropyl | Cl | | | |
| 46 | -CH₂-CH₂-CH(CH₃)-CH₂-CH₂- | | 2-Chlorphenyl | Cyclopropyl | Cl | | | |
| 47 | Cyclohexyl | -H | 2-Chlorphenyl | Cyclopropyl | Cl | | | |
| 48 | Cyclopropylmethyl | -H | 2-Chlorphenyl | Cyclopropyl | Cl | | | |
| 49 | i-Propylamino | -H | 2,4,6- Trifluorphenyl | -C₂H₅ | Cl | | 3,05 | |
| 50 | 1-Cyclopropylethylamino | -H | 2-Chlorphenyl | Cyclopropyl | Cl | | | |
| 51 | n-Butylamino | -H | 2-Chlorphenyl | Cyclopropyl | Cl | | | |
| 52 | -NH-CH₂-CF₂-CHF₂ | -H | 2-Chlorphenyl | Cyclopropyl | Cl | | | |
| 53 | -NH-CH₂-CH(CH₃)₂ -H | | 2-Chlorphenyl | Cyclopropyl | Cl | | | |
| 54 | Allylamino | -H | 2-Chlorphenyl | Cyclopropyl | Cl | | | |
| 55 | -NH-CH₂-CF₃ | -H | 2-Chlorphenyl | Cyclopropyl | Cl | | | |
| 56 | i-Propylamino | -H | 2-Chlorphenyl | Cyclopropyl | Cl | | | |
| 57 | t-Butylamino | -H | 2-Chlorphenyl | Cyclopropyl | Cl | | | |
| 58 | -CH₂-CH(CH₃)-O-CH(CH₃)-CH₂- | | 2,4,6-Trifluorphenyl | -CH₃ | Cl | | 3 | |
| 59 | -CH₂-CH₂-CH₂-CH₂- | | 2,4,6-Trifluorphenyl | -CH₃ | Cl | | 2,86 | |
| 60 | -CH₂-CH₂-CH₂-CH₂-CH₂- | | 2,4,6-Trifluorphenyl | -CH₃ | Cl | | 3,43 | |
| 61 | n-Propyl | -H | 2,4,6-Trifluorphenyl | -CH₃ | Cl | | 2,75 | |
| 62 | Cyclopentyl | -H | 2,4,6-Trifluorphenyl | -CH₃ | Cl | | 3,23 | |
| 63 | -C₂H₅ | -C₂H₅ | 2,4,6-Trifluorphenyl | -CH₃ | Cl | | 3,23 | |
| 64 | 2-Methoxyethyl | -H | 2,4,6-Trifluorphenyl | -CH₃ | Cl | | 2,34 | |
| 65 | Cyclopropyl | -H | 2,4,6-Trifluorphenyl | -CH₃ | Cl | | 2,51 | |
| 66 | -CH₂-CH₂-S-CH₂-CH₂- | | 2,4,6-Trifluorphenyl | -CH₃ | Cl | | 3,09 | |
| 67 | -CH₂-CH₂-CH(CF₃)-CH₂-CH₂- | | 2,4,6- Trifluorphenyl | -CH₃ | Cl | | 3,6 | |
| 68 | Cyclopropylmethyl | -H | 2,4,6-Trifluorphenyl | -CH₃ | Cl | | 2,86 | |
| 69 | 2,2,2-Trifluor-1-methylethyl | -H | 2,4,6-Trifluorphenyl | -CH₃ | Cl | | 3,04 | |
| 70 | -CH₂-CH₂-CF₃ | -H | 2,4,6-Trifluorphenyl | -CH₃ | Cl | | 2,85 | |
| 71 | -CH₂-CH(CH₃)-O-CH(CH₃)-CH₂- | | 2,4,6-Trifluorphenyl | -C₂H₅ | Cl | | 3,57 | |
| 72 | -CH₂-CH₂-CH₂-CH₂- | | 2,4,6-Trifluorphenyl | -C₂H₅ | Cl | | 3,29 | |
| 73 | -CH₂-CH₂-CH₂-CH₂-CH₂- | | 2,4,6-Trifluorphenyl | -C₂H₅ | Cl | | 3,94 | |
| 74 | n-Propyl | -H | 2,4,6-Trifluorphenyl | -C₂H₅ | Cl | | 3,14 | |
| 75 | Cyclopentyl | -H | 2,4,6-Trifluorphenyl | -C₂H₅ | Cl | | 3,66 | |
| 76 | -i-Propyl | -H | 2,4,6-Trifluorphenyl | -C₂H₅ | Cl | | 3,15 | |
| 77 | -C₂H₅ | -C₂H₅ | 2,4,6-Trifluorphenyl | -C₂H₅ | Cl | | 3,71 | |
| 78 | 2-Methoxyethyl | -H | 2,4,6-Trifluorphenyl | -C₂H₅ | Cl | | 2,68 | |
| 79 | Cyclopropyl | -H | 2,4,6-Trifluorphenyl | -C₂H₅ | Cl | | 2,85 | |
| 80 | -CH₂-CH₂-S-CH₂-CH₂- | | 2,4,6-Trifluorphenyl | -C₂H₅ | Cl | | 3,53 | |
| 81 | Cyclopropylmethyl -H | | 2,4,6- Trifluorphenyl | -C₂H₅ | Cl | | 3,23 | |
| 82 | -i-Propyl | -H | 3-Chlor-4-fluorphenyl | -CH₃ | Cl | | 3,01 | |
| 83 | -CH₂-CH₂-CH=CH-CH₂- | | 2,4,6-Trifluorphenyl | -CH₃ | Cl | | 3,2 | |
| 84 | i-Propylamino | -H | 2,4,6-Trifluorphenyl | -CF₃ | Cl | | 3,68 | |
| 85 | -CH₂-CH₂-CH=C(CH₃)-CH₂- | | 2,4,6-Trifluorphenyl | -CH₃ | Cl | | 3,57 | |
| 86 | -CH₃ | -CH₃ | 2,4,6-Trifluorphenyl | -CH₃ | Cl | | 2,49 | |
| 87 | -C₂H₅ | -H | 2,4,6-Trifluorphenyl | -CH₃ | Cl | | 2,44 | |
| 88 | -C(CH₃)₂-CF₃ | -H | 2,4,6- Trifluorphenyl | -CH₃ | Cl | | 3,7 | |
| 89 | -CH₂-CH₂-O-CH₂-CH₂- | | 2,4,6-Trifluorphenyl | -CH₃ | Cl | | 2,43 | |
| 90 | -CH₃ | -H | 2,4,6- Trifluorphenyl | -CH₃ | Cl | | 2,12 | |
| 91 | -C₂H₅ | -H | 2,4,6-Trifluorphenyl | -C₂H₅ | Cl | | 2,78 | |
| 92 | -CH₂-CH₂-O-CH₂-CH₂- | | 2,4,6- Trifluorphenyl | -C₂H₅ | Cl | | 2,8 | |
| 93 | -CH₃ | -H | 2,4,6-Trifluorphenyl | -C₂H₅ | Cl | | 2,44 | |
| 94 | -CH(CF₃)-CH₂-CH₂-CH₂-CH₂- | | 2,4,6-Trifluorphenyl | -C₂H₅ | Cl | | 4,27 | |
| 95 | -CH₂-C(CH₃)=CH₂ -C₂H₅ | | 2,4-Difluorphenyl | -CF₃ | Cl | | 4,69 | 123-25 |
| 96 | -CH₂-C(CH₃)=CH₂ | -C₂H₅ | 2,4-Difluorphenyl | -CH₃ | Cl | | 3,73 | 100-02 |
| 97 | 2,2,2-Trifluor-1-methylethyl | -H | 3-Chlor-4-fluorphenyl | -CH₃ | Cl | | 3,32 | |
| 98 | -CH₂-CH(OH)-CH₂-CH₂- | | 2,4,6-Trifluorphenyl | -CH₃ | Cl | | 1,85 | |
| 99 | -CH₂-CH(OH)-CH₂-CH₂-CH₂- | | 2,4,6- Trifluorphenyl | -CH₃ | Cl | | 2,15 | |
| 100 | -CH₃ | -CH₃ | 2,4,6-Trifluorphenyl | -CF₃ | Cl | | 3,6 | |
| 101 | -CH₂-CH(CH₃)-O-CH(CH₃)-CH₂- | | 2,4,6-Trifluorphenyl | -CF₃ | Cl | | 4,18 | |
| 102 | -C₂H₅ | -H | 2,4,6-Trifluorphenyl | -CF₃ | Cl | | 3,5 | |
| 103 | -CH₂-CH₂-CH₂-CH₂- | | 2,4,6-Trifluorphenyl | -CF₃ | Cl | | 3,92 | |
| 104 | -CH₂-CH₂-CH₂-CH₂-CH₂- | | 2,4,6-Trifluorphenyl | -CF₃ | Cl | | 4,46 | |
| 105 | -C(CH₃)₂-CF₃ | -H | 2,4,6-Trifluorphenyl | -CF₃ | Cl | | 4,37 | |
| 106 | n-Propyl | -H | 2,4,6-Trifluorphenyl | -CF₃ | Cl | | 3,82 | |
| 107 | 2-Methoxyethyl | -H | 2,4,6-Trifluorphenyl | -CF₃ | Cl | | 3,38 | |
| 108 | -CH₃ | -H | 2,4,6-Trifluorphenyl | -CF₃ | Cl | | 3,17 | |
| 109 | -CH(CF₃)-CH₂-CH₂-CH₂-CH₂- | | 2,4,6-Trifluorphenyl | -CF₃ | Cl | | 4,76 | |
| 110 | -CH₂-CH₂-S-CH₂-CH₂- | | 2,4,6- Trifluorphenyl | -CF₃ | Cl | | 4,09 | |
| 111 | Cyclopropylmethyl | -H | 2,4,6-Trifluorphenyl | -CF₃ | Cl | | 3,89 | |
| 112 | -CH₂-S-CH₂-CH₂- | | 2,4,6-Trifluorphenyl | -CH₃ | Cl | | 2,84 | |
| 113 | 2,2,2-Trifluor-1-methylethyl | -H | 2,4-Difluorphenyl | -CH₃ | Cl | | 3,03 | |
| 114 | -CH₂-C(CH₃)=CH₂ | -C₂H₅ | 2-Chlor-6-Fluorphenyl | -CH₃ | Cl | | 3,91 | |
| 115 | -CH(CH₂OH)-COOCH₃ | -H | 2,4,6-Trifluorphenyl | -CH₃ | Cl | | 1,93 | |
| 116 | -CH(CH₃)-CH₂-O-CH₃ | -H | 2,4,6-Trifluorphenyl | -CH₃ | Cl | | 2,64 | |
| 117 | -CH(CH₃)-CH=CH-CH(CH₃)- | | 2,4,6-Trifluorphenyl | -CH₃ | Cl | | 3,52 | |
| 118 | AB3 | -H | 2,4,6-Trifluorphenyl | -CH₃ | Cl | | 3,11 | |
| 119 | AB4 | | 2,4,6-Trifluorphenyl | -CH₃ | Cl | | 2,65 | |
| 120 | AB5 | -H | 2,4,6-Trifluorphenyl | -CH₃ | Cl | | 3,47 | |
| 121 | AB6 | | 2,4,6-Trifluorphenyl | -CH₃ | Cl | | 2,13 | |
| 122 | -CH(CH₃)-CH₂-OH | -H | 2,4,6-Trifluorphenyl | -CH₃ | Cl | | 1,93 | |
| 123 | -CH(CH₃)-CH(O-CH₃)₂ -H | | 2,4,6-Trifluorphenyl | -CH₃ | Cl | | 2,74 | |
| 124 | -CH(CH₃)-COOCH₃ | -H | 2,4,6-Trifluorphenyl | -CH₃ | Cl | | 2,45 | |
| 125 | -CH₂-COOCH₃ | -H | 2,4,6-Trifluorphenyl | -CH₃ | Cl | | 2,13 | |
| 126 | -CH(CH₃)-COO-t-butyl | -H | 2,4,6-Trifluorphenyl | -CH₃ | Cl | | 3,44 | |
| 127 | -NH₂ | i-Butyl | 2,4,6-Trifluorphenyl | -C₂H₅ | Cl | | 3,47 | 176-78 |
| 128 | 2-Methoxyethyl | -C₂H₅ | 2,4-Difluorphenyl | -CH₃ | Cl | | 2,84 | |
| 129 | -NH₂ | i-Butyl | 2,4,6-Trifluorphenyl | -CH₃ | Cl | | 3,06 | Paste |
| 130 | -NH₂ | i-Butyl | 2-Chlor-6-Fluorphenyl | -CH₃ | Cl | | 3,12 | 157-8 |
| 131 | -NH₂ | i-Butyl | 3-Chlor-4-fluorphenyl | -CH₃ | Cl | | 3,31 | 155-8 |
| 132 | 2,2,2-Trifluor-1-methylethyl | -H | 2-Chlor-6-Fluorphenyl | -CH₃ | Cl | | 3,01 | |
| 133 | -CH₂-C(CH₃)=CH₂ | -C₂H₅ | 2-Chlorphenyl | -CH₃ | Cl | | 3,9 | |
| 134 | 1,2-Dimethylpropyl | -H | 2,4,6-Trifluorphenyl | -CH₃ | Cl | | 3,37 | |
| 135 | i-Butoxy | -H | 2,6-Difluorphenyl | -CH₃ | Cl | | 2,88 | |
| 136 | -O-C₂H₅ | -H | 2,6-Difluorphenyl | -CH₃ | Cl | | 2,22 | |
| 137 | 3-Chlorbenzyloxy | -H | 2,6-Difluorphenyl | -CH₃ | Cl | | 3,21 | |
| 138 | 4-Chlorbenzyloxy | -H | 2,6- Difluorphenyl | -CH₃ | Cl | | 3,23 | |
| 139 | -O-CH(CH₃)-CH₂-CH₃ | -H | 2,6-Difluorphenyl | -CH₃ | Cl | | 2,78 | |
| 140 | Allyloxy | -H | 2,6-Difluorphenyl | -CH₃ | Cl | | 2,38 | |
| 141 | t-Butoxy | -H | 2,6-Difluorphenyl | -CH₃ | Cl | | 2,68 | |
| 142 | 2-Hexahydropyranyloxy | -H | 2,6-Difluorphenyl | -CH₃ | Cl | | 2,54 | |
| 143 | -O-CH₃ | -CH₃ | 2,6-Difluorphenyl | -CH₃ | Cl | | 2,54 | |
| 144 | O-i-Propyl | -H | 2,6-Difluorphenyl | -CH₃ | Cl | | 2,46 | |
| 145 | i-Butyl | -H | 2,4,6-Trifluorphenyl | -CH₃ | Cl | | 3,14 | |
| 146 | -CH₂-C(CH₃)₃ | -H | 2,4,6-Trifluorphenyl | -CH₃ | Cl | | 3,5 | |
| 147 | 2-Butyl | -H | 2,4,6-Trifluorphenyl | -CH₃ | Cl | | 3,1 | |
| 148 | -CH₂-CH₂-N(CH₃)₂ | -CH₃ | 2,4,6-Trifluorphenyl | -CH₃ | Cl | | 1,5 | |
| 149 | Propargyl | -CH₃ | 2,4,6-Trifluorphenyl | -CH₃ | Cl | | 2,77 | |
| 150 | -CH₂-COOC₂H₅ | -CH₃ | 2,4,6-Trifluorphenyl | -CH₃ | Cl | | 2,8 | |
| 151 | 1,3-Dioxolan-2-ylmethyl | -CH₃ | 2,4,6-Trifluorphenyl | -CH₃ | Cl | | 2,67 | |
| 152 | Allyl | -CH₃ | 2,4,6-Trifluorphenyl | -CH₃ | Cl | | 3,18 | |
| 153 | (2-Furyl)methyl | -CH₃ | 2,4,6-Trifluorphenyl | -CH₃ | Cl | | 3,22 | |
| 154 | i-Butyl | -CH₃ | 2,4,6-Trifluorphenyl | -CH₃ | Cl | | 3,63 | |
| 155 | -CH₂-C(CH₃)=CH₂ | -CH₃ | 2,4,6-Trifluorphenyl | -CH₃ | Cl | | 3,55 | |
| 156 | -CH₂-CH₂-N(CH₃)₂ | -C₂H₅ | 2,4,6-Trifluorphenyl | -CH₃ | Cl | | 1,59 | |
| 157 | Allyl | -C₂H₅ | 2,4,6-Trifluorphenyl | -CH₃ | Cl | | 3,5 | |
| 158 | (2-Furyl)methyl | -C₂H₅ | 2,4,6-Trifluorphenyl | -CH₃ | Cl | | 3,55 | |
| 159 | (2-Tetrahydrofuryl)methyl | -C₂H₅ | 2,4,6-Trifluorphenyl | -CH₃ | Cl | | 3,26 | |
| 160 | -CH₂-COOC₂H₅ | -C₂H₅ | 2,4,6-Trifluorphenyl | -CH₃ | Cl | | 3,14 | |
| 161 | n-Butyl | -C₂H₅ | 2,4,6-Trifluorphenyl | -CH₃ | Cl | | 4,08 | |
| 162 | Cyclopropylmethyl | n-Propyl | 2,4,6-Trifluorphenyl | -CH₃ | Cl | | 4,08 | |
| 163 | (2-Tetrahydrofuryl)methyl | n-Propyl | 2,4,6-Trifluorphenyl | -CH₃ | Cl | | 3,68 | |
| 164 | -CH₂-CH₂-CH₂-CH₂-CH(CH₃)- | | 2,4,6-Trifluorphenyl | -CH₃ | Cl | | 3,81 | |
| 165 | -CH₂-CH₂-CH₂-CH(CH₃)-CH₂- | | 2,4,6- Trifluorphenyl | -CH₃ | Cl | | 3,9 | |
| 166 | -CH₂-CH₂-CH(CH₃)-CH₂-CH₂- | | 2,4,6- Trifluorphenyl | -CH₃ | Cl | | 3,9 | |
| 167 | -CH₂-CH₂-CHF-CH₂-CH₂- | | 2,4,6-Trifluorphenyl | -CH₃ | Cl | | 3,03 | |
| 168 | AB7 | | 2,4,6-Trifluorphenyl | -CH₃ | Cl | | 3,73 | |
| 169 | -CH₂-CH(CH₃)-O-CH(CH₃)-CH₂- | | 2,6-Difluorphenyl | -CH₃ | Cl | | 2,84 | |
| 170 | -CH₂-CH₂-CH₂-CH₂- | | 2,6-Difluorphenyl | -CH₃ | Cl | | 2,61 | |
| 171 | -CH₂-CH₂-CH₂-CH₂-CH₂- | | 2,6-Difluorphenyl | -CH₃ | Cl | | 3,17 | |
| 172 | n-Propyl | -H | 2,6-Difluorphenyl | -CH₃ | Cl | | 2,52 | |
| 173 | Cyclopentyl | -H | 2,6-Difluorphenyl | -CH₃ | Cl | | 2,97 | |
| 174 | -i-Propyl | -H | 2,6-Difluorphenyl | -CH₃ | Cl | | 2,52 | |
| 175 | -C₂H₅ | -C₂H₅ | 2,6-Difluorphenyl | -CH₃ | Cl | | 2,98 | |
| 176 | -CH₂-CH₂-O-CH₂-CH₂- | | 2,6-Difluorphenyl | -CH₃ | Cl | | 2,2 | |
| 177 | 2-Methoxyethyl | -H | 2,6-Difluorphenyl | -CH₃ | Cl | | 2,12 | |
| 178 | Cyclopropyl | -H | 2,6-Difluorphenyl | -CH₃ | Cl | | 2,28 | |
| 179 | -CH₂-CH₂-S-CH₂-CH₂- | | 2,6-Difluorphenyl | -CH₃ | Cl | | 2,84 | |
| 180 | -CH₂-CH₂-CH(CF₃)-CH₂-CH₂- | | 2,6-Difluorphenyl | -CH₃ | Cl | | 3,36 | |
| 181 | Cyclopropylmethyl | -H | 2,6-Difluorphenyl | -CH₃ | Cl | | 2,62 | |
| 182 | 2,2,2-Trifluor-1-methylethyl | -H | 2,6-Difluorphenyl | -CH₃ | Cl | | 2,78 | |
| 183 | -CH₂-C(CH₃)=CH₂ | -H | 2,6-Difluorphenyl | -CH₃ | Cl | | 2,56 | |
| 184 | -CH₂-CH₂-CF₃ | -H | 2,6-Difluorphenyl | -CH₃ | Cl | | 2,63 | |
| 185 | 1-Cyclohexylethyl | -H | 2,6-Difluorphenyl | -CH₃ | Cl | | 4 | |
| 186 | Cyclohexyl | -H | 2,6-Difluorphenyl | -CH₃ | Cl | | 3,28 | |
| 187 | 2-Butyl | -H | 2,6-Difluorphenyl | -CH₃ | Cl | | 2,82 | |
| 188 | 3-Trifluormethylcyclohexyl | -H | 2,6- Difluorphenyl | -CH₃ | Cl | | 3,37 | |
| 189 | 3,5-bis-Trifluormethylcyclohexyl | -H | 2,6-Difluorphenyl | -CH₃ | Cl | | 3,62 | |
| 190 | 4-Trifluormethylcyclohexyl | -H | 2,6-Difluorphenyl | -CH₃ | Cl | | 3,39 | |
| 191 | i-Butyl | -H | 2,4-Difluorphenyl | -CH₃ | Cl | | 3 | |
| 192 | n-Butyl | -H | 2,4-Difluorphenyl | -CH₃ | Cl | | 3,04 | |
| 193 | -CH₂-C(CH₃)₃ | -H | 2,4-Difluorphenyl | -CH₃ | Cl | | 3,4 | |
| 194 | 2-Butyl | -H | 2,4-Difluorphenyl | -CH₃ | Cl | | 3,01 | |
| 195 | -CH₂-CH₂-CF₃ | -H | 2,4-Difluorphenyl | -CH₃ | Cl | | 2,76 | |
| 196 | -i-Propyl | -H | 2,4-Difluorphenyl | -CH₃ | Cl | | 2,69 | |
| 197 | Cyclohexyl | -H | 2,4-Difluorphenyl | -CH₃ | Cl | | 3,46 | |
| 198 | 1-Cyclohexylethyl | -H | 2,4-Difluorphenyl | -CH₃ | Cl | | 4,24 | |
| 199 | Cyclopropyl | -H | 2,4-Difluorphenyl | -CH₃ | Cl | | 2,45 | |
| 200 | Cyclopropylmethyl | -H | 2,4-Difluorphenyl | -CH₃ | Cl | | 2,77 | |
| 201 | -CH₂-C(CH₃)=CH₂ | -H | 2,4-Difluorphenyl | -CH₃ | Cl | | 2,72 | |
| 202 | 1,3-Dioxolan-2-ylmethyl | -CH₃ | 2,4-Difluorphenyl | -CH₃ | Cl | | 2,56 | |
| 203 | Allyl | -CH₃ | 2,4-Difluorphenyl | -CH₃ | Cl | | 3,06 | |
| 204 | (2-Furyl)methyl | -CH₃ | 2,4-Difluorphenyl | -CH₃ | Cl | | 3,1 | |
| 205 | i-Butyl | -CH₃ | 2,4-Difluorphenyl | -CH₃ | Cl | | 3,49 | |
| 206 | -CH₂-C(CH₃)=CH₂ | -CH₃ | 2,4-Difluorphenyl | -CH₃ | Cl | | 3,41 | |
| 207 | Allyl | -C₂H₅ | 2,4- Difluorphenyl | -CH₃ | Cl | | 3,35 | |
| 208 | (2-Tetrahydrofuryl)methyl | -C₂H₅ | 2,4-Difluorphenyl | -CH₃ | Cl | | 3,18 | |
| 209 | 2-Methoxyethyl | n-Propyl | 2,4-Difluorphenyl | -CH₃ | Cl | | 3,26 | |
| 210 | i-Butyl | -H | 2-Chlorphenyl | -CH₃ | Cl | | 3,1 | |
| 211 | -CH₂-C(CH₃)₃ | -H | 2-Chlorphenyl | -CH₃ | Cl | | 3,57 | |
| 212 | 2-Butyl | -H | 2-Chlorphenyl | -CH₃ | Cl | | 3,09 | |
| 213 | Cyclopentyl | -H | 2-Chlorphenyl | -CH₃ | Cl | | 3,28 | |
| 214 | -i-Propyl | -H | 2-Chlorphenyl | -CH₃ | Cl | | 2,75 | |
| 215 | Cyclopropyl | -H | 2-Chlorphenyl | -CH₃ | Cl | | 2,54 | |
| 216 | Cyclopropylmethyl | -H | 2-Chlorphenyl | -CH₃ | Cl | | 2,85 | |
| 217 | -CH₂-C(CH₃)=CH₂ | -H | 2-Chlorphenyl | -CH₃ | Cl | | 2,83 | |
| 218 | -CH(CH₃)-CH₂-CH(CH₃)₂ | -H | 2-Chlorphenyl | -CH₃ | Cl | | 3,84 | |
| 219 | 1,3-Dioxolan-2-ylmethyl | -CH₃ | 2-Chlorphenyl | -CH₃ | Cl | | 2,62 | |
| 220 | Allyl | -CH₃ | 2-Chlorphenyl | -CH₃ | Cl | | 3,19 | |
| 221 | i-Butyl | -CH₃ | 2-Chlorphenyl | -CH₃ | Cl | | 3,65 | |
| 222 | 2-Methoxyethyl | -CH₃ | 2-Chlorphenyl | -CH₃ | Cl | | 2,64 | |
| 223 | -CH₂-C(CH₃)=CH₂ | -CH₃ | 2-Chlorphenyl | -CH₃ | Cl | | 3,57 | |
| 224 | Allyl | -C₂H₅ | 2-Chlorphenyl | -CH₃ | Cl | | 3,5 | |
| 225 | 2-Methoxyethyl | -C₂H₅ | 2-Chlorphenyl | -CH₃ | Cl | | 2,94 | |
| 226 | -CH₂-CH₂-CH₂-CH(CH₃)- | | 2-Chlorphenyl | -CH₃ | Cl | | 3,27 | |
| 227 | -CH₂-CH₂-CH₂-CH₂- | | 2-Chlorphenyl | -CH₃ | Cl | | 0,75 | |
| 228 | -CH₂-CH₂-CH=CH-CH₂- | | 2-Chlorphenyl | -CH₃ | Cl | | 0,75 | |
| 229 | -CH₂-CH₂-CH₂-CH(CH₃)-CH₂- | | 2-Chlorphenyl | -CH₃ | Cl | | 3,92 | |
| 230 | -CH₂-CH₂-CH(CH₃)-CH₂-CH₂- | | 2-Chlorphenyl | -CH₃ | Cl | | 3,94 | |
| 231 | -CH₂-CH₂-CH=C(CH₃)-CH₂- | | 2-Chlorphenyl | -CH₃ | Cl | | 3,7 | |
| 232 | -CH₂-CH₂-CH(CF₃)-CH₂-CH₂- | | 2-Chlorphenyl | -CH₃ | Cl | | 3,63 | |
| 233 | -CH₂-CH₂-CH₂-CH₂-CH₂- | | 2-Chlorphenyl | -CH₃ | Cl | | 3,48 | |
| 234 | -CH₂-CH₂-O-CH₂-CH₂- | | 2-Chlorphenyl | -CH₃ | Cl | | 2,38 | |
| 235 | -CH₂-CH₂-S-CH₂-CH₂- | | 2-Chlorphenyl | -CH₃ | Cl | | 3,09 | |
| 236 | 2-Methoxyethyl | -H | 2-Chlorphenyl | -CH₃ | Cl | | 2,3 | |
| 237 | Propargyl | -CH₃ | 2-Chlorphenyl | -CH₃ | Cl | | 2,75 | |
| 238 | (2-Furyl)methyl | -CH₃ | 2-Chlorphenyl | -CH₃ | Cl | | 3,2 | |
| 239 | (2-Tetrahydrofuryl)methyl | -C₂H₅ | 2-Chlorphenyl | -CH₃ | Cl | | 3,23 | |
| 240 | -CH₂-COOC₂H₅ | -C₂H₅ | 2-Chlorphenyl | -CH₃ | Cl | | 3,11 | |
| 241 | n-Butyl | -C₂H₅ | 2-Chlorphenyl | -CH₃ | Cl | | 4,1 | |
| 242 | -C₂H₅ | -C₂H₅ | 2-Chlorphenyl | -CH₃ | Cl | | 3,24 | |
| 243 | Cyclopropylmethyl | n-Propyl | 2-Chlorphenyl | -CH₃ | Cl | | 4,07 | |
| 244 | (2-Tetrahydrofuryl)methyl | n-Propyl | 2-Chlorphenyl | -CH₃ | Cl | | 3,7 | |
| 245 | -CH₂-CH(OH)-CH₂-CH₂- | | 2-Chlorphenyl | -CH₃ | Cl | | 0,75 | |
| 246 | -CH₂-CH₂-O-CH₂-CH₂- | | 2-Chlor-6-Fluorphenyl | -CH₃ | Cl | | 2,48 | |
| 247 | n-Butyl | -C₂H₅ | 2-Chlor-6-Fluorphenyl | -CH₃ | Cl | | 4,18 | |
| 248 | i-Butyl | -H | 2-Chlor-6-Fluorphenyl | -CH₃ | Cl | | 3,14 | |
| 249 | -CH₂-C(CH₃)₃ | -H | 2-Chlor-6- Fluorphenyl | -CH₃ | Cl | | 3,59 | |
| 250 | -CH₂-C(CH₃)=CH₂ | -H | 2-Chlor-6-Fluorphenyl | -CH₃ | Cl | | 2,8 | |
| 251 | -CH₂-CH₂-CH₂-CH₂- | | 2-Chlor-6-Fluorphenyl | -CH₃ | Cl | | 2,95 | |
| 252 | -C₂H₅ | -C₂H₅ | 2-Chlor-6-Fluorphenyl | -CH₃ | Cl | | 3,33 | |
| 253 | -CH₂-CN | -H | 2-Chlor-6-Fluorphenyl | -CH₃ | Cl | | 2,11 | |
| 254 | -CH₂-CH₂-CH₂-CH₂-CH₂- | | 2-Chlor-6-Fluorphenyl | -CH₃ | Cl | | 3,55 | |
| 255 | Cyclopentyl | -H | 2-Chlor-6-Fluorphenyl | -CH₃ | Cl | | 3,29 | |
| 256 | -i-Propyl | -H | 2-Chlor-6-Fluorphenyl | -CH₃ | Cl | | 2,77 | |
| 257 | 2-Methoxyethyl | -H | 2-Chlor-6-Fluorphenyl | -CH₃ | Cl | | 2,42 | |
| 258 | Cyclopropyl | -H | 2-Chlor-6-Fluorphenyl | -CH₃ | Cl | | 2,77 | |
| 259 | -CH₂-CH₂-S-CH₂-CH₂- | | 2-Chlor-6-Fluorphenyl | -CH₃ | Cl | | 3,18 | |
| 260 | -CH₂-CF₃ | -H | 2-Chlor-6-Fluorphenyl | -CH₃ | Cl | | 2,77 | |
| 261 | -CH₂-CH₂-CH(CF₃)-CH₂-CH₂- | | 2-Chlor-6-Fluorphenyl | -CH₃ | Cl | | 3,72 | |
| 262 | -CH₂-CH₂-CH(CH₃)-CH₂-CH₂- | | 2-Chlor-6-Fluorphenyl | -CH₃ | Cl | | 3,99 | |
| 263 | Cyclopropylmethyl | -H | 2-Chlor-6-Fluorphenyl | -CH₃ | Cl | | 2,95 | |
| 264 | 2-Butyl | -H | 2-Chlor-6-Fluorphenyl | -CH₃ | Cl | | 3,14 | |
| 265 | -CH₂-CH₂-CH=CH-CH₂- | | 2-Chlor-6-Fluorphenyl | -CH₃ | Cl | | 3,33 | |
| 266 | -CH₂-CH₂-CHF-CH₂-CH₂- | | 2-Chlor-6-Fluorphenyl | -CH₃ | Cl | | 3,07 | |
| 267 | Allyl | -C₂H₅ | 2-Chlor-6- Fluorphenyl | -CH₃ | Cl | | 3,59 | |
| 268 | (2-Tetrahydrofuryl)methyl | -C₂H₅ | 2-Chlor-6-Fluorphenyl | -CH₃ | Cl | | 3,29 | |
| 269 | 2-Methoxyethyl | -C₂H₅ | 2-Chlor-6-Fluorphenyl | -CH₃ | Cl | | 3,03 | |
| 270 | -CH₂-COOC₂H₅ | -C₂H₅ | 2-Chlor-6-Fluorphenyl | -CH₃ | Cl | | 3,14 | |
| 271 | Propargyl | -CH₃ | 2-Chlor-6- Fluorphenyl | -CH₃ | Cl | | 2,77 | |
| 272 | -CH₂-COOC₂H₅ | -CH₃ | 2-Chlor-6-Fluorphenyl | -CH₃ | Cl | | 2,48 | |
| 273 | Allyl | -CH₃ | 2-Chlor-6- Fluorphenyl | -CH₃ | Cl | | 3,26 | |
| 274 | (2-Furyl)methyl | -CH₃ | 2-Chlor-6-Fluorphenyl | -CH₃ | Cl | | 3,26 | |
| 275 | -CH₂-C(CH₃)=CH₂ | -CH₃ | 2-Chlor-6-Fluorphenyl | -CH₃ | Cl | | 3,63 | |
| 276 | i-Butyl | -CH₃ | 2-Chlor-6-Fluorphenyl | -CH₃ | Cl | | 3,68 | |
| 277 | (2-Tetrahydrofuryl)methyl | n-Propyl | 2-Chlor-6-Fluorphenyl | -CH₃ | Cl | | 3,72 | |
| 278 | -CH₂-CH(OH)-CH₂-CH₂- | | 2-Chlor-6-Fluorphenyl | -CH₃ | Cl | | 2,02 | |
| 279 | 1,3-Dioxolan-2-ylmethyl | -CH₃ | 2-Chlor-6-Fluorphenyl | -CH₃ | Cl | | | |
| 280 | -CH₂-CH₂-CH₂-CH(CH₃)- | | 2,4-Difluorphenyl | -CH₃ | Cl | | 3,14 | |
| 281 | -CH₂-CH₂-CH₂-CH₂- | | 2,4- Difluorphenyl | -CH₃ | Cl | | 2,74 | |
| 282 | -CH₂-CH₂-CH(OH)-CH₂-CH₂- | | 2,4-Difluorphenyl | -CH₃ | Cl | | 1,9 | |
| 283 | -CH₂-CH₂-CH=CH-CH₂- | | 2,4-Difluorphenyl | -CH₃ | Cl | | 3,11 | |
| 284 | AB10 | | 2,4-Difluorphenyl | -CH₃ | Cl | | 2,68 | |
| 285 | -CH₂-CH₂-CH₂-CH₂-CH(CH₃)- | | 2,4-Difluorphenyl | -CH₃ | Cl | | 3,7 | |
| 286 | -CH₂-CH₂-CH₂-CH(CH₃)-CH₂- | | 2,4-Difluorphenyl | -CH₃ | Cl | | 3,74 | |
| 287 | -CH₂-CH₂-CH(CH₃)-CH₂-CH₂- | | 2,4-Difluorphenyl | -CH₃ | Cl | | 3,75 | |
| 288 | -CH₂-CH₂-C(CH₃)₂-CH₂-CH₂- | | 2,4-Difluorphenyl | -CH₃ | Cl | | 4,01 | |
| 289 | -CH₂-CH₂-CH(COCH₃)-CH₂-CH₂- | | 2,4-Difluorphenyl | -CH₃ | Cl | | 2,5 | |
| 290 | -CH₂-CH=C(C₂H₅)-CH₂-CH₂- | | 2,4-Difluorphenyl | -CH₃ | Cl | | 3,94 | |
| 291 | -CH₂-CH₂-CH=C(CH₃)-CH₂- | | 2,4-Difluorphenyl | -CH₃ | Cl | | 3,49 | |
| 292 | -CH₂-CH₂-CH(COOCH₃)-CH₂-CH₂- | | 2,4-Difluorphenyl | -CH₃ | Cl | | 2,78 | |
| 293 | -CH₂-CH₂-CH(CF₃)-CH₂-CH₂- | | 2,4-Difluorphenyl | -CH₃ | Cl | | 3,49 | |
| 294 | -CH₂-CH₂-CH(NH-COCH₃)-CH₂-CH₂- | | 2,4-Difluorphenyl | -CH₃ | Cl | | 1,85 | |
| 295 | -CH₂-CH₂-CH₂-CH₂-CH₂- | | 2,4-Difluorphenyl | -CH₃ | Cl | | 3,32 | |
| 296 | -CH₂-CH₂-O-CH₂-CH₂- | | 2,4-Difluorphenyl | -CH₃ | Cl | | 2,3 | |
| 297 | -CH₂-CH₂-S-CH₂-CH₂- | | 2,4-Difluorphenyl | -CH₃ | Cl | | 2,97 | |
| 298 | 4-Tolyl | -H | 2,4-Difluorphenyl | -CH₃ | Cl | | 3,05 | |
| 299 | 4-Fluorphenyl | -H | 2,4-Difluorphenyl | -CH₃ | Cl | | 2,7 | |
| 300 | AB13 | | 2,4-Difluorphenyl | -CH₃ | Cl | | 3,28 | |
| 301 | -CH(CH₃)-CH₂-CH(CH₃)₂ | -H | 2,4-Difluorphenyl | -CH₃ | Cl | | 3,7 | |
| 302 | -CH₂-CH₂-CH₂-CH(CH₃)-CH₂- | | 2-Chlor-6-Fluorphenyl | -CH₃ | Cl | | 3,99 | |
| 303 | -CH₂-CH(OH)-CH₂-CH₂- | | 2,6-Difluorphenyl | -CH₃ | Cl | | 1,73 | |
| 304 | -CH₂-CH₂-CH₂-CH(CH₃)- | | 2,6-Difluorphenyl | -CH₃ | Cl | | 3,02 | |
| 305 | AB8 | | 2,6-Difluorphenyl | -CH₃ | Cl | | 1,64 | |
| 306 | AB9 | | 2,6-Difluorphenyl | -CH₃ | Cl | | 1,18 | |
| 307 | -CH₂-CH₂-CH(OH)-CH₂-CH₂- | | 2,6-Difluorphenyl | -CH₃ | Cl | | 1,83 | |
| 308 | -CH₂-CH₂-CH=CH-CH₂- | | 2,6-Difluorphenyl | -CH₃ | Cl | | 3,01 | |
| 309 | AB10 | | 2,6-Difluorphenyl | -CH₃ | Cl | | 2,6 | |
| 310 | -CH₂-CH(CH₃)-CH₂-CH(CH₃)-CH₂- | | 2,6-Difluorphenyl | -CH₃ | Cl | | 4,08 | |
| 311 | -CH₂-CH₂-CH₂-CH₂-CH(CH₃)- | | 2,6-Difluorphenyl | -CH₃ | Cl | | 3,57 | |
| 312 | -CH₂-CH₂-CH₂-CH(CH₃)-CH₂- | | 2,6-Difluorphenyl | -CH₃ | Cl | | 3,63 | |
| 313 | -CH₂-CH₂-CH(CH₃)-CH₂-CH₂- | | 2,6-Difluorphenyl | -CH₃ | Cl | | 3,64 | |
| 314 | -CH₂-CH(OH)-CH₂-CH₂-CH₂- | | 2,6-Difluorphenyl | -CH₃ | Cl | | 2,03 | |
| 315 | -CH₂-CH₂-C(CH₃)₂-CH₂-CH₂- | | 2,6-Difluorphenyl | -CH₃ | Cl | | 3,91 | |
| 316 | -CH₂-CH=C(C₂H₅)-CH₂-CH₂- | | 2,6-Difluorphenyl | -CH₃ | Cl | | 3,84 | |
| 317 | -CH₂-CH₂-CH=C(CH₃)-CH₂- | | 2,6- Difluorphenyl | -CH₃ | Cl | | 3,39 | |
| 318 | -CH₂-CH₂-CH(COOCH₃)-CH₂-CH₂- | | 2,6-Difluorphenyl | -CH₃ | Cl | | 2,69 | |
| 319 | -CH₂-CH₂-CHBr-CH₂-CH₂- | | 2,6-Difluorphenyl | -CH₃ | Cl | | 3,33 | |
| 320 | -CH(COOCH₃)-CH₂-CH₂-CH₂-CH₂- | | 2,6-Difluorphenyl | -CH₃ | Cl | | 3,04 | |
| 321 | AB12 | | 2,6-Difluorphenyl | -CH₃ | Cl | | 3,15 | |
| 322 | AB11 | | 2-Fluorphenyl | -CH₃ | Cl | | 1,26 | |
| 323 | -CH₂-CH₂-CH(NH-COCH₃)-CH₂-CH₂- | | 2,6-Difluorphenyl | -CH₃ | Cl | | 1,78 | |
| 324 | -CH₂-CH₂-N(CH₃)-CH₂-CH₂- | | 2,6-Difluorphenyl | -CH₃ | Cl | | 1,12 | |
| 325 | AB14 | | 2,6-Difluorphenyl | -CH₃ | Cl | | 3,56 | |
| 326 | 3-Tolyl | -H | 2,6-Difluorphenyl | -CH₃ | Cl | | 2,89 | |
| 327 | AB13 | | 2,6-Difluorphenyl | -CH₃ | Cl | | 3,22 | |
| 328 | i-Butyl | -H | 2,6-Difluorphenyl | -CH₃ | Cl | | 2,85 | |
| 329 | -CH₂-C(CH₃)₃ | -H | 2,6-Difluorphenyl | -CH₃ | Cl | | 3,27 | |
| 330 | -CH(CH₃)-CH₂-CH(CH₃)₂ | -H | 2,6- Difluorphenyl | -CH₃ | Cl | | 3,55 | |
| 331 | Propargyl | -CH₃ | 2,6-Difluorphenyl | -CH₃ | Cl | | 2,54 | |
| 332 | 1,3-Dioxolan-2-ylmethyl | -CH₃ | 2,6-Difluorphenyl | -CH₃ | Cl | | 2,43 | |
| 333 | Allyl | -CH₃ | 2,6- Difluorphenyl | -CH₃ | Cl | | 2,95 | |
| 334 | -CH₂-CH(OCH₃)₂ | -CH₃ | 2,6- Difluorphenyl | -CH₃ | Cl | | 2,55 | |
| 335 | i-Butyl | -CH₃ | 2,6-Difluorphenyl | -CH₃ | Cl | | 3,37 | |
| 336 | 2-Methoxyethyl | -CH₃ | 2,6-Difluorphenyl | -CH₃ | Cl | | 2,45 | |
| 337 | -CH₂-C(CH₃)=CH₂ | -CH₃ | 2,6-Difluorphenyl | -CH₃ | Cl | | 3,3 | |
| 338 | n-Butyl | -CH₃ | 2,6-Difluorphenyl | -CH₃ | Cl | | 3,46 | |
| 339 | -CH₂-C(CH₃)=CH₂ | -C₂H₅ | 2,6-Difluorphenyl | -CH₃ | Cl | | 3,65 | |
| 340 | (2-Furyl)methyl | -C₂H₅ | 2,6-Difluorphenyl | -CH₃ | Cl | | 3,28 | |
| 341 | (2-Tetrahydrofuryl)methyl | -C₂H₅ | 2,6-Difluorphenyl | -CH₃ | Cl | | 3 | |
| 342 | 2-Methoxyethyl | -C₂H₅ | 2,6-Difluorphenyl | -CH₃ | Cl | | 2,74 | |
| 343 | -CH₂-COOC₂H₅ | -C₂H₅ | 2,6-Difluorphenyl | -CH₃ | Cl | | 2,85 | |
| 344 | n-Butyl | -C₂H₅ | 2,6-Difluorphenyl | -CH₃ | Cl | | 3,82 | |
| 345 | Cyclopropylmethyl | n-Propyl | 2,6-Difluorphenyl | -CH₃ | Cl | | 3,83 | |
| 346 | (2-Tetrahydropyranyl)methyl | n-Propyl | 2,6-Difluorphenyl | -CH₃ | Cl | | 3,95 | |
| 347 | (2-Tetrahydrofuryl)methyl | n-Propyl | 2,6- Difluorphenyl | -CH₃ | Cl | | 3,39 | |
| 348 | 2-Methoxyethyl | n-Propyl | 2,6-Difluorphenyl | -CH₃ | Cl | | 3,12 | |
| 349 | 3-Trifluormethylcyclohexyl | -H | 2,4-Difluorphenyl | -CH₃ | Cl | | 3,52 | |
| 350 | -CH₂-CH₂-N(CH₃)₂ | -CH₃ | 2,4-Difluorphenyl | -CH₃ | Cl | | 1,23 | |
| 351 | Propargyl | -CH₃ | 2,4- Difluorphenyl | -CH₃ | Cl | | 2,67 | |
| 352 | 2-Methoxyethyl | -CH₃ | 2,4-Difluorphenyl | -CH₃ | Cl | | 2,58 | |
| 353 | -CH₂-CH₂-N(CH₃)₂ | -C₂H₅ | 2,4-Difluorphenyl | -CH₃ | Cl | | 1,37 | |
| 354 | (2-Furyl)methyl | -C₂H₅ | 2,4-Difluorphenyl | -CH₃ | Cl | | 3,39 | |
| 355 | -CH₂-COOC₂H₅ | -C₂H₅ | 2,4-Difluorphenyl | -CH₃ | Cl | | 3,04 | |
| 356 | Cyclopropylmethyl | n-Propyl | 2,4-Difluorphenyl | -CH₃ | Cl | | 3,91 | |
| 357 | (2-Tetrahydrofuryl)methyl | n-Propyl | 2,4- Difluorphenyl | -CH₃ | Cl | | 3,6 | |
| 358 | 2,2,2-Trifluor-1-methylethyl | -H | 2,4-Difluorphenyl | -CF₃ | Cl | | 3,89 | |
| 359 | 2,2,2-Trifluor-1-methylethyl | -H | 2,4- Difluorphenyl | -CH₃ | Cl | AS | 2,95 | |
| 360 | 2,2,2-Trifluor-1-methytethyl | -H | 2,4-Difluorphenyl | -CH₃ | Cl | BS | 2,96 | |
| 361 | i-Butoxy | -H | 2-Fluorphenyl | -CH₃ | Cl | | 2,98 | |
| 362 | -O-C₂H₅ | -H | 2-Fluorphenyl | -CH₃ | Cl | | 2,28 | |
| 363 | Benzyloxy | -H | 2-Fluorphenyl | -CH₃ | Cl | | 2,93 | |
| 364 | 3,5-Dichlorbenzyloxy | -H | 2-Fluorphenyl | -CH₃ | Cl | | 3,76 | |
| 365 | 4-Trifluormethylbenzyloxy | -H | 2-Fluorphenyl | -CH₃ | Cl | | 3,43 | |
| 366 | 2-Chlorbenzyloxy | -H | 2-Fluorphenyl | -CH₃ | Cl | | 3,21 | |
| 367 | 3-Chlorbenzyloxy | -H | 2-Fluorphenyl | -CH₃ | Cl | | 3,28 | |
| 368 | 4-Chlorbenzyloxy | -H | 2-Fluorphenyl | -CH₃ | Cl | | 3,3 | |
| 369 | 4-Fluorbenryloxy | -H | 2-Fluorphenyl | -CH₃ | Cl | | 2,98 | |
| 370 | -O-CH(CH₃)-CH₂-CH₃ | -H | 2-Fluorphenyl | -CH₃ | Cl | | 2,87 | |
| 371 | 3-Trifluormethylbenzyloxy | -H | 2-Fluorphenyl | -CH₃ | Cl | | 3,38 | |
| 372 | -n-Butoxy | -H | 2-Fluorphenyl | -CH₃ | Cl | | 2,98 | |
| 373 | Allyloxy | -H | 2-Fluorphenyl | -CH₃ | Cl | | 2,42 | |
| 374 | t-Butoxy | -H | 2-Fluorphenyl | -CH₃ | Cl | | 2,75 | |
| 375 | 2-Hexahydropyranyloxy | -H | 2-Fluorphenyl | -CH₃ | Cl | | 2,59 | |
| 376 | -O-CH₃ | -H | 2-Fluorphenyl | -CH₃ | Cl | | 2,03 | |
| 377 | -O-CH₃ | -CH₃ | 2-Fluorphenyl | -CH₃ | Cl | | 2,5 | |
| 378 | O-i-Propyl | -H | 2-Fluorphenyl | -CH₃ | Cl | | 2,54 | |
| 379 | 1-Cyclopropylethyl | -H | 2,4,6-Trifluorphenyl | -CH₃ | Cl | | 3,09 | |
| 380 | -CH₂-CF₃ | -C₂H₅ | 2,4,6-Trifluorphenyl | -CH₃ | Cl | | 3,42 | |
| 381 | -CH₃ | -CH₃ | 2-Fluorphenyl | -CH₃ | Cl | | 2,34 | |
| 382 | -CH₂-CH(CH₃)-O-CH(CH₃)-CH₂- | | 2-Fluorphenyl | -CH₃ | Cl | | 2,8 | |
| 383 | -C₂H₅ | -H | 2-Fluorphenyl | -CH₃ | Cl | | 2,3 | |
| 384 | -CH₂-CH₂-CH₂-CH₂-CH₂- | | 2-Fluorphenyl | -CH₃ | Cl | | 3,19 | |
| 385 | Cyclopentyl | -H | 2-Fluorphenyl | -CH₃ | Cl | | 3,11 | |
| 386 | -i-Propyl | -H | 2-Fluorphenyl | -CH₃ | Cl | | 2,63 | |
| 387 | -C₂H₅ | -C₂H₅ | 2-Fluorphenyl | -CH₃ | Cl | | 2,95 | |
| 388 | -CH₂-CH₂-O-CH₂-CH₂- | | 2-Fluorphenyl | -CH₃ | Cl | | 2,16 | |
| 389 | -CH₃ | -H | 2-Fluorphenyl | -CH₃ | Cl | | 2,02 | |
| 390 | Cyclopropyl | -H | 2-Fluorphenyl | -CH₃ | Cl | | 2,41 | |
| 391 | -CH₂-CF₃ | -H | 2-Fluorphenyl | -CH₃ | Cl | | 2,53 | |
| 392 | -CH₂-CH₂-CH(CF₃)-CH₂-CH₂- | | 2-Fluorphenyl | -CH₃ | Cl | | 3,37 | |
| 393 | Cyclopropylmethyl | -H | 2-Fluorphenyl | -CH₃ | Cl | | 2,72 | |
| 394 | -CH₂-C(CH₃)=CH₂ | -H | 2-Fluorphenyl | -CH₃ | Cl | | 2,68 | |
| 395 | -CH₂-CH₂-CF₃ | -H | 2-Fluorphenyl | -CH₃ | Cl | | 2,7 | |
| 396 | 1-Cyclohexylethyl | -H | 2-Fluorphenyl | -CH₃ | Cl | | 4,16 | |
| 397 | Cyclohexyl | -H | 2-Fluorphenyl | -CH₃ | Cl | | 3,43 | |
| 398 | 2-Trifluormethylcyclohexyl | -H | 2-Fluorphenyl | -CH₃ | Cl | | 3,49 | |
| 399 | 3,5-bis-Trifluormethylcyclohexyl | -H | 2-Fluorphenyl | -CH₃ | Cl | | 3,73 | |
| 400 | 4-Trifluormethylcyclohexyl | -H | 2-Fluorphenyl | -CH₃ | Cl | | 3,48 | |
| 401 | -CH₂-CH₂-CH₂-CH₂-CH(CH₃)- | | 2-Chlorphenyl | -CH₃ | Cl | | 3,91 | |
| 402 | -CH₂-CH₂-CHF-CH₂-CH₂- | | 2-Chlorphenyl | -CH₃ | Cl | | 3,05 | |
| 403 | -CH₂-CN | -H | 2-Chlorphenyl | -CH₃ | Cl | | 2,03 | |
| 404 | (2-Furyl)methyl | -C₂H₅ | 2-Chlorphenyl | -CH₃ | Cl | | 3,56 | |
| 405 | Cyclopentyl | -H | 2,4-Difluorphenyl | -CH₃ | Cl | | 3,24 | |
| 406 | -CH₂-CF₃ | -H | 2,4-Difluorphenyl | -CH₃ | Cl | | 2,68 | |
| 407 | -C(CH₃)₂-CH₂-COCH₃ | -H | 2,4-Difluorphenyl | -CH₃ | Cl | | 2,53 | |
| 408 | 2-Trifluormethylcyclohexyl | -H | 2,4- Difluorphenyl | -CH₃ | Cl | | 3,56 | |
| 409 | 4-Trifluormethylcyclohexyl | -H | 2,4-Difluorphenyl | -CH₃ | Cl | | 3,56 | |
| 410 | -CH₂-COOC₂H₅ | -CH₃ | 2,4-Difluorphenyl | -CH₃ | Cl | | 2,8 | |
| 411 | -CH₂-CH₂-CN | -CH₃ | 2,4-Difluorphenyl | -CH₃ | Cl | | 2,34 | |
| 412 | -CH₂-CN | -CH₃ | 2,4-Difluorphenyl | -CH₃ | Cl | | 2,34 | |
| 413 | -CH₂-CH₂-CN | -C₂H₅ | 2,4-Difluorphenyl | -CH₃ | Cl | | 2,58 | |
| 414 | -CH₂-COOC₂H₅ | Cycloprop yl | 2,4-Difluorphenyl | -CH₃ | Cl | | 3,29 | |
| 415 | -CH₂-CH(OH)-CH₂-CH₂- | | 2,4-Difluorphenyl | -CH₃ | Cl | | 1,95 | |
| 416 | 2-Methoxyethyl | n-Propyl | 2-Chlorphenyl | -CH₃ | Cl | | 3,36 | |
| 417 | -CH(CH₃)-CH=CH₂ | -H | 2,4,6-Trifluorphenyl | -CH₃ | Cl | | 2,85 | |
| 418 | -CH₃ | -CH₃ | 2,6-Difluorphenyl | -CH₃ | Cl | | 2,37 | |
| 419 | -C₂H₅ | -H | 2,6- Difluorphenyl | -CH₃ | Cl | | 2,31 | |
| 420 | -CH₂-CN | -H | 2,6- Difluorphenyl | -CH₃ | Cl | | 1,91 | |
| 421 | -C(CH₃)₂-CF₃ | -H | 2,6-Difluorphenyl | -CH₃ | Cl | | 3,34 | |
| 422 | -CH₃ | -H | 2,6-Difluorphenyl | -CH₃ | Cl | | 2,03 | |
| 423 | -CH(CF₃)-CH₂-CH₂-CH₂-CH₂- | | 2,6-Difluorphenyl | -CH₃ | Cl | | 3,33 | |
| 424 | -CH₂-CF₃ | -H | 2,6-Difluorphenyl | -CH₃ | Cl | | 2,56 | |
| 425 | 2-Trifluormethylcyclohexyl | -H | 2,6-Difluorphenyl | -CH₃ | Cl | | 3,52 | |
| 426 | -OH | -i-Propyl | 2,6-Difluorphenyl | -CH₃ | Cl | | 2,52 | |
| 427 | Benzyloxy | -H | 2,6-Difluorphenyl | -CH₃ | Cl | | 2,93 | |
| 428 | 3,5-Dichlorbenzyloxy | -H | 2,6-Difluorphenyl | -CH₃ | Cl | | 3,75 | |
| 429 | 2,4-Dichlorbenzyloxy | -H | 2,6-Difluorphenyl | -CH₃ | Cl | | 3,69 | |
| 430 | 4-Trifluormethylbenzyloxy | -H | 2,6-Difluorphenyl | -CH₃ | Cl | | 3,44 | |
| 431 | 2-Chlorbenzyloxy | -H | 2,6-Difluorphenyl | -CH₃ | Cl | | 3,13 | |
| 432 | 4-Fluorbenzyloxy | -H | 2,6-Difluorphenyl | -CH₃ | Cl | | 2,9 | |
| 433 | 3-Trifluormethylbenzyloxy | -H | 2,6- Difluorphenyl | -CH₃ | Cl | | 3,41 | |
| 434 | -n-Butoxy | -H | 2,6-Difluorphenyl | -CH₃ | Cl | | 2,96 | |
| 435 | 2,6-Dichlorbenzyloxy | -H | 2,6-Difluorphenyl | -CH₃ | Cl | | 3,31 | |
| 436 | -O-CH₃ | -H | 2,6- Difluorphenyl | -CH₃ | Cl | | 2,07 | |
| 437 | 2,2,2-Trifluor-1-methylethyl | -H | 2-Chlorphenyl | -CH₃ | Cl | | 3,1 | |
| 438 | 2,2,2-Trifluor-1-methylethyl | -H | 2-Chlorphenyl | -CH₃ | F | | 2,97 | |
| 439 | 2-Fluorcyclopropyl | -H | 2,4,6-Trifluorphenyl | -CH₃ | Cl | | 2,3 | |
| 440 | -CH₂-COOC₂H₅ | Cyclopropyl | 2,6-Difluorphenyl | -CH₃ | Cl | | 3 | |
| 441 | -CH₂-CH(NH₂)-CH₂-CH₂- | | 2,6-Difluorphenyl | -CH₃ | Cl | | 1,19 | |
| 442 | -CH₂-CH₂-CH(COCH₃)-CH₂-CH₂- | | 2,6-Difluorphenyl | -CH₃ | Cl | | 2,49 | |
| 443 | -CH₂-CH₂-CHF-CH₂-CH₂- | | 2,6-Difluorphenyl | -CH₃ | Cl | | 2,85 | |
| 444 | 4-Tolyl | -H | 2,6- Difluorphenyl | -CH₃ | Cl | | 3,01 | |
| 445 | 4-Fluorphenyl | -H | 2,6-Difluorphenyl | -CH₃ | Cl | | 2,71 | |
| 446 | AB28 | | 2,6- Difluorphenyl | -CH₃ | Cl | | 3,34 | |
| 447 | -CH₂-C(CH₃)=CH₂ | -C₂H₅ | 2-Chlorphenyl | Cyclopropyl | Cl | | 4,59 | |
| 448 | -CH₂-C(CH₃)=CH₂ | -C₂H₅ | 2,4,6-Trifluorphenyl | Cyclopropyl | Cl | | 4,49 | |
| 449 | -CH₂-C(CH₃)=CH₂ | -C₂H₅ | 2-Chlor-6-Fluorphenyl | Cyclopropyl | Cl | | 4,61 | |
| 450 | AB8 | | 2,4- Difluorphenyl | -CH₃ | Cl | | 1,73 | |
| 451 | -CH₂-CH(CH₃)-CH₂-CH(CH₃)-CH₂- | | 2,4-Difluorphenyl | -CH₃ | Cl | | 4,16 | |
| 452 | -CH₂-CH(OH)-CH₂-CH₂-CH₂- | | 2,4-Difluorphenyl | -CH₃ | Cl | | 2,2 | |
| 453 | -CH(COOCH₃)-CH₂-CH₂-CH₂-CH₂- | | 2,4-Difluorphenyl | -CH₃ | Cl | | 3,36 | |
| 454 | -CH₂-CH₂-CHF-CH₂-CH₂- | | 2,4-Difluorphenyl | -CH₃ | Cl | | 2,96 | |
| 455 | -CH₂-CH(CH₃)-O-CH(CH₃)-CH₂- | | 2,4-Difluorphenyl | -CH₃ | Cl | | 2,94 | |
| 456 | 3-Tolyl | -H | 2,4-Difluorphenyl | -CH₃ | Cl | | 3 | |
| 457 | 2,2,2-Trifluor-1-methylethyl | -H | 2-Chlor-6-Fluorphenyl | Cyclopropyl | Cl | | 3,59 | |
| 458 | 2,2,2-Trifluor-1-methylethyl | -H | 2,4,6-Trifluorphenyl | Cyclopropyl | Cl | | 3,58 | |
| 459 | -NH-CH₂-CH₂-CH₂-CH₂- | | 2-Chlor-6-Fluorphenyl | Cyclopropyl | Cl | | 3,46 | |
| 460 | i-Butyl | -H | 2,4,6-Trifluorphenyl | Cyclopropyl | Cl | | 3,69 | |
| 461 | -CH₂-C(CH₃)₃ | -H | 2,4,6-Trifluorphenyl | Cyclopropyl | Cl | | 4,19 | |
| 462 | 2-Butyl | -H | 2,4,6-Trifluorphenyl | Cyclopropyl | Cl | | 3,69 | |
| 463 | Cyclopentyl | -H | 2,4,6-Trifluorphenyl | Cyclopropyl | Cl | | 3,9 | |
| 464 | -i-Propyl | -H | 2,4,6-Trifluorphenyl | Cyclopropyl | Cl | | 3,35 | |
| 465 | Cyclopropylmethyl | -H | 2,4,6-Trifluorphenyl | Cyclopropyl | Cl | | 3,43 | |
| 466 | -CH₂-C(CH₃)=CH₂ | -H | 2,4,6-Trifluorphenyl | Cyclopropyl | Cl | | 3,34 | |
| 467 | i-Butyl | -CH₃ | 2,4,6-Trifluorphenyl | Cyclopropyl | Cl | | 4,36 | |
| 468 | 2-Methoxyethyl | -CH₃ | 2,4,6-Trifluorphenyl | Cyclopropyl | Cl | | 3,29 | |
| 469 | -CH₂-C(CH₃)=CH₂ | -CH₃ | 2,4,6-Trifluorphenyl | Cyclopropyl | Cl | | 4,27 | |
| 470 | 2-Methoxyethyl | -C₂H₅ | 2,4,6-Trifluorphenyl | Cyclopropyl | Cl | | 3,64 | |
| 471 | -CH₂-CH₂-CH₂-CH(CH₃)- | | 2,4,6-Trifluorphenyl | Cyclopropyl | Cl | | 3,9 | |
| 472 | -CH₂-CH₂-CH₂-CH₂- | | 2,4,6-Trifluorphenyl | Cyclopropyl | Cl | | 3,52 | |
| 473 | -CH₂-CH₂-CH(CH₃)-CH₂-CH₂- | | 2,4,6-Trifluorphenyl | Cyclopropyl | Cl | | 4,66 | |
| 474 | -CH₂-CH₂-CH₂-CH₂-CH₂- | | 2,4,6-Trifluorphenyl | Cyclopropyl | Cl | | 4,22 | |
| 475 | -CH₂-CH₂-O-CH₂-CH₂- | | 2,4,6- Trifluorphenyl | Cyclopropyl | Cl | | 3,02 | |
| 476 | i-Butyl | -H | 2-Chlor-6-Fluorphenyl | Cyclopropyl | Cl | | 3,64 | |
| 477 | -CH₂-C(CH₃)₃ | -H | 2-Chlor-6-Fluorphenyl | Cyclopropyl | Cl | | 4,08 | |
| 478 | 2-Butyl | -H | 2-Chlor-6-Fluorphenyl | Cyclopropyl | Cl | | 3,66 | |
| 479 | Cyclopentyl | -H | 2-Chlor-6-Fluorphenyl | Cyclopropyl | Cl | | 3,83 | |
| 480 | -i-Propyl | -H | 2-Chlor-6-Fluorphenyl | Cyclopropyl | Cl | | 3,33 | |
| 481 | -CH₂-CF₃ | -H | 2-Chlor-6-Fluorphenyl | Cyclopropyl | Cl | | 3,19 | |
| 482 | Cyclopropylmethyl | -H | 2-Chlor-6-Fluorphenyl | Cyclopropyl | Cl | | 3,4 | |
| 483 | -CH₂-C(CH₃)=CH₂ | -H | 2-Chlor-6-Fluorphenyl | Cyclopropyl | Cl | | 3,31 | |
| 484 | -O-CH₂-CH₂-CH₂-CH₂- | | 2-Chlorphenyl | Cyclopropyl | Cl | | 3,71 | |
| 485 | -O-CH₂-CH₂-CH₂-CH₂- | | 2,4,6-Trifluorphenyl | Cyclopropyl | Cl | | 3,74 | |
| 486 | -O-CH₂-CH₂-CH₂-CH₂- | | 2-Chlor-6-Fluorphenyl | Cyclopropyl | Cl | | 3,74 | |
| 487 | -NH-CH₂-CH₂-CH₂-CH₂- | | 2,4,6-Trifluorphenyl | Cyclopropyl | Cl | | 3,42 | |
| 488 | -NH-CH₂-CH₂-CH₂-CH₂- | | 2-Chlorphenyl | Cyclopropyl | Cl | | 3,39 | |
| 489 | 2,2,2-Trifluor-1-methylethyl | -H | 2,4-Difluorphenyl | Cyclopropyl | Cl | AS | 3,51 | |
| 490 | 2,2,2-Trifluor-1-methylethyl | -H | 2,4-Difluorphenyl | Cyclopropyl | Cl | BS | 3,52 | |
| 491 | -CH₂-C(CH₃)=CH₂ | -C₂H₅ | 2,4-Difluorphenyl | Cyclopropyl | Cl | | 4,37 | 96-98 |
| 492 | i-Butyl | -H | 2-Chlorphenyl | Cyclopropyl | Cl | | 3,76 | |
| 493 | -CH₂-CH₂-CH₂-CH₂-CH₂- | | 2-Chlorphenyl | Cyclopropyl | Cl | | 4,27 | |
| 494 | 2-Butyl | -H | 2-Chlorphenyl | Cyclopropyl | Cl | | 3,76 | |
| 495 | -CH₂-C(CH₃)=CH₂ | -H | 2-Chlorphenyl | Cyclopropyl | Cl | | 3,41 | |
| 496 | -CH₂-C(CH₃)=CH₂ | -CH₃ | 2-Chlorphenyl | Cyclopropyl | Cl | | 4,32 | |
| 497 | i-Butyl | -CH₃ | 2-Chlorphenyl | Cyclopropyl | Cl | | 4,41 | |
| 498 | -CH₂-CH₂-CH₂-CH(CH₃)- | | 2-Chlorphenyl | Cyclopropyl | Cl | | 3,99 | |
| 499 | -CH₂-CH₂-CH₂-CH₂-CH(CH₃)- | | 2-Chlorphenyl | Cyclopropyl | Cl | | 4,61 | |
| 500 | -CH₂-CF₃ | -H | 2-Chlorphenyl | Cyclopropyl | Cl | | 3,22 | |
| 501 | -CH(CF₃)-CH₂-CH₂-CH₂- | | 2-Chlorphenyl | Cyclopropyl | Cl | | 4,41 | |
| 502 | (2,2-Dichlorcyclopropyl)methyl | -CH₃ | 2,4,6-Trifluorphenyl | -CH₃ | Cl | | 3,68 | |
| 503 | -i-Propyl | -H | 2-Chlor-4-fluorphenyl | Cyclopropyl | Cl | | 3,5 | |
| 504 | -NH-CH₂-CH₂-CH₂-CH₂- | | 2-Chlor-4-fluorphenyl | Cyclopropyl | Cl | | 3,58 | |
| 505 | 2,2,2-Trifluor-1-methylethyl | -H | 2-Chlor-4-fluorphenyl | Cyclopropyl | Cl | AS+ BS | 3,75 | |
| 506 | 1,2-Dimethylpropyl | -H | 2,4,6- Trifluorphenyl | -CH₃ | Cl | | 3,35 | |
| 507 | (2,2-Dichlorcyclopropyl)methyl | -CH₃ | 2,4-Difluorphenyl | Cyclopropyl | Cl | | 4,18 | 133-36 |
| 508 | (2,2-Dichlorcyclopropyl)methyl | -CH₃ | 2-Chlorphenyl | Cyclopropyl | Cl | | 4,29 | |
| 509 | (2,2-Dichlorcyclopropyl)methyl | -CH₃ | 2,4,6-Trifluorphenyl | Cyclopropyl | Cl | | 4,26 | |
| 510 | (2,2-Dichlorcyclopropyl)methyl | -CH₃ | 2-Chlor-6-Fluorphenyl | Cyclopropyl | Cl | | 4,37 | |
| 511 | -CH₂-CH=C(C₂H₅)-CH₂-CH₂- | | 2,4-Difluorphenyl | Cyclopropyl | Cl | | 4,59 | |
| 512 | -CH₂-CH₂-CH=C(CH₃)-CH₂- | | 2,4-Difluorphenyl | Cyclopropyl | Cl | | 4,14 | |
| 513 | -CH₂-CH₂-CHF-CH₂-CH₂- | | 2,4-Difluorphenyl | Cyclopropyl | Cl | | 3,45 | |
| 514 | AB12 | | 2,4-Difluorphenyl | Cyclopropyl | Cl | | 3,78 | |
| 515 | -CH₂-CH₂-CH(CF₃)-CH₂-CH₂- | | 2,4-Difluorphenyl | Cyclopropyl | Cl | | 4,07 | |
| 516 | -CH₂-CH₂-CH(NH-COCH₃)-CH₂-CH₂- | | 2,4-Difluorphenyl | Cyclopropyl | Cl | | 2,2 | |
| 517 | -CH₂-CH₂-CH₂-CH₂-CH₂- | | 2,4-Difluorphenyl | Cyclopropyl | Cl | | 4,03 | |
| 518 | -CH₂-CH₂-O-CH₂-CH₂- | | 2,4-Difluorphenyl | Cyclopropyl | Cl | | 2,85 | |
| 519 | -CH₂-CH₂-S-CH₂-CH₂- | | 2,4-Difluorphenyl | Cyclopropyl | Cl | | 3,58 | |
| 520 | AB14 | | 2,4-Difluorphenyl | Cyclopropyl | Cl | | 4,25 | |
| 521 | 4-Tolyl | -H | 2,4-Difluorphenyl | Cyclopropyl | Cl | | 3,6 | |
| 522 | AB 13 | | 2,4- Difluorphenyl | Cyclopropyl | Cl | | 3,87 | |
| 523 | 1,2-Dimethylpropyl | -H | 2,4,6-Trifluorphenyl | -CH₃ | Cl | | 3,38 | |
| 524 | -O-CH₂-CH₂-CH₂-CH₂- | | 2,6-Difluorphenyl | -CH₃ | Cl | | 2,84 | |
| 525 | i-Butyl | -H | 2,4-Difluorphenyl | -CF₃ | Cl | | 4,03 | |
| 526 | n-Butyl | -H | 2,4-Difluorphenyl | -CF₃ | Cl | | 4,07 | |
| 527 | -CH₂-C(CH₃)₃ | -H | 2,4-Difluorphenyl | -CF₃ | Cl | | 4,4 | |
| 528 | 2-Butyl | -H | 2,4-Difluorphenyl | -CF₃ | Cl | | 4,03 | |
| 529 | -CH₂-CH₂-CF₃ | -H | 2,4- Difluorphenyl | -CF₃ | Cl | | 3,7 | |
| 530 | Cyclopentyl | -H | 2,4-Difluorphenyl | -CF₃ | Cl | | 4,21 | |
| 531 | Cyclopropyl -H | | 2,4-Difluorphenyl | -CF₃ | Cl | | 3,49 | |
| 532 | -CH₂-CF₃ | -H | 2,4-Difluorphenyl | -CF₃ | Cl | | 3,58 | |
| 533 | Cyclopropylmethyl | -H | 2,4-Difluorphenyl | -CF₃ | Cl | | 3,8 | |
| 534 | -CH₂-C(CH₃)=CH₂ | -H | 2,4-Difluorphenyl | -CF₃ | Cl | | 3,75 | |
| 535 | t-Butyl | -H | 2,4-Difluorphenyl | -CF₃ | Cl | | 4,19 | |
| 536 | 3-Trifluormethylcyclohexyl | -H | 2,4-Difluorphenyl | -CF₃ | Cl | | 4,44 | |
| 537 | 4-Trifluormethylcyclohexyl | -H | 2,4-Difluorphenyl | -CF₃ | Cl | | 4,43 | |
| 538 | -CH₂-CH₂-N(CH₃)₂ | -CH₃ | 2,4-Difluorphenyl | -CF₃ | Cl | | 1,77 | |
| 539 | Propargyl | -CH₃ | 2,4-Difluorphenyl | -CF₃ | Cl | | 3,65 | |
| 540 | 1,3-Dioxolan-2-ylmethyl | -CH₃ | 2,4-Difluorphenyl | -CF₃ | Cl | | 3,62 | |
| 541 | Allyl | -CH₃ | 2,4-Difluorphenyl | -CF₃ | Cl | | 4,09 | |
| 542 | -CH₂-CH₂-CN | -CH₃ | 2,4-Difluorphenyl | -CF₃ | Cl | | 3,19 | |
| 543 | (2-Furyl)methyl | -CH₃ | 2,4-Difluorphenyl | -CF₃ | Cl | | 4,11 | |
| 544 | i-Butyl | -CH₃ | 2,4-Difluorphenyl | -CF₃ | Cl | | 4,54 | |
| 545 | 2-Methoxyethyl | -CH₃ | 2,4-Difluorphenyl | -CF₃ | Cl | | 3,73 | |
| 546 | -CH₂-C(CH₃)=CH₂ | -CH₃ | 2,4-Difluorphenyl | -CF₃ | Cl | | 4,44 | |
| 547 | -CH₂-CH₂-N(CH₃)₂ | -C₂H₅ | 2,4- Difluorphenyl | -CF₃ | Cl | | 1,91 | |
| 548 | Allyl | -C₂H₅ | 2,4-Difluorphenyl | -CF₃ | Cl | | 4,4 | |
| 549 | (2-Furyl)methyl | -C₂H₅ | 2,4-Difluorphenyl | -CF₃ | Cl | | 4,42 | |
| 550 | -CH₂-CH₂-CN | -C₂H₅ | 2,4-Difluorphenyl | -CF₃ | Cl | | 3,46 | |
| 551 | -CH₂-COOC₂H₅ | -C₂H₅ | 2,4-Difluorphenyl | -CF₃ | Cl | | 4,05 | |
| 552 | (2-Tetrahydrofuryl)methyl | n-Propyl | 2,4-Difluorphenyl | -CF₃ | Cl | | 4,77 | |
| 553 | 2-Methoxyethyl | n-Propyl | 2,4-Difluorphenyl | -CF₃ | Cl | | 4,39 | |
| 554 | -CH₂-COOC₂H₅ | Cyclopropyl | 2,4-Difluorphenyl | -CF₃ | Cl | | 4,28 | |
| 555 | -CH₂-CH(OH)-CH₂-CH₂- | | 2,4-Difluorphenyl | -CF₃ | Cl | | 2,76 | |
| 556 | Cyclohexyl | -H | 2,4-Difluorphenyl | -CF₃ | Cl | | 4,47 | |
| 557 | 1-Cyclohexylethyl | -H | 2,4-Difluorphenyl | -CF₃ | Cl | | 5,12 | |
| 558 | 2-Methoxyethyl | -C₂H₅ | 2,4-Difluorphenyl | -CF₃ | Cl | | 4,03 | |
| 559 | AB30 | -H | 2,4,6-Trifluorphenyl | -CH₃ | Cl | | 3,71 | |
| 560 | n-Butyl | -H | 2,6-Difluorphenyl | -CH₃ | Cl | | 2,89 | |
| 561 | -CH₂-CH₂-N(CH₃)₂ | -CH₃ | 2,6-Difluorphenyl | -CH₃ | Cl | | 1,28 | |
| 562 | -CH₂-COOC₂H₅ | -CH₃ | 2,6-Difluorphenyl | -CH₃ | Cl | | 2,62 | |
| 563 | -CH₂-CH₂-CN | -CH₃ | 2,6-Difluorphenyl | -CH₃ | Cl | | 2,17 | |
| 564 | -CH₂-CN | -CH₃ | 2,6-Difluorphenyl | -CH₃ | Cl | | 2,18 | |
| 565 | -CH₂-COOCH₃ | -CH₃ | 2,6-Difluorphenyl | -CH₃ | Cl | | 2,32 | |
| 566 | (2-Furyl)methyl | -CH₃ | 2,6-Difluorphenyl | -CH₃ | Cl | | 2,97 | |
| 567 | -CH₂-CH₂-N(CH₃)₂ | -C₂H₅ | 2,6-Difluorphenyl | -CH₃ | Cl | | 1,46 | |
| 568 | Allyl | -C₂H₅ | 2,6-Difluorphenyl | -CH₃ | Cl | | 3,3 | |
| 569 | -CH₂-CH₂-CN | -C₂H₅ | 2,6-Difluorphenyl | -CH₃ | Cl | | 2,41 | |
| 570 | 2-Thienylmethyl | n-Propyl | 2,6-Difluorphenyl | -CH₃ | Cl | | 3,96 | |
| 571 | -CH₂-CH₂-NH₂ | -i-Propyl | 2,6-Difluorphenyl | -CH₃ | Cl | | 1,28 | |
| 572 | AB31 | | 2,6-Difluorphenyl | -CH₃ | Cl | | 2,66 | |
| 573 | AB31 | | 2,4,6- -Trifluorphenyl | CH₃ | Cl | | 2,9 | |
| 574 | -O-CH₂-CH₂-CH₂-CH₂- | | 2,4,6-Trifluorphenyl | -CH₃ | Cl | | 3,07 | |
| 575 | AB31 | | 2,4-Difluorphenyl | -CH₃ | Cl | | 2,74 | |
| 576 | -O-CH₂-CH₂-CH₂-CH₂- | | 2,4-Difluorphenyl | -CH₃ | Cl | | 2,95 | |
| 577 | 1,2-Dimethylpropyl | -H | 2,4-Difluorphenyl | -CH₃ | Cl | | | |
| 578 | -CH(CH₃)-CH₂-CH₂-O- | | 2,6-Difluorphenyl | -CH₃ | Cl | | 2,61 | |
| 579 | 1,2-Dimethylpropyl | -H | 2-Chlorphenyl | Cyclopropyl | Cl | | 4,14 | |
| 580 | -CH(CH₃)-CH=CH-CH(CH₃)- | | 2-Chlorphenyl | Cyclopropyl | Cl | | 4,19 | |
| 581 | 1,2-Dimethylpropyl | -H | 2,4,6-Trifluorphenyl | Cyclopropyl | Cl | | 4,03 | |
| 582 | -CH(CH₃)-CH=CH-CH(CH₃)- | | 2,4,6-Trifluorphenyl | Cyclopropyl | Cl | | 4,17 | |
| 583 | 1,2-Dimethylpropyl | -H | 2-Chlor-6-Fluorphenyl | Cyclopropyl | Cl | | 4,12 | |
| 584 | -CH(CH₃)-CH=CH-CH(CH₃)- | | 2-Chlor-6-Fluorphenyl | Cyclopropyl | Cl | | 4,15 | |
| 585 | -CH(CH₃)-CH₂-CH₂-O- | | 2,4,6-Trifluorphenyl | -CH₃ | Cl | | 2,93 | |
| 586 | 1,2-Dimethylpropyl | -H | 2-Chlor-6-Fluorphenyl | Cyclopropyl | Cl | AS + BR | 4,25 | |
| 587 | 1,2-Dimethylpropyl | -H | 2-Chlor-4-fluorphenyl | Cyclopropyl | Cl | AR + BS | 4,25 | |
| 588 | 1,2-Dimethylpropyl | -H | 2-Chlor-4-fluorphenyl | -CH₃ | Cl | | 3,6 | |
| 589 | -O-CH₂-CH₂-CH₂-CH₂- | | 2-Chlor-4-fluorphenyl | -CH₃ | Cl | | 3,23 | |
| 590 | AB31 | | 2-Chlor-4-fluorphenyll | -CH₃ | Cl | | 2,99 | |
| 591 | Allyl | -C₂H₅ | 2-Chlor-4-fluorphenyl | Cyclopropyl | Cl | | 3,96 | |
| 592 | (2-Furyl)methyl | -C₂H₅ | 2-Chlor-4-fluorphenyl | Cyclopropyl | Cl | | 3,97 | |
| 593 | (2-Tetrahydrofuryl)methyl | -C₂H₅ | 2-Chlor-4-fluorphenyl | Cyclopropyl | Cl | | 3,78 | |
| 594 | 2-Methoxyethyl | -C₂H₅ | 2-Chlor-4-fluorphenyll | Cyclopropyl | Cl | | 3,46 | |
| 595 | -CH₂-COOC₂H₅ | -C₂H₅ | 2-Chlor-4-fluorphenyl | Cyclopropyl | Cl | | 3,54 | |
| 596 | n-Butyl | -C₂H₅ | 2-Chlor-4-fluorphenyl | Cyclopropyl | Cl | | 4,52 | |
| 597 | -C₂H₅ | -C₂H₅ | 2-Chlor-4-fluorphenyl | Cyclopropyl | Cl | | 3,73 | |
| 598 | Cyclopropylmethyl | n-Propyl | 2-Chlor-4-fluorphenyl | Cyclopropyl | Cl | | 4,53 | |
| 599 | (2-Tetrahydropyranyl)methyl | n-Propyl | 2-Chlor-4-fluorphenyl | Cyclopropyl | Cl | | 4,71 | |
| 600 | (2-Tetrahydrofuryl)methyl | n-Propyl | 2,4-Difluorphenyl | Cyclopropyl | Cl | | 4,22 | |
| 601 | 2-Thienylmethyl | n-Propyl | 2,4-Difluorphenyl | Cyclopropyl | Cl | | 4,64 | |
| 602 | 2-Methoxyethyl | n-Propyl | 2,4-Difluorphenyl | Cyclopropyl | Cl | | 3,84 | |
| 603 | -CH₂-CH(OH)-CH₂-CH₂- | | 2,4-Difluorphenyl | Cyclopropyl | Cl | | 2,2 | |
| 604 | -CH₂-CH₂-CH₂-CH(CH₃)- | | 2,4-Difluorphenyl | Cyclopropyl | Cl | | 3,76 | |
| 605 | AB9 | | 2,4-Difluorphenyl | Cyclopropyl | Cl | | 1,46 | |
| 606 | -CH₂-CH₂-CH₂-CH₂- | | 2,4-Difluorphenyl | Cyclopropyl | Cl | | 3,36 | |
| 607 | -CH₂-CH₂-CH(OH)-CH₂-CH₂- | | 2,4-Difluorphenyl l | Cyclopropyl | Cl | | 2,29 | |
| 608 | -CH₂-CH₂-CH=CH-CH₂- | | 2,4-Difluorphenyl | Cyclopropyl | Cl | | 3,72 | |
| 609 | AB10 | | 2,4-Difluorphenyl | Cyclopropyl | Cl | | 3,22 | |
| 610 | -CH₂-CH(CH₃)-CH₂-CH(CH₃)-CH₂- | | 2,4-Difluorphenyl | Cyclopropyl | Cl | | 4,72 | |
| 611 | -CH₂-CH₂-CH₂-CH₂-CH(CH₃)- | | 2,4-Difluorphenyl | Cyclopropyl | Cl | | 4,3 | |
| 612 | -CH₂-CH₂-CH₂-CH(CH₃)-CH₂- | | 2,4-Difluorphenyl | Cyclopropyl | Cl | | 4,37 | |
| 613 | -CH₂-CH₂-CH(CH₃)-CH₂-CH₂- | | 2,4-Difluorphenyl | Cyclopropyl | Cl | | 4,41 | |
| 614 | -CH₂-CH(OH)-CH₂-CH₂-CH₂- | | 2,4-Difluorphenyl | Cyclopropyl | Cl | | 2,57 | |
| 615 | -CH₂-CH₂-C(CH₃)₂-CH₂-CH₂- | | 2,4-Difluorphenyl | Cyclopropyl | Cl | | 4,67 | |
| 616 | 2-Methoxyethyl | -H | 2-Chlor-4-fluorphenyl | Cyclopropyl | Cl | | 3,07 | |
| 617 | -CH₂-CH₂-CH₂-CH₂-CH(CH₃)- | | 2-Chlor-4-fluorphenyl | Cyclopropyl | Cl | | 4,72 | |
| 618 | -CH₂-CH₂-CH(CH₃)-CH₂-CH₂- | | 2-Chlor-4-fluorphenyl | Cyclopropyl | Cl | | 4,82 | |
| 619 | Dimethylamino | -H | 2-Chlor-4-fluorphenyl | Cyclopropyl | Cl | | 3,29 | |
| 620 | 1-Cyclopropylethylamino | -H | 2-Chlor-4-fluorphenyl | Cyclopropyl | Cl | | 3,81 | |
| 621 | i-Butyl | -H | 2-Chlor-4-fluorphenyl | Cyclopropyl | Cl | | 3,9 | |
| 622 | -CH₂-C(CH₃)₃ | -H | 2-Chlor-4-fuorphenyl | Cyclopropyl | Cl | | 4,37 | |
| 623 | 2-Butyl | -H | 2-Chlor-4-fuorphenyl | Cyclopropyl | Cl | | 3,9 | |
| 624 | Cyclopentyl | -H | 2-Chlor-4-fuorphenyl | Cyclopropyl | Cl | | 4,13 | |
| 625 | Cyclopropylmethyl | -H | 2-Chlor-4-fuorphenyl | Cyclopropyl | Cl | | 3,63 | |
| 626 | -CH₂-C(CH₃)=CH₂ | -H | 2-Chlor-4-fuorphenyl | Cyclopropyl | Cl | | 3,59 | |
| 627 | i-Butyl | -CH₃ | 2-Chlor-4-fuorphenyl | Cyclopropyl | Cl | | 4,51 | |
| 628 | 2-Methoxyethyl | -CH₃ | 2-Chlor-4-fluorphenyl | Cyclopropyl | Cl | | 3,46 | |
| 629 | -CH₂-C(CH₃)=CH₂ | -CH₃ | 2-Chlor-4-fluorphenyl | Cyclopropyl | Cl | | 4,41 | |
| 630 | -CH₂-C(CH₃)=CH₂ | -C₂H₅ | 2-Chlor-4-fluorphenyl | Cyclopropyl | Cl | | 4,77 | |
| 631 | 2-Methoxyethyl | -C₂H₅ | 2-Chlor-4-fluorphenyl | Cyclopropyl | Cl | | 3,81 | |
| 632 | -C₂H₅ | -C₂H₅ | 2-Chlor-4-fluorphenyl | Cyclopropyl | Cl | | 4,13 | |
| 633 | -CH₂-CH₂-CH₂-CH(CH₃)- | | 2-Chlor-4-fluorphenyl | Cyclopropyl | Cl | | 4,08 | |
| 634 | -CH₂-CH₂-CH₂-CH₂- | | 2-Chlor-4-fluorphenyl | Cyclopropyl | Cl | | 3,72 | |
| 635 | -CH₂-CH₂-CH₂-CH₂-CH₂- | | 2-Chlor-4-fluorphenyl | Cyclopropyl | Cl | | 4,37 | |
| 636 | -CH₂-CH₂-O-CH₂-CH₂- | | 2-Chlor-4-fluorphenyl | Cyclopropyl | Cl | | 3,18 | |
| 637 | -CH(CH₃)-CH₂-CH(CH₃)₂ | -H | 2-Chlor-4-fluorphenyl | Cyclopropyl | Cl | | 4,61 | |
| 638 | -CH₂-CF₃ | -H | 2-Chlor-4-fluorphenyl | Cyclopropyl | Cl | | 3,37 | |
| 639 | -CH(CF₃)-CH₂-CH₂-CH₂- | | 2-Chlor-4-fluorphenyl | Cyclopropyl | Cl | | 4,23 | |
| 640 | 2,2,2-Trifluor-1-methylethyl | -H | 2-Chlor-4-fluorphenyl | -CH₃ | Cl | AS + BS | 3,19 | |
| 641 | 2,2,2-Trifluor-1-methylethyl | -H | 2-Chlor-4-fluorphenyl | -CH₃ | Cl | AS | 3,2 | |
| 642 | 2,2,2-Trifluor-1-methylethyl | -H | 2-Chlor-4-fluorphenyl | -CH₃ | Cl | BS | 3,17 | |
| 643 | -CH₂-C(CH₃)=CH₂ | -C₂H₅ | 2-Chlor-4-fluorphenyl | -CH₃ | Cl | | 4,06 | |
| 644 | i-Butyl | -H | 2,4-Difluorphenyl | Cyclopropyl | Cl | | 3,38 | |
| 645 | n-Butyl | -H | 2,4-Difluorphenyl | Cyclopropyl | Cl | | 3,6 | |
| 646 | -CH₂-C(CH₃)₃ | -H | 2,4-Difluorphenyl | Cyclopropyl | Cl | | 4 | |
| 647 | 2-Butyl | -H | 2,4-Difluorphenyl | Cyclopropyl | Cl | | 3,57 | |
| 648 | -CH₂-CH₂-CF₃ | -H | 2,4-Difluorphenyl | Cyclopropyl | Cl | | 3,25 | |
| 649 | n-Propyl | -H | 2,4-Difluorphenyl | Cyclopropyl | Cl | | 3,2 | |
| 650 | Cyclopentyl | -H | 2,4-Difluorphenyl | Cyclopropyl | Cl | | 3,76 | |
| 651 | -i-Propyl | -H | 2,4-Difluorphenyl | Cyclopropyl | Cl | | 3,2 | |
| 652 | Cyclohexyl | -H | 2,4- Difluorphenyl | Cyclopropyl | Cl | | 4,02 | |
| 653 | 1-Cyclohexylethyl | -H | 2,4-Difluorphenyl | Cyclopropyl | Cl | | 4,84 | |
| 654 | 2-Methoxyethyl | -H | 2,4-Difluorphenyl | Cyclopropyl | Cl | | 2,75 | |
| 655 | Cyclopropyl | -H | 2,4-Difluorphenyl | Cyclopropyl | Cl | | 2,95 | |
| 656 | Cyclopropylmethyl | -H | 2,4-Difluorphenyl | Cyclopropyl | Cl | | 3,26 | |
| 657 | -CH₂-C(CH₃)=CH₂ | -H | 2,4-Difluorphenyl | Cyclopropyl | Cl | | 3,22 | |
| 658 | 3-Trifluormethylcyclohexyl | -H | 2,4-Difluorphenyl | Cyclopropyl | Cl | | 3,98 | |
| 659 | 4-Trifluormethylcyclohexyl | -H | 2,4-Difluorphenyl | Cyclopropyl | Cl | | 3,93 | |
| 660 | -CH(CH₃)-CH₂-CH(CH₃)₂ | -H | 2,4-Difluorphenyl | Cyclopropyl | Cl | | 4,24 | |
| 661 | -CH₂-CH₂-N(CH₃)₂ | -CH₃ | 2,4-Difluorphenyl | Cyclopropyl | Cl | | 1,56 | |
| 662 | Propargyl | -CH₃ | 2,4-Difluorphenyl | Cyclopropyl | Cl | | 3,21 | |
| 663 | 1,3-Dioxolan-2-ylmethyl | -CH₃ | 2,4-Difluorphenyl | Cycloprop yl | Cl | | 3,1 | |
| 664 | Allyl | -CH₃ | 2,4-Difluorphenyl | Cyclopropyl | Cl | | 3,61 | |
| 665 | 3-(Dimethylamino)-propyl | -CH₃ | 2,4-Difluorphenyl | Cyclopropyl | Cl | | 1,61 | |
| 666 | -CH₂-CH(OCH₃)₂ | -CH₃ | 2,4-Difluorphenyl | Cyclopropyl | Cl | | 3,24 | |
| 667 | (2-Furyl)methyl | -CH₃ | 2,4-Difluorphenyl | Cyclopropyl | Cl | | 3,63 | |
| 668 | i-Butyl | -CH₃ | 2,4-Difluorphenyl | Cyclopropyl | Cl | | 4,07 | |
| 669 | 2-Methoxyethyl | -CH₃ | 2,4-Difluorphenyl | Cyclopropyl | Cl | | 3,16 | |
| 670 | -CH₂-C(CH₃)=CH₂ | -CH₃ | 2,4-Difluorphenyl | Cyclopropyl | Cl | | 4,05 | |
| 671 | n-Butyl | -CH₃ | 2,4-Difluorphenyl | Cyclopropyl | Cl | | 4,21 | |
| 672 | -CH₂-CF₃ | -H | 2,4,6-Trifluorphenyl | Cyclopropyl | Cl | | 3,25 | |
| 673 | -CH(CF₃)-CH₂-CH₂-CH₂- | | 2,4,6-Trifluorphenyl | Cyclopropyl | Cl | | 4,08 | |
| 674 | -CH₂-CH₂-CH₂-CH₂-CH(CH₃)- | | 2,4,6-Trifluorphenyl | Cyclopropyl | Cl | | 4,6 | |
| 675 | -CH₂-CH₂-CHF-CH₂-CH₂- | | 2,4,6-Trifluorphenyl | Cyclopropyl | Cl | | 3,68 | |
| 676 | i-Butyl | -CH₃ | 2,6-Difluorphenyl | Cyclopropyl | Cl | | 4,08 | |
| 677 | -CH₂-C(CH₃)=CH₂ | -CH₃ | 2,6-Difluorphenyl | Cyclopropyl | Cl | | 3,98 | |
| 678 | -CH₂-CH₂-CH₂-CH(CH₃)- | | 2,6-Difluorphenyl | Cyclopropyl | Cl | | 3,68 | |
| 679 | -CH₂-CH₂-CH₂-CH₂- | | 2,6-Difluorphenyl | Cyclopropyl | Cl | | 3,25 | |
| 680 | -CH₂-CH₂-CH₂-CH₂-CH(CH₃)- | | 2,6-Difluorphenyl | Cyclopropyl | Cl | | 3,17 | |
| 681 | -CH₂-CH₂-CH(CH₃)-CH₂-CH₂- | | 2,6-Difluorphenyl | Cyclopropyl | Cl | | 4,34 | |
| 682 | -CH₂-CH₂-CH₂-CH₂-CH₂- | | 2,6-Difluorphenyl | Cyclopropyl | Cl | | 3,9 | |
| 683 | -CH₂-CH₂-O-CH₂-CH₂- | | 2,6-Difluorphenyl | Cycloprpyl | Cl | | 2,78 | |
| 684 | 1,2-Dimethylpropyl | -H | 2,6-Difluorphenyl | -CH₃ | Cl | | 3,18 | |
| 685 | -CH(CH₃)-C(CH₃)₃ | -H | 2,6-Difluorphenyl | -CH₃ | Cl | | 3,56 | |
| 686 | -O-CH₂-CH₂-CH₂-CH(CH₃)- | | 2,4,6-Trifluorphenyl | -CH₃ | Cl | | 3,42 | |
| 687 | -C₂H₅ | -H | 2,4-Difluorphenyl | -CH₃ | Cl | | 3,08 | |
| 688 | 1,2-Dimethylpropyl | -H | 2-Chlor-6-Fluorphenyl | -CH₃ | Cl | | 3,43 | |
| 689 | AB31 | | 2,4-Difluorphenyl | Cyclopropyl | Cl | | 3,32 | |
| 690 | -O-CH₂-CH₂-CH₂-CH₂- | | 2,4-Difluorphenyl | Cyclopropyl | Cl | | 3,56 | |
| 691 | -NH-CH₂-CH₂-CH₂-CH₂- | | 2-Chlor-4-fluorphenyl | -CH₃ | Cl | | 2,94 | |
| 692 | -NH-CH₂-CH₂-CH₂-CH₂- | | 2,4,6- Trifluorphenyl | -CH₃ | Cl | | 2,83 | |
| 693 | -O-CH₂-CH₂-CH₂-CH(CH₃)- | | 2,4-Difluorphenyl | -CH₃ | Cl | | 3,3 | |
| 694 | -CH₂-CHF₂ | -H | 2,4,6-Trifluorphenyl | -CH₃ | Cl | | 2,41 | |
| 695 | -CH(CH₃)-CH₂-CH₂-O- | | 2,4- Difluorphenyl | -CH₃ | Cl | | 2,79 | |
| 696 | 1,2-Dimethylpropyl | -H | 2-Chlorphenyl | -CH₃ | Cl | | 3,46 | |
| 697 | -CH₂-CHF₂ | -H | 2-Chlor-4-fluorphenyl | -CH₃ | Cl | | 2,54 | |
| 698 | i-Butyl | -CH₃ | 2-Chlor-6-Fluorphenyl | Cyclopropyl | Cl | | 4,32 | |
| 699 | 2-Methoxyethyl | -CH₃ | 2-Chlor-6-Fluorphenyl | Cyclopropyl | Cl | | 3,3 | |
| 700 | -CH₂-C(CH₃)=CH₂ | -CH₃ | 2-Chlor-6-Fluorphenyl | Cyclopropyl | Cl | | 4,21 | |
| 701 | 2-Methoxyethyl | -C₂H₅ | 2-Chlor-6-Fluorphenyl | Cyclopropyl | Cl | | 3,66 | |
| 702 | -CH₂-CH₂-CH₂-CH(CH₃)- | | 2-Chlor-6-Fluorphenyl | Cyclopropyl | Cl | | 3,94 | |
| 703 | -CH(CF₃)-CH₂-CH₂-CH₂- | | 2-Chlor-6-Fluorphenyl | Cyclopropyl | Cl | | 4,1 | |
| 704 | -CH₂-CH₂-CH₂-CH₂- | | 2-Chlor-6-Fluorphenyl | Cyclopropyl | Cl | | 3,55 | |
| 705 | -CH₂-CH₂-CH₂-CH₂-CH(CH₃)- | | 2-Chlor-6-Fluorphenyl | Cyclopropyl | Cl | | 4,54 | |
| 706 | -CH₂-CH₂-CH(CH₃)-CH₂-CH₂- | | 2-Chlor-6-Fluorphenyl | Cyclopropyl | Cl | | 4,59 | |
| 707 | -CH₂-CH₂-CHF-CH₂-CH₂- | | 2-Chlor-6-Fluorphenyl | Cyclopropyl | Cl | | 3,63 | |
| 708 | -CH₂-CH₂-CH₂-CH₂-CH₂- | | 2-Chlor-6-Fluorphenyl | Cyclopropyl | Cl | | 4,19 | |
| 709 | -CH₂-CH₂-O-CH₂-CH₂- | | 2-Chlor-6-Fluorphenyl | Cyclopropyl | Cl | | 3,05 | |
| 710 | 2,2,2-Trifluor-1-methylethyl | -H | 2,4-Dichlorphenyl | Cyclopropyl | Cl | AS | 4,12 | |
| 711 | 2,2,2-Trifluor-1-methylethyl | -H | 2,4-Dichlorphenyl | Cyclopropyl | Cl | BS | 4,19 | |
| 712 | -C₂H₅ | -H | 2,4-Difluorphenyl | Cyclopropyl | Cl | | 2,97 | |
| 713 | -CH₂-CN | -H | 2,4-Difluorphenyl | Cyclopropyl | Cl | | 2,42 | |
| 714 | -CH₂-CF₃ | -H | 2,4-Difluorphenyl | Cyclopropyl | Cl | | 3,17 | |
| 715 | 2-Trifluormethylcyclohexyl | -H | 2,4-Difluorphenyl | Cyclopropyl | Cl | | 4,08 | |
| 716 | 3,5-bis-Trifluormethylcyclohexyl | -H | 2,4-Difluorphenyl | Cyclopropyl | Cl | | 4,31 | |
| 717 | -CH₂-COOC₂H₅ | -CH₃ | 2,4-Difluorphenyl | Cyclopropyl | Cl | | 3,28 | |
| 718 | -CH₂-CH₂-CN | -CH₃ | 2,4-Difluorphenyl | Cyclopropyl | Cl | | 2,79 | |
| 719 | -CH₂-CN | -CH₃ | 2,4-Difluorphenyl | Cyclopropyl | Cl | | 2,79 | |
| 720 | -CH₂-COOCH₃ | -CH₃ | 2,4-Difluorphenyl | Cyclopropyl | Cl | | 2,95 | |
| 721 | -CH₂-CH₂-Cl | -CH₃ | 2,4-Difluorphenyl | Cyclopropyl | Cl | | 3,5 | |
| 722 | -CH₃ | -CH₃ | 2,4-Difluorphenyl | Cyclopropyl | Cl | | 3,06 | |
| 723 | -CH₂-CH₂-N(CH₃)₂ | -C₂H₅ | 2,4Difluorphenyl | Cyclopropyl | Cl | | 1,65 | |
| 724 | -CH₂CH₂-NH₂ | -C₂H₅ | 2,4-Difluorphenyl | Cyclopropyl | Cl | | 1,47 | |
| 725 | -CH₂-CH₂-CN | -C₂H₅ | 2,4-Difluorphenyl | Cyclopropyl | Cl | | 3,07 | |
| 726 | 3-Aminopropyl | n-Propyl | 2,4-Difluorphenyl | Cyclopropyl | Cl | | 1,61 | |
| 727 | -CH₂-COOC₂H₅ | Cycloprop yl | 2,4-Difluorphenyl | Cyclopropyl | Cl | | 3,84 | |
| 728 | AB8 | | 2,4-Difluorphenyl | Cyclopropyl | Cl | | 2,17 | |
| 729 | -CH(CF₃)-CH₂-CH₂-CH₂- | | 2,4-Difluorphenyl | Cyclopropyl | Cl | | 4,14 | |
| 730 | -CH₂-CH₂-CH(COCH₃)-CH₂-CH₂- | | 2,4-Difluorphenyl | Cyclopropyl | Cl | | 3,07 | |
| 731 | -CH₂-CH₂-CH(COOCH₃)-CH₂-CH₂- | | 2,4-Difluorphenyl | Cyclopropyl | Cl | | 3,38 | |
| 732 | -CH₂-CH₂-CHBr-CH₂-CH₂- | | 2,4-Difluorphenyl | Cyclopropyl | Cl | | 4,08 | |
| 733 | -CH(COOCH₃)-CH₂-CH₂-CH₂-CH₂- | | 2,4-Difluorphenyl | Cyclopropyl | Cl | | 3,92 | |
| 734 | AB11 | | 2,4-Difluorphenyl | Cyclopropyl | Cl | | 1,55 | |
| 735 | -CH₂-CH₂-N(CH₃)-CH₂-CH₂- | | 2,4-Difluorphenyl | Cyclopropyl | Cl | | 1,43 | |
| 736 | -CH₂-CH(CH₃)-O-CH(CH₃)-CH₂- | | 2,4-Difluorphenyl | Cyclopropyl | Cl | | 3,63 | |
| 737 | 3-Tolyl | -H | 2,4-Difluorphenyl | Cyclopropyl | Cl | | 3,61 | |
| 738 | 4-Fluorphenyl | -H | 2,4-Difluorphenyl | Cyclo-propyl | Cl | | 3,27 | |
| 739 | -i-Propyl | -H | 2,6-Difluorphenyl | Cyclopropyl | Cl | | 3,14 | |
| 740 | Cyclopentyl | -H | 2,6-Difluorphenyl | Cyclopropyl | Cl | | 3,66 | |
| 741 | -CH₂-CHF₂ | -H | 2,4-Dichlorphenyl | Cyclopropyl | Cl | | 3,42 | |
| 742 | -CH₂-CF₃ | -H | 2,4-Dichlorphenyl | Cyclopropyl | Cl | | 3,72 | |
| 743 | AB31 | | 2,6-Difluorphenyl | Cyclopropyl | Cl | | 3,23 | |
| 744 | i-Butyl -H | | 2,6-Difluorphenyl | Cyclopropyl | Cl | | 3,44 | |
| 745 | (2,2-Dichlorcyclopropyl)methyl | -H | 2-Chlorphenyl | Cyclopropyl | Cl | 3,78 | | |
| 746 | 2,2,2-Trifluor-1-methylethyl | -H | 2-Chlor-4-fluorphenyl | Cyclopropyl | Cl | AS + BS + AR + BR | | |
| 747 | 2,2,2-Trifluor-1-methylethyl | -H | 2-Chlor-4-fluorphenyl | Cyclopropyl | Cl | AS + BR | 3,73 | |
| 748 | 2,2,2-Trifluor-1-methylethyl | -H | 2-Chlor-4-fluorphenyl | Cyclopropyl | Cl | AR + BS | 3,77 | |
| 749 | 1,2-Dimethylpropyl | -H | 2,4-Dichlorphenyl | Cyclopropyl | Cl | | 4,72 | |
| 750 | -NH₂ | i-Butyl | 2,4-Dichlorphenyl | Cyclopropyl | Cl | | 4,36 | |
| 751 | 1,2-Dimethylpropyl | -H | 2,6-Difluorphenyl | Cyclopropyl | Cl | | 3,82 | |
| 752 | -CH₂-C(CH₃)=CH₂ | -C₂H₅ | 2,6-Difluorphenyl | Cyclopropyl | Cl | | 4,31 | |
| 753 | -O-CH₂-CH₂-CH₂-CH₂- | | 2,6-Difluorphenyl | Cyclopropyl | Cl | | 3,47 | |
| 754 | -NH₂ | i-Butyl | 2,6-Difluorphenyl | Cyclopropyl | Cl | | 3,47 | |
| 755 | -NH-CH₂-CH₂-CH₂-CH₂- | | 2,4-Dichlorphenyl | Cyclopropyl | Cl | | 4,02 | 208-9 |
| 756 | 1,2-Dimethylpropyl | -H | 2,4- Dichlorphenyl | Cyclopropyl | Cl | AR | 4,72 | |
| 757 | 1,2-Dimethylpropyl | -H | 2,4-Dichlorphenyl | Cyclopropyl | Cl | BR | 4,72 | |
| 758 | 1,2-Dimethylpropyl | -H | 2,4-Dichlorphenyl | Cyclopropyl | Cl | AS | 4,72 | |
| 759 | 1,2-Dimethylpropyl | -H | 2,4-Dichlorphenyl | Cyclopropyl | Cl | BS | 4,72 | |
| 760 | -CH₂-C(CH₃)=CH₂ | -H | 2,6-Difluorphenyl | Cyclopropyl | Cl | | 3,28 | |
| 761 | 2-Butyl | -H | 2,6-Difluorphenyl | Cyclopropyl | Cl | | 3,44 | |
| 762 | -CH₂-C(CH₃)₃ | -H | 2,6-Difluorphenyl | Cyclopopyl | Cl | | 3,9 | |
| 763 | -CH₂-CHF₂ | -H | 2,6-Difluorphenyl | Cyclopropyl | Cl | | 2,72 | |
| 764 | 2,2,2-Trifluor-1-methylethyl | -H | 2,6-Difluorphenyl | Cyclopropyl | Cl | | 3,98 | |
| 765 | -CH(CH₃)-CH₂-CH₂-O- | | 2,6-Difluorphenyl | Cyclopropyl | Cl | | 3,26 | |
| 766 | -CH₂-CF₃ | -H | 2-Chlor-4-fluorphenyl | -CH₃ | Cl | | 2,8 | |
| 767 | 2,2,2-Trifluor-1-methylethyl | -H | 2-Chlor-4-fluorphenyl | Cyclopropyl | Cl | AS | 3,73 | |
| 768 | 2,2,2-Trifluor-1-methylethyl | -H | 2-Chlor-4-fluorphenyl | Cyclopropyl | Cl | BS | 3,77 | |
| 769 | -CH₂-C(CH₃)=CH₂ | -C₂H₅ | 2,4-Dichlorphenyl | Cyclopropyl | Cl | | 5,25 | |
| 770 | AB32 | | 2,6-Difluorphenyl | Cyclo-1 | Cl | | 3,32 | |
| 771 | -CH₂-CF₃ | -H | 2,6-Difluorphenyl | Cyclopropyl | Cl | | 3,04 | |
| 772 | -NH-CH₂-CH₂-CH₂-CH₂- | | 2,6-Difluorphenyl | Cyclopropyl | Cl | | 3,19 | |
| 773 | -NH₂ | i-Butyl | 2,4,6-Trifluorphenyl | Cyclopropyl | Cl | | 3,65 | 186-8 |
| 774 | AB33 | -H | 2,4-Dichlorphenyl | Cyclopropyl | Cl | | 2,7 | |
| 775 | 1,2-Dimethylpropyl | -H | 2-Chlor-4-fluorphenyl | Cyclopropyl | Cl | AR + BR | 4,23 | |
| 776 | 1,2-Dimethylpropyl | -H | 2-Chlor-4-fluorphenyl | Cyclopropyl | Cl | BR | 4,25 | |
| 777 | 1,2-Dimethylpropyl | -H | 2-Chlor-4-fluorphenyl | Cyclopropyl | Cl | AR | 4,23 | |
| 778 | 1,2-Dimethylpropyl | -H | 2-Chlor-4-fluorphenyl | Cyclopropyl | Cl | AS | 4,23 | |
| 779 | 1,2-Dimethylpropyl | -H | 2-Chlor-4-fluorphenyl | Cyclopropyl | Cl | BS | 4,23 | |
| 780 | AB31 | | 2,4-Dichlorphenyl | -CH₃ | Cl | | 3,41 | |
| 781 | 2,2,2-Trifluor-1-methylethyl | -H | 2,4-Dichlorphenyl | -CH₃ | Cl | AS | 3,55 | 210-1 |
| 782 | 2,2,2-Trifluor-1-methylethyl | -H | 2,4-Dichlorphenyl | -CH₃ | Cl | BS | 3,58 | 216-7 |
| 783 | -CH₂-C(CH₃)=CH₂ | -C₂H₅ | 2,4-Dichlorphenyl | -CH₃ | Cl | | 4,56 | |
| 784 | -NH-CH₂-CH₂-CH₂-CH₂- | | 2,4-Dichlorphenyl | -CH₃ | Cl | | 3,35 | |
| 785 | -O-CH₂-CH₂-CH₂-CH₂- | | 2-Chlor-4-fluorphenyl | Cyclopropyl | Cl | | 3,87 | |
| 786 | AB32 | | 2,4,6-Trifluorphenyl | Cyclopropyl | Cl | | 3,56 | |
| 787 | 1-Methylethylidenamino | i-Butyl | 2,6-Difluorphenyl | Cyclopropyl | Cl | | 4,21 | |
| 788 | 1,2-Dimethylpropyl | -H | 2,4-Dichlorphenyl | -CH₃ | Cl | | 4,06 | 132-35 |
| 789 | i-Butyl | -H | 2,4-Dichlorphenyl | -CH₃ | Cl | | 3,68 | |
| 790 | i-Butyl | -H | 2,4- Dichlorphenyl | Cyclopropyl | Cl | | 4,3 | |
| 791 | -NH₂ | i-Butyl | 2-Chlor-6-Fluorphenyl | Cyclopropyl | Cl | | 3,74 | 175-6 |
| 792 | -CH₂-CH₂-CH₂-CH(CH₃)- | | 2,4-Difluorphenyl | -CF₃ | Cl | | 4,27 | |
| 793 | AB8 | | 2,4-Difluorphenyl | -CF₃ | Cl | | 2,56 | |
| 794 | -CH(CF₃)-CH₂-CH₂-CH₂- | | 2,4-Difluorphenyl | -CF₃ | Cl | | 4,44 | |
| 795 | AB9 | | 2,4-Difluorphenyl | -CF₃ | Cl | | 1,67 | |
| 796 | AB10 | | 2,4-Difluorphenyl | -CF₃ | Cl | | 3,8 | |
| 797 | -CH₂-CH(CH₃)-CH₂-CH(CH₃)-CH₂- | | 2,4-Difluorphenyl | -CF₃ | Cl | | 5,18 | |
| 798 | -CH₂-CH₂-CH(CH₃)-CH₂-CH₂- | | 2,4- Difluorphenyl | -CF₃ | Cl | | 4,8 | |
| 799 | -CH₂-CH(OH)-CH₂-CH₂-CH₂- | | 2,4-Difluorphenyl | -CF₃ | Cl | | 3,07 | |
| 800 | -CH₂-CH₂-C(CH₃)₂-CH₂-CH₂- | | 2,4-Difluorphenyl | -CF₃ | Cl | | 5,03 | |
| 801 | -CH₂-CH=C(C₂H₅)-CH₂-CH₂- | | 2,4-Difluorphenyl | -CF₃ | Cl | | 4,95 | |
| 802 | -CH₂-CH₂-CH=C(CH₃)-CH₂- | | 2,4- Difluorphenyl | -CF₃ | Cl | | 4,55 | |
| 803 | -CH₂-CH₂-CH(COOCH₃)-CH₂-CH₂- | | 2,4-Difluorphenyl | -CF₃ | Cl | | 3,81 | |
| 804 | -CH(COOCH₃)-CH₂-CH₂-CH₂-CH₂- | | 2,4-Difluorphenyl | -CF₃ | Cl | | 4,32 | |
| 805 | -CH₂-CH₂-CH(CF₃)-CH₂-CH₂- | | 2,4-Difluorphenyl | -CF₃ | Cl | | 4,43 | |
| 806 | -CH₂-CH₂-CH(NH-COCH₃)-CH₂-CH₂- | | 2,4-Difluorphenyl | -CF₃ | Cl | | 2,71 | |
| 807 | -CH₂-CH₂-N(CH₃)-CH₂-CH₂- | | 2,4-Difluorphenyl | -CF₃ | Cl | | 1,65 | |
| 808 | -CH₂-CH(CH₃)-O-CH(CH₃)-CH₂- | | 2,4-Difluorphenyl | -CF₃ | Cl | | 4,1 | |
| 809 | -CH₂-CH₂-CH₂-CH₂-CH₂- | | 2,4-Difluorphenyl | -CF₃ | Cl | | 4,45 | |
| 810 | -CH₂-CH₂-S-CH₂-CH₂- | | 2,4-Difluorphenyl | -CF₃ | Cl | | 4,04 | |
| 811 | 2-Tolyl | -H | 2,4-Difluorphenyl | -CF₃ | Cl | | 3,99 | |
| 812 | 3-Tolyl | -H | 2,4-Difluorphenyl | -CF₃ | Cl | | 4,04 | |
| 813 | 4-Tolyl -H | | 2,4- Difluorphenyl | -CF₃ | Cl | | 4,08 | |
| 814 | 4-Fluorphenyl | -H | 2,4-Difluorphenyl | -CF₃ | Cl | | 3,69 | |
| 815 | AB13 | | 2,4-Difluorphenyl | -CF₃ | Cl | | 4,35 | |
| 816 | -NH₂ | i-Butyl | 2,4-Dichlorphenyl | -CH₃ | Cl | | 3,71 | |
| 817 | -O-CH₂-CH₂-CH₂-CH₂- | | 2,4-Dichlorphenyl | -CH₃ | Cl | | 3,67 | |
| 818 | -CH(CH₃)-CH₂-CH(CH₃)₂ | -H | 2,4-Difluorphenyl | -CF₃ | Cl | | 4,73 | |

Die Bestimmung der logP-Werte erfolgte gemäß EEC-Directive 79/831 Annex V. A8 durch HPLC (Gradientenmethode, Acetonitril/0,1 % wässrige Phosphorsäure)
*) bedeutet, dass R¹ und R² gemeinsam mit dem Stickstoffatom, an das sie gebunden sind, einen heterocyclischen Ring bilden.
**) Die Produkte wurden teilweise als Stereoisomere isoliert. "S", bzw. "R" bedeutet S- bzw. R-Konfiguration am Chiralitätszentrum; "AS" bedeutet eine eindeutige aber unbekannte Konfiguration am Atropiezentrum und S-Konfiguration am Chiralitätszentrum. BS bedeutet die jeweils andere eindeutige aber unbekannte Konfiguration am Atropiezentrum und S-Konfiguration am Chiralitätszentrum. "AR" und "BR" bedeuten wiederum die jeweiligen komplementären Konfigurationen am Atropiezentrum gepaart mit der R-Konfiguration am Chiralitätszentrum. Demnach sind bei gleichen Substituenten "AR" und "BS", sowie "AS" und "BR" jeweils Enantiomerenpaare.

| | |
|---|---|
| AB10 | 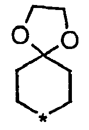 |
| AB11 | 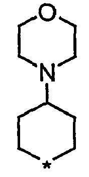 |
| AB12 | 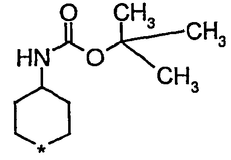 |
| AB14 | 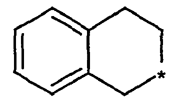 |
| AB8 | 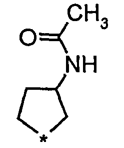 |
| AB9 | 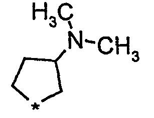 |
| AB28 | 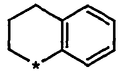 |
| AB3 | 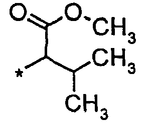 |
| AB30 | 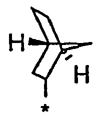 |
| AB31 | 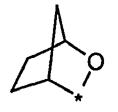 |
| AB32 | 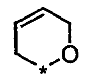 |
| AB33 | 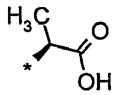 |
| AB4 | 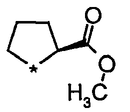 |
| AB5 | 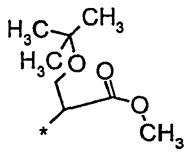 |
| AB6 | 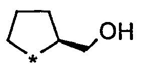 |
| AB7 | 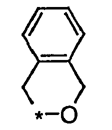 |

* markiert jeweils die Bindungsstelle

### Herstellung von Ausgangsstoffen

### Beispiel 819

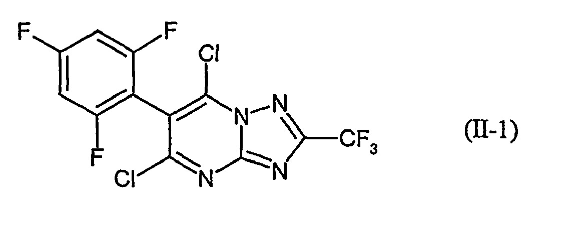

2,6 g (7,43 mMol) 2-Trifluormethyl-6-(2,4,6-trifluorphenyl)-[1,2,4]triazolo[1,5-a]-pyrimidin-5,7-diol werden bei Raumtemperatur in 20 ml Phosphoroxychlorid gelöst, portionsweise mit 1,2 g Phosphorpentachlorid versetzt und anschließend 6 Stunden unter Rückfluss erhitzt. Flüchtige Bestandteile der Reaktionsmischung werden unter vermindertem Druck abdestilliert. Der Rückstand wird mit 20 ml Wasser versetzt und mit 20 ml Dichlormethan extrahiert. Die organische Phase wird über Natriumsulfat getrocknet und an Kieselgel mit Dichlormethan chromatografiert. Man erhält 1,2 g (37,6 % der Theorie) 5,7-Dichlor-2-(trifluormethyl)-6-(2,4,6-trifluorphenyl)-[1,2,4]triazolo[1,5-a]pyrimidin.
HPLC: logP = 3,71

### Beispiel 820

### Verfahren (e), erste Stufe:

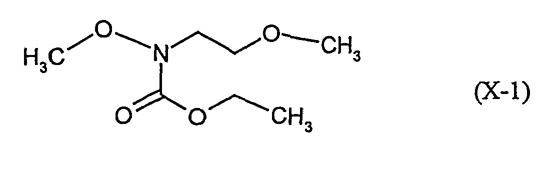

1 000 mg N-Methoxy-carbaminsäureethylester werden in 10,0 ml Dimethylformamid vorgelegt und portionsweise mit 403 mg Natriumhydrid versetzt, wobei die Temperatur durch Kühlung auf 30°C eingestellt wurde. Die Reaktionsmischung wird für 2 Stunden bei 30°C gerührt und anschließend mit 3 500 mg 2-Bromethyl-methylether versetzt. Die Reaktionsmischung wird für 18 Stunden bei 20°C bis 25°C gerührt und anschließend in 20 ml Wasser eingerührt. Die erhaltene Reaktionsmischung wird unter vermindertem Druck zur Trockne eingeengt und viermal mit je 30 ml Dichlormethan extrahiert. Die organischen Extrakte werden über Natriumsulfat getrocknet, filtriert und unter vermindertem Druck zur Trockne eingeengt.

Man erhält 1 200 mg (N-Methoxy-N-methoxyethyl)carbaminsäure-ethylester (Reinheit 77,6 %, Ausbeute 62,6 %).

### Verfahren (e), zweite Stufe:

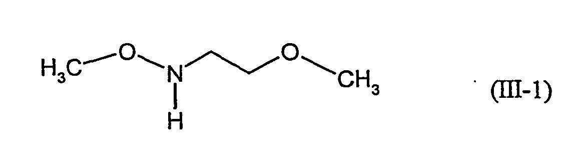

200 mg (N-Methoxy-N-methoxyethyl)-carbaminsäureethylester werden in 4,0 ml wässrigem Ethanol (59 %ig) vorgelegt, mit 240,6 mg Kaliumhydroxid versetzt und für 18 Stunden bei 40°C gerührt. Die Reaktionsmischung wird dann in 50 ml Wasser eingerührt, dreimal mit je 20 ml Diethylether und dreimal mit je 20 ml Dichlormethan extrahiert. Die vereinigten organischen Phasen werden zweimal mit je 20 ml Wasser gewaschen, getrocknet und bei 20°C unter vermindertem Druck auf ein Volumen von 20 ml eingeengt.

Die erhaltene Lösung wird unter Eiskühlung mit 2 ml Salzsäure versetzt, 1 Stunde bei Raumtemperatur gerührt und bei 20°C unter vermindertem Druck zur Trockne eingeengt.

Das erhaltene Produkt wird dreimal mit je 15 ml Methanol digeriert und anschließend bei 20°C unter vermindertem Druck zur Trockne eingeengt.

Man erhält 140 mg N-Methoxy-N-methoxyethylamin-hydrochlorid (Ausbeute 87,6 %).

### Beispiel 821

### Verfahren (f), erste Stufe:

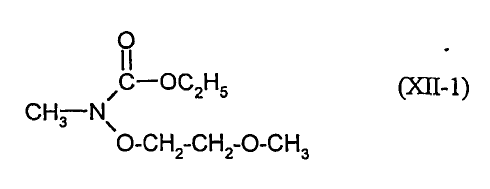

Ein Gemisch aus 1000 mg N-Hydroxy-N-methyl-carbaminsäure-ethylester, 1166 mg 2-Bromethyl-methylether und Rühren auf Rückflusstemperatur erhitzt und dann tropfenweise mit einer Lösung von 493 mg Kaliumhydroxid in 5 ml Ethanol versetzt Man kocht das Reaktionsgemisch 10 Stunden unter Rückfluss und arbeitet dann auf, indem man das Reaktionsgemisch filtriert und das Filtrat unter vermindertem Druck einengt. Der verbleibende Rückstand wird mit einem Gemisch aus Wasser und Essigsäureethylester versetzt. Die organische Phase wird abgetrennt, mit gesättigter, wässriger Ammoniumchlorid-Lösung und dann mit Wasser gewaschen. Anschließend wird die organische Phase über Natriumsulfat getrocknet und unter vermindertem Druck eingeengt. Man erhält auf diese Weise 0,7 g eines Produktes, das gemäß Gaschromatogramm zu 83 % aus (N-Methyl-N-methoxyethoxy)-carbaminsäure-ethylester besteht. Die Ausbeute errechnet sich danach zu 39 % der Theorie.

### Verfahren (f), zweite Stufe:

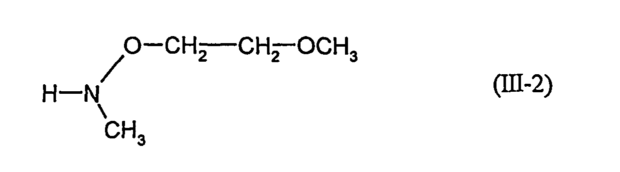

Ein Gemisch aus 200 mg (N-Methyl-N-methoxyethoxy)-carbaminsäure-ethylester, 4 ml Ethanol und 4 ml Wasser wird mit 240,6 mg pulverisiertem Kaliumhydroxid versetzt und 2 Stunden bei 40°C gerührt. Das Reaktionsgemisch wird danach in 50 ml Wasser eingerührt, dann dreimal mit je 20 ml Diethylether und anschließend dreimal mit je 20 ml Methylenchlorid extrahiert. Die vereinigten organischen Phasen werden zweimal mit je 20 ml Wasser gewaschen, über Natriumsulfat getrocknet und bei Raumtemperatur unter vermindertem Druck auf ein Volumen von 20 ml eingeengt. Die erhaltene Lösung wird unter Eiskühlung mit 1 ml etherischer Salzsäure versetzt. Die sich abscheidenden Kristalle werden abfiltriert und getrocknet. Man erhält auf diese Weise 190 mg an N-Methyl-N-methoxyethoxy-amin-hydrochlorid.

### Beispiel 822

### Verfahren (g), erste Stufe:

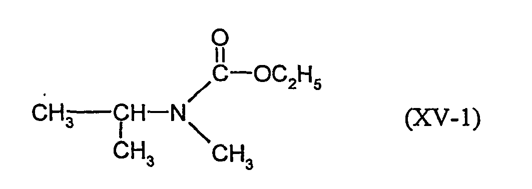

Ein Gemisch aus 2000 mg N-(2,2,2-Trifluor-1-methyl-ethyl)-carbaminsäure-ethylester und 20 ml Tetrahydrofuran wird bei Raumtemperatur unter Rühren mit 475 mg Natriumhydrid versetzt. Danach wird unter Rühren bei Raumtemperatur eine Lösung von 4600 mg Iodmethan in 10 ml Tetrahydrofuran hinzugetropft. Das Reaktionsgemisch wird 16 Stunden bei 50°C gerührt und dann mit Wasser versetzt. Man extrahiert dreimal mit je 20 ml Methylenchlorid, trocknet die vereinigten organischen Phasen über Natriumsulfat und engt unter vermindertem Druck ein. Man erhält 1000 mg eines Produktes, das gemäß Gaschromatogramm zu 75 % aus N-(2,2,2-Trifluor-1-methyl-ethyl)-N-methyl-carbaminsäure-ethylester besteht. Die Ausbeute errechnet sich danach zu 34,86 %.

### Verfahren (g), zweite Stufe:

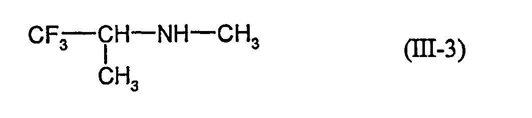

Ein Gemisch aus 1000 mg N-(2,2,2-Trifluor-1-methyl-ethyl)-N-methyl-carbaminsäure-ethylester, 20 ml Ethanol und 20 ml Wasser wird mit 1070 mg pulverisiertem Kaliumhydroxid versetzt und 66 Stunden bei 40°C gerührt. Danach wird das Reaktionsgemisch mit Wasser verdünnt und dreimal mit je 20 ml eines Gemisches extrahiert, das zu gleichen Teilen aus Methylenchlorid und Diethylether besteht. Die vereinigten organischen Phasen werden über Natriumsulfat getrocknet und dann bei Raumtemperatur unter leicht vermindertem Druck eingeengt. Die erhaltene Lösung wird unter Eiskühlung mit etherischer Salzsäure versetzt und 60 Stunden bei Raumtemperatur gerührt. Nach dem Einengen unter vermindertem Druck erhält man 280 mg an N-(2,2,2-Trifluor-1-methyl-ethyl)-N-methylamin-hydrochlorid. Die Ausbeute errechnet sich danach zu 34 % der Theorie.

### Beispiel 823

### Verfahren (h):

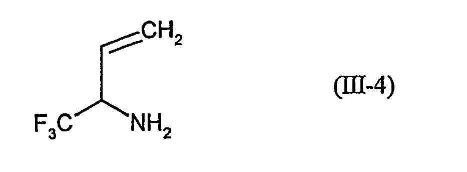

600 mg N(1-Trifluormethyl-2-propen)-carbaminsäurebenzylester werden in 8,0 ml 16 %iger Salzsäure für 1,5 Stunden unter Rückfluss erhitzt. Nach dem Abkühlen auf 20°C wird zweimal mit je 20 ml Diethylether extrahiert.

Die verbleibende wässrige Phase wird unter vermindertem Druck zur Trockne eingeengt und dreimal mit je 10 ml Methanol versetzt. Nach Entfernen des Methanols unter vermindertem Druck werden 310 mg an (1-Trifluormethyl-prop-2-en)-amin-hydrochlorid isoliert. Die Ausbeute errechnet sich danach zu 82,9 % der Theorie.

Nach den zuvor angegebenen Methoden lassen sich auch die in den folgenden Tabellen angegebenen Carbamate herstellen.

**Tabelle 2**

| | | | |
|---|---|---|---|
| 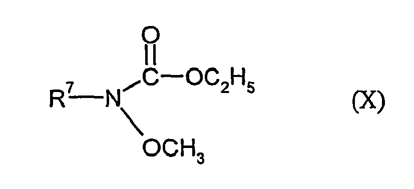 | | | |

| **Beispiel-Nr.** | **Verb.-Nr.** | **R⁷** | **logP** |
|---|---|---|---|
| 824 | X-2 | 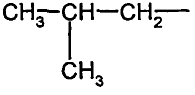 | 2,38 |
| 825 | X-3 | 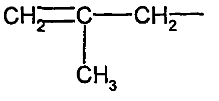 | 2,06 |

**Tabelle 3**

| | | | |
|---|---|---|---|
| 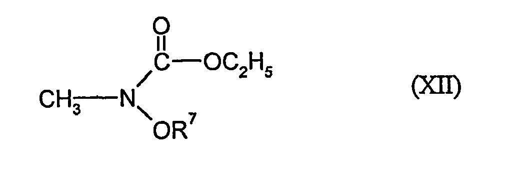 | | | |

| **Beispiel-Nr.** | **Verb.-Nr.** | **R⁷** | **Physikalische Konst.** |
|---|---|---|---|
| 826 | XII-2 | 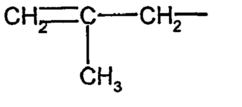 | |

**Tabelle 4**

| | | | |
|---|---|---|---|
| 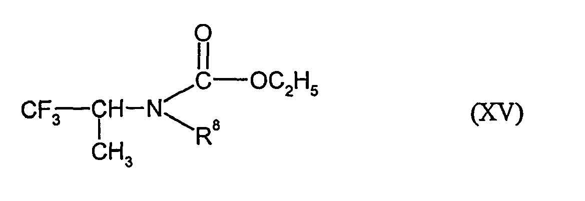 | | | |

| Beispiel-Nr. | Verb.-Nr. | R⁸ | Physikalische Konst. |
|---|---|---|---|
| 827 | XV-2 | -C₂H₅ | ¹H-NMR (400 MHz, CD₃CN): δ (ppm) = 1,13 (t, CH₃CH₂N), 1,21 (t, CH₃CHCF₃), 1,23 (t, CH₃CH₂O), 3,20 (m, CH₂N, CHCF₃), 4,1 (q, CH₃CH₂O). |

Nach den zuvor angegebenen Methoden lassen sich auch die in der folgenden Tabelle aufgeführten Amine herstellen.

**Tabelle 5**

| | | | | |
|---|---|---|---|---|
| 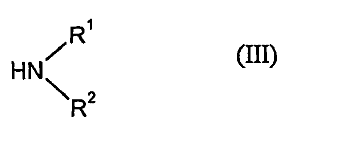 | | | | |

| Beispiel-Nr. | Verb.-Nr. | R¹ | R² | Physikal. Konst. |
|---|---|---|---|---|
| 828 | III-5 | 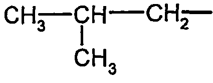 | -OCH₃ | ¹H-NMR (400 MHz, CD₃CN): δ (ppm) = 1,03 (d, CH₃)₂CH), 3,06 (d, CH₂), 3,28 (b, (CH₃)₂CH), 4,01 (s, OCH₃) |
| 829 | III-6 | 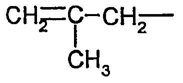 | -OCH₃ | ¹H-NMR (400 MHz, DMSO): δ (ppm) = 1,76 (s, CH₃(CCH₂)CH₂), 3,29 (b, NH, CH₃(CCH₂)CH₂, OCH₃), 7,89, 5,02 (2 s, CH₃(CCH₂)CH₂). |
| 830 | III-7 | 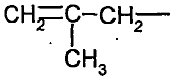 | | |
| 831 | III-8 | 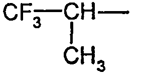 | -C₂H₅ | ¹H-NMR (400 MHz, DMSO): δ (ppm) = 1,06 (m, CH₃CH₂N, CH₃CHCF₃), 3,20 (m, CH₂N), 4,1 (m, CHCF₃). |

Die in den Beispielen 828 bis 831 aufgeführten Amine wurden jeweils in Form ihrer Hydrochloride isoliert und charakterisiert.

### Beispiel 832

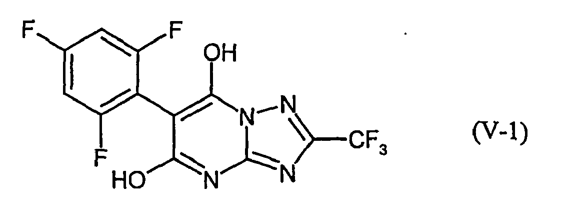

10,1 g (38,5 mmol) 2-(2,4,6-trifluor-phenyl)malonsäuredimethylester und 5,85 g (38,5 mmol) 5-Trifluormethyl-1H-[1,2,4]triazol-3-ylamin werden in 10,1 ml Tri-n-Butylamin 6 Stunden auf 180°C erhitzt, wobei entstehendes Methanol abdestilliert wird. Das Tri-n-Butylamin wird unter stark vermindertem Druck abdestilliert. Man erhält 17,8 g rohes 2-Trifluormethyl-6-(2,4,6-trifluor-phenyl)-[1,2,4]triazolo[1,5-a]-pyrimidin-5,7-diol, das ohne Reinigung weiter umgesetzt wird.
HPLC:logP=0,81

### Beispiel A

### Puccinia-Test (Weizen) / protektiv

| | |
|---|---|
| Lösungsmittel: | 25 Gewichtsteile N,N-Dimethylacetamid |
| Emulgator: | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Puccinia recondita besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle A**

| | | | | |
|---|---|---|---|---|
| Puccinia-Test (Weizen) / protektiv | | | | |

| Wirkstoff | | | Aufwandmenge an Wirkstoff in g/ha | Wirkungsgrad in % |
|---|---|---|---|---|
| Erfindungsgemäß | | | | |
| Chiral | | | | |
| 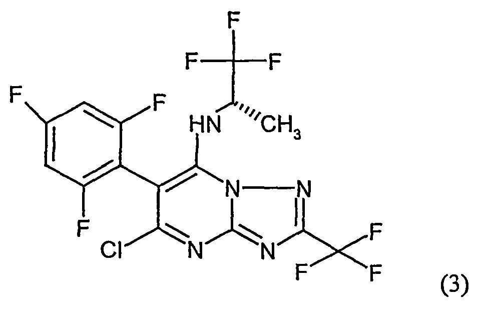 | | | 500 | 100 |

### Beispiel B

Podosphaera-Test (Apfel) / protektiv

| | |
|---|---|
| Lösungsmittel: | 24,5 Gewichtsteile Aceton |
| | 24,5 Gewichtsteile Dimethylacetamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension des Apfelmehltauerreegers Podosphaera leucotricha inokuliert. Die Pflanzen werden dann im Gewächshaus bei ca. 23°C und einer relativen Luftfeuchtigkeit von ca. 70 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle B**

| | | |
|---|---|---|
| Podosphaera-Test (Apfel) / protektiv | | |

| Wirkstoff | Aufwandmenge an Wirkstoff in g/ha | Wirkungsgrad in % |
|---|---|---|
| Erfindungsgemäß | | |
| 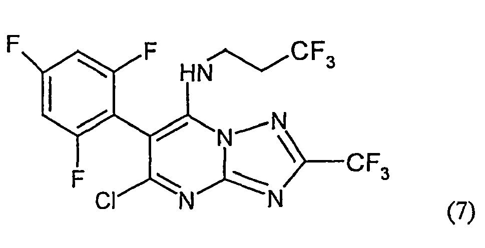 | 100 | 100 |
| 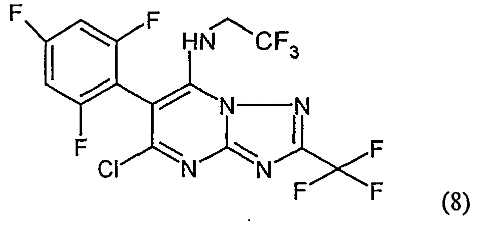 | 100 | 100 |

### Beispiel C

Spaerotheca-Test (Gurke) / protektiv

| | |
|---|---|
| Lösungsmittel: | 49 Gewichtsteile N,N-Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge. 1 Tag nach der Behandlung werden die Pflanzen mit einer Sporensuspension von Sphaerotheca fuliginea inokuliert. Anschließend werden die Pflanzen in einem Gewächshaus bei 70 % relativer Luftfeuchtigkeit und einer Temperatur von 23°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

**Tabelle C**

| | | | |
|---|---|---|---|
| Spaerotheca-Test (Gurke) / protektiv | | | |

| Wirkstoff | | Aufwandmenge an Wirkstoff in g/ha | Wirkungsgrad in % |
|---|---|---|---|
| Erfindungsgemäß: | | | |
| Chiral | | | |
| 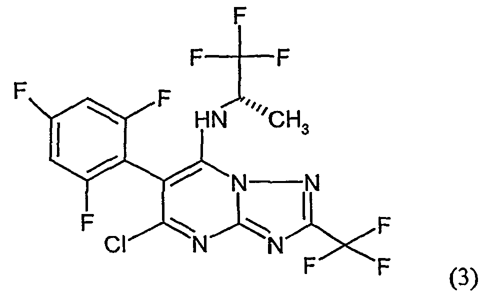 | | 750 | 93 |
| Chiral | | | |
| 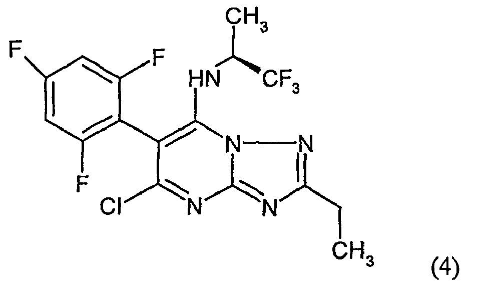 | | 750 | 93 |
| 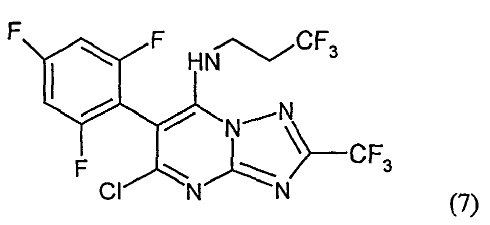 | | 750 | 100 |
| 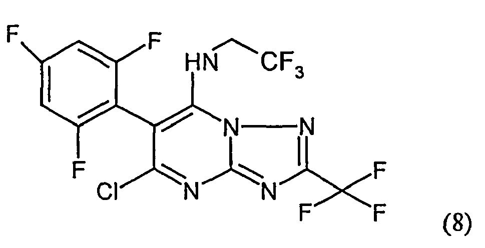 | | 750 | 100 |

## Patentansprüche

1. Triazolopyrimidine der nachfolgenden Formel 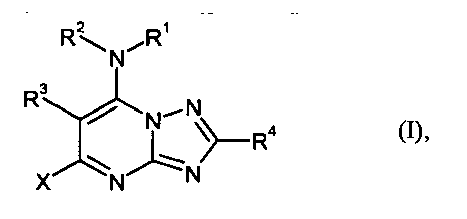 in welcher
R¹ für Amino oder Hydroxy,
für gegebenenfalls durch Halogen, Cyano, Hydroxy, Amino, Phenyl, Heterocyclyl, Alkoxy mit 1 bis 4 Kohlenstoffatomen, Alkoxycarbonyl mit 1 bis 4 Kohlenstoffatomen, Alkylamino mit 1 bis 4 Kohlenstoffatomen, Dialkylamino mit 2 bis 8 Kohlenstoffatomen, Cycloalkyl mit 3 bis 6 Kohlenstoffatomen, Halogencycloalkyl mit 3 bis 6 Kohlenstoffatomen und 1 bis 5 Halogenatomen, Alkylthio mit 1 bis 4 Kohlenstoffatomen, Oxo, Hydroxyimino und/oder Alkoximino mit 1 bis 4 Kohlenstoffatomen substituiertes Alkyl mit 1 bis 6 Kohlenstoffatomen,
für gegebenenfalls durch Halogen, Cycloalkyl, Cyano, Phenyl und/oder Heterocyclyl substituiertes Alkenyl mit 2 bis 6 Kohlenstoffatomen,
für gegebenenfalls durch Halogen, Cycloalkyl, Cyano, Phenyl und/oder Heterocyclyl substituiertes Alkinyl mit 2 bis 6 Kohlenstoffatomen,
für gegebenenfalls durch Halogen, Cycloalkyl, Cyano, Halogenalkyl mit 1 bis 2 Kohlenstoffatomen und 1 bis 5 Halogenatomen, Phenyl und/oder Heterocyclyl substituiertes Cycloalkyl mit 3 bis 7 Kohlenstoffatomen,
für gegebenenfalls durch Halogen, Cycloalkyl, Cyano, Phenyl und/oder Heterocyclyl substituiertes Alkoxy mit 1 bis 7 Kohlenstoffatomen,
für gegebenenfalls durch Halogen, Cycloalkyl, Cyano, Phenyl und/oder Heterocyclyl substituiertes Alkenyloxy mit 2 bis 6 Kohlenstoffatomen,
für gegebenenfalls durch Halogen, Cycloalkyl, Cyano, Phenyl und/oder Heterocyclyl substituiertes Alkinyloxy mit 2 bis 6 Kohlenstoffatomen,
für gegebenenfalls durch Halogen, Cycloalkyl, Cyano, Phenyl und/oder Heterocyclyl substituiertes Cycloalkyloxy mit 3 bis 7 Kohlenstoffatomen,
für gegebenenfalls durch Halogen, Cycloalkyl, Cyano, Phenyl und/oder Heterocyclyl substituiertes Alkylamino mit 1 bis 7 Kohlenstoffatomen,
für gegebenenfalls durch Halogen, Cycloalkyl, Cyano, Phenyl und/oder Heterocyclyl substituiertes Dialkylamino mit 1 bis 7 Kohlenstoffatomen in jedem der Alkylreste,
für gegebenenfalls durch Halogen, Cycloalkyl, Cyano, Phenyl und/oder Heterocyclyl substituiertes Alkenylamino mit 2 bis 6 Kohlenstoffatomen,
für gegebenenfalls durch Halogen, Cycloalkyl, Cyano, Phenyl und/oder Heterocyclyl substituiertes Alkinylamino mit 2 bis 6 Kohlenstoffatomen,
für gegebenenfalls durch Halogen, Cycloalkyl, Cyano, Phenyl und/oder Heterocyclyl substituiertes Cycloalkylamino mit 3 bis 7 Kohlenstoffatomen,
für gegebenenfalls durch Halogen, Cyano, Phenyl und/oder Heterocyclyl substituiertes N-Alkyl-N-Alkenylamino mit 1 bis 6 Kohlenstoffatomen im Alkylteil und 2 bis 6 Kohlenstoffatomen im Alkenylteil,
für gegebenenfalls durch Halogen, Cycloalkyl, Cyano, Phenyl und/oder Heterocyclyl substituiertes N-Cycloalkyl-N-alkyl-amino mit 3 bis 7 Kohlenstoffatomen im Cycloalkylteil und 1 bis 7 Kohlenstoffatomen in Alkylteil,
für gegebenenfalls durch Halogen, Cycloalkyl, Cyano, Phenyl und/oder Heterocyclyl substituiertes Alkylidenamino mit 2 bis 6 Kohlenstoffatomen,
für gegebenenfalls durch Halogen, Cycloalkyl, Cyano, Phenyl und/oder Heterocyclyl substituiertes Phenyl,
für gegebenenfalls durch Halogen, Alkyl, Cycloalkyl, Cyano, Phenyl und/oder Heterocyclyl substituiertes Heterocyclyl mit 5 oder 6 Ringgliedem,
für gegebenenfalls durch Halogen, Alkyl, Cycloalkyl, Cyano, Phenyl und/oder Heterocyclyl substituiertes Heterocyclyloxy mit 5 oder 6 Ringgliedern, oder
für einen Rest der Formel -SR⁵ steht, worin
R⁵ für gegebenenfalls durch Halogen, Cycloalkyl, Cyano, Phenyl und/oder Heterocyclyl substituiertes Alkyl mit 1 bis 6 Kohlenstoffatomen,
für gegebenenfalls durch Halogen, Cycloalkyl, Cyano, Phenyl und/oder Heterocyclyl substituiertes Alkenyl mit 2 bis 6 Kohlenstoffatomen,
für gegebenenfalls durch Halogen, Cycloalkyl, Cyano, Phenyl und/oder Heterocyclyl substituiertes Alkinyl mit 2 bis 6 Kohlenstoffatomen, oder für gegebenenfalls durch Halogen, Cycloalkyl, Cyano, Phenyl und/oder Heterocyclyl substituiertes Cycloalkyl mit 3 bis 7 Kohlenstoffatomen steht, und
wobei
die zuvor genannten Heterocyclyl-Reste einfach bis dreifach; gleichartig oder verschieden substituiert sein können durch Halogen, Hydroxy, Phenyl, 1,2-Dioxyethylen, Alkyl mit 1 bis 4 Kohlenstoffatomen, Halogenalkyl mit 1 oder 2 Kohlenstoffatomen und 1 bis 5 Halogenatomen, Alkoxy mit 1 bis 4 Kohlenstoffatomen, Alkylthio mit 1 bis 4 Kohlenstoffatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, und
die zuvor genannten Heterocyclyl-Reste gesättigt oder teilweise ungesättigt sind,
die zuvor genannten Phenyl-Reste einfach bis dreifach, gleichartig oder verschieden substituiert sein können durch
Halogen, Cyano, Nitro, Amino, Hydroxy, Formyl, Carboxy, Carbamoyl, Thiocarbamoyl,
jeweils geradkettiges oder verzweigtes Alkyl, Alkoxy, Alkylthio, Alkylsulfinyl oder Alkylsulfonyl mit jeweils 1 bis 6 Kohlenstoffatomen,
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen,
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen,
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen,
Cycloalkyl mit 3 bis 6 Kohlenstoffatomen,
in 2,3-Position verknüpftes 1,3-Propandiyl, 1,4-Butandiyl, Methylendioxy (-O-CH₂-O-), 1,2-Ethylendioxy (-O-CH₂-CH₂-O-), wobei diese Reste einfach oder mehrfach, gleichartig oder verschieden substituiert sein können durch Halogen, Alkyl mit 1 bis 4 Kohlenstoffatomen und/oder Halogenalkyl mit 1 bis 4 Kohlenstoffatomen und 1 bis 9 gleichen oder verschiedenen Halogenatomen;
R² für Wasserstoff,
für gegebenenfalls durch Halogen, Cycloalkyl mit 3 bis 6 Kohlenstoffatomen Alkoxy mit 1 bis 4 Kohlenstoffatomen, Alkylthio mit 1 bis 4 Kohlenstoffatomen, Oxo, Hydroximino und/oder Alkoximino mit 1 bis 4 Kohlenstoffatomen substituiertes Alkyl mit 1 bis 4 Kohlenstoffatomen,
für gegebenenfalls durch Halogen und/oder Cycloalkyl mit 3 bis 6 Kohlenstoffatomen substituiertes Alkenyl mit 2 bis 4 Kohlenstoffatomen,
für gegebenenfalls durch Halogen und/oder Cycloalkyl mit 3 bis 6 Kohlenstoffatomen substituiertes Alkinyl mit 2 bis 4 Kohlenstoffatomen, oder
für gegebenenfalls durch Halogen und/oder Cycloalkyl mit 3 bis 6 Kohlenstoffatomen substituiertes Cycloalkyl mit 3 bis 6 Kohlenstoffatomen steht; oder
R¹ und R² gemeinsam mit dem Stickstoffatom, an das sie gebunden sind, für einen 3- bis 6-gliedrigen heterocyclischen Ring stehen, der gesättigt oder teilweise gesättigt ist, der neben dem bereits erwähnten Stickstoffatom noch ein weiteres Heteroatom aus der Reihe Stickstoff, Sauerstoff und Schwefel enthalten kann, und der einfach bis dreifach, gleichartig oder verschieden substituiert sein kann durch
Halogen, Hydroxy, Cyano, Morpholinyl, Amino, einen annelierten Phenylring, eine Methylen- oder Ethylenbrücke, Alkyl mit 1 bis 4 Kohlenstoffatomen,
Halogenalkyl mit 1 bis 4 Kohlenstoffatomen und 1 bis 9 gleichen oder verschiedenen Halogenatomen,
Alkylcarbonylamino mit 1 bis 4 Kohlenstoffatomen, Dialkylamino mit 2 bis 8 Kohlenstoffatomen,
Alkoxycarbonylamino mit 1 bis 4 Kohlenstoffatomen,
Di(alkoxycarbonyl)amino mit 2 bis 8 Kohlenstoffatomen,
Hydroxyalkyl mit 1 bis 4 Kohlenstoffatomen,
Alkoxycarbonyl mit 1 bis 4 Kohlenstoffatomen, und/oder Alkylcarbonyl mit 1 bis 4 Kohlenstoffatomen;
R³ für Phenyl steht, das einfach bis vierfach, gleichartig oder verschieden substituiert sein kann durch
Halogen, Cyano, Nitro, Amino, Hydroxy, Formyl, Carboxy, Carbamoyl, Thiocarbamoyl,
jeweils geradkettiges oder verzweigtes Alkyl, Alkoxy, Alkylthio, Alkylsulfinyl oder Alkylsulfonyl mit jeweils 1 bis 6 Kohlenstoffatomen,
jeweils geradkettiges oder verzweigtes Alkenyl oder Alkenyloxy mit jeweils 2 bis 6 Kohlenstoffatomen,
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen,
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen,
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen,
Cycloalkyl mit 3 bis 6 Kohlenstoffatomen,
in 2,3-Position verknüpftes 1,3-Propandiyl, 1,4-Butandiyl, Methylendioxy (-O-CH₂-O-), 1,2-Ethylendioxy (-O-CH₂-CH₂-O-),
wobei diese Reste einfach oder mehrfach, gleichartig oder verschieden substituiert sein können durch Halogen, Alkyl mit 1 bis 4 Kohlenstoffatomen und/oder Halogenalkyl mit 1 bis 4 Kohlenstoffatomen und 1 bis 9 gleichen oder verschiedenen Halogenatomen;
R⁴ X für gegebenenfalls durch 1 bis 9 Halogenatome substituiertes Alkyl mit 1 bis 4 Kohlenstoffatomen oder für gegebenenfalls durch 1 bis 9 Halogenatome substituiertes Cycloalkyl mit 3 bis 6 Kohlenstoffatomen steht; und für Fluor, Chlor oder Brom steht;
sowie Säure-Additionssalze von denjenigen Verbindungen der Formel (I), in denen R¹ für Amino steht.

2. Triazolopyrimidine wie in Anspruch 1 definiert, wobei
R¹ für Hydroxy, Amino, Methyl, Ethyl, n-Propyl, i-Propyl, n-, i-, s- oder t-Butyl, Methoxymethyl, 2-Methoxy-ethyl, Methylthiomethyl, 2-Methylthio-ethyl, Hydroximinomethyl, Methoximinomethyl, Acetylmethyl, 2-Hydroximino-propyl, 2-Methoximino-propyl, Allyl, 2-Methyl-prop-2-enyl, Propargyl, 2,2,2-Trifluorethyl, 1-(Trifluormethyl)ethyl, 3,3,3-Trifluorpropyl, Cyclopentyl, Cyclohexyl,
für Cyclopropyloxy, Cyclobutyloxy, Cyclopentyloxy, Cyclohexyloxy, Difluormethoxy, Trifluormethoxy, Difluorchlormethoxy, Trifluorethoxy,
für Methylamino, Ethylamino, n- oder i-Propylamino, n-, i-, s- oder t-Butylamino, Dimethylamino, Diethylamino, Trifluorethylamino, Cyclohexylmethylamino, 2-Cyanethylamino, Allylamino, 1-Cyclopropylethylamino, Cyclopropylamino, Cyclobutylamino, Cyclopentylamino, Cyclohexylamino, 1-Methylethylidenarnino, Phenyl, Benzyloxy, Piperidinyl, Morpholinyl, Pyridylmethoxy, Thiazolylmethoxy,
oder
für -S-R⁵ steht, worin
R⁵ für Methyl, Ethyl, n- oder i-Propyl, Difluormethyl, Difluorchlormethyl, Dichlorfluormethyl oder Trifluormethyl steht,
oder
R¹ für (2,2-Dichlorcyclopropyl)methyl, (2-Furyl)methyl, (2-Tetrahydrofuryl)methyl, (2-Tetrahydropyranyl)methyl, 1,2-Dimethylpropyl, 1,3-Dioxolan-2-ylmethyl, 1-Cyclopropylethyl, 1-Cyclopropylethylamino, 1-Methylethylidenamino, 2,2,2-Trifluor-1-methylethyl, 2,4-Dichlorbenzyloxy, 2,6-Dichlorbenzyloxy, 2-Butyl, 2-Chlorbenzyloxy, 2-Fluorcyclopropyl, 2-Hexahydropyranyloxy, 2-Methoxyethyl, 2-Thienylmethyl, 2-Tolyl, 2-Trifluormethylcyclohexyl, 3-(Dimethylamino)propyl, 3,5-bis-Trifluormethylcyclohexyl, 3,5-Dichlorbenzyloxy, 3-Aminopropyl, 3-Chlorbenzyloxy, 3-Tolyl, 3-Trifluormethylbenzyloxy, 3-Trifluormethylcyclohexyl, 3,5-(Bis-trifluormethyl)-cyclohexyl, 2-Trifluormethyl-cyclohexyl, 4-Trifluormethyl-cyclohexyl, 4-Chlorbenzyloxy, 4-Fluorbenzyloxy, 4-Fluorphenyl, 4-Tolyl, 4-Trifluormethylbenzyloxy, 4-Trifluormethylcyclohexyl, Allyl, Allylamino, Allyloxy, Benzyloxy, -C(CH₃)₂-CF₃, -C(CH₃)₂-CH₂-COCH₃, -C₂H₅, -CH(CH₂OH)-COOCH₃, -CH(CH₃)-C(CH₃)₃-, -CH(CH₃)-CH(O-CH₃)₂-, -CH(CH₃)-CH=CH₂-, -CH(CH₃)-CH₂-CH(CH₃)₂-, -CH(CH₃)-CH₂-O-CH₃-, -CH(CH₃)-CH₂-OH, -CH(CH₃)-COOCH₃, -CH(CH₃)-COO-t-butyl, -CH₂-C(CH₃)=CH₂, -CH₂-C(CH₃)₃, -CH₂-CF₃, -CH₂-CH(OCH₃)₂,-CH₂-CH₂-CF₃, -CH₂-CH₂-Cl, -CH₂-CH₂-CN, -CH₂-CH₂-N(CH₃)₂, -CH₂-CH₂-N(CH₃)₂, -CH₂-CH₂-NH₂, -CH₂-CHF₂, -CH₂-CN, -CH₂-COOC₂H₅, -CH₂-COOC₂H₅, -CH₂-COOCH₃, -CH₃, Cyclohexyl, Cyclopentyl, Cyclopropyl, Cyclopropylmethyl, Dimethylamino, i-Butoxy, i-Butyl, i-Propylamino, n-Butoxy, n-Butyl, n-Butylamino, -NH₂, -NH-CH₂-CF₂-CHF₂, -NH-CH₂-CF₃, -NH-CH₂-CH(CH₃)₂, -O-C₂H₅, -O-CH(CH₃)-CH₂-CH₃, -O-CH₃, -OH, O-i-Propyl, Propargyl, t-Butoxy, t-Butyl, t-Butylamino, oder für eine aus den nachfolgenden Gruppierungen 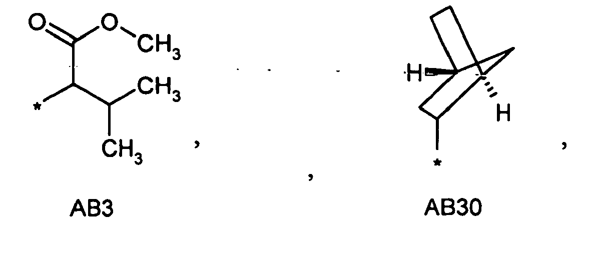 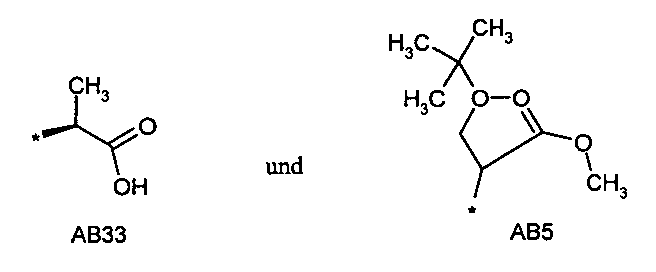 ausgewählte Gruppierung, worin * jeweils die Bindungsstelle markiert, steht,
wobei die zuvor genannten Thiazolyl- und Pyridyl-Reste im Falle von Thiazolyl einfach oder zweifach und im Falle von Pyridyl einfach bis dreifach, jeweils gleichartig oder verschieden substituiert sein können durch Fluor, Chlor Brom, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, Difluormethoxy, Trifluormethoxy, Difluorchlormethoxy, Trifluorethoxy, Difluormethylthio, Difluorchlormethylthio, Dichlorfluormethylthio, Trifluormethylthio und/oder Phenyl, und
wobei die zuvor genannten Phenyl- und Benzyloxy-Reste im Phenylteil einfach bis dreifach, gleichartig oder verschieden substituiert sein können durch
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n-oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl oder Cyclohexyl, oder
in 2,3-Position verknüpftes 1,3-Propandiyl, Methylendioxy (-O-CH₂-O-), oder 1,2-Ethylendioxy (-O-CH₂-CH₂-O-), wobei diese Reste einfach oder mehrfach, gleichartig oder verschieden substituiert sein können durch Fluor, Chlor, Methyl, Ethyl, n-Propyl, i-Propyl und/oder Trifluormethyl;
R² für Wasserstoff, Methyl, Ethyl, n- oder i-Propyl, n-, i-, s- oder t-Butyl, Methoxymethyl, 2-Methoxy-ethyl, Methylthiomethyl, 2-Methylthio-ethyl, Hydroximinomethyl, Methoximinomethyl, Acetylmethyl, 2-Hydroxyimino-propyl, 2-Methoxyimino-propyl, Allyl, Propargyl, 2,2,2-Trifluorethyl, 1-(1,1,1-Trifluormethyl)ethyl, Cyclopropylmethyl, Cyclobutylmethyl, Cyclopentylmethyl oder Cyclohexylmethyl steht;
oder
R¹ und R² gemeinsam mit dem Stickstoffatom, an welches sie gebunden sind, für 1-Pyrrolinyl, 3-Pyrrolinyl, Pyrrolidinyl, Dihydropyridinyl, Piperidinyl, Pyrazolinyl, Pyrazolidinyl, Imidazolinyl, Imidiazolidinyl, 1,2-Diazinan-yl, 1,3-Diazinanyl, Piperazinyl, Oxazolinyl, Oxazolidinyl, Isoxazolyl, Isoxazolidinyl, Dihydrooxazinyl, Morpholinyl, Thiazolinyl, Thiazolidinyl oder Thiomorpholinyl stehen, wobei die genannten Heterocyclen substituiert sein können durch
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n-oder i-Propylthio, Methylsulfinyl, Ethylsulfinyl, Methylsulfonyl, Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Methylamino, Ethylamino, n-oder i-Propylamino, Dimethylamino, Diethylamino, Acetyl, Propionyl, Acetyloxa, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl oder Cyclohexyl,
einen annelierten Phenylring, oder
eine Methandiyl- oder Ethandiyl-Brücke;
R³ für Phenyl steht, das einfach bis dreifach, gleichartig oder verschieden substituiert sein kann durch Fluor, Chlor, Brom, Cyano, Nitro, Formyl, Methyl, Ethyl, n- oder i-Propyl, n-, i-, s- oder t-Butyl, Allyl, Propargyl, Methoxy, Ethoxy, n-oder i-Propoxy, Methylthio, Ethylthio, n- oder i-Propylthio, Methylsulfinyl, Ethylsulfinyl, Methylsulfonyl, Ethylsulfonyl, Allyloxy, Propargyloxy, Trifluormethyl, Trifluorethyl, Difluormethoxy, Trifluormethoxy, Difluorchlormethoxy, Trifluorethoxy, Difluormethylthio, Difluorchlormethylthio, Trifluormetliyithio, Trifluormethylsulfinyl, Trifluormethylsulfonyl, Trichlorethinyloxy, Trifluorethinyloxy, Chlorallyloxy, Iodpropargyloxy, Methylamino, Ethylamino, n- oder i-Propylamino, Dimethylamino, Diethylamino, Acetyl, Propionyl, Acetyloxy, Methoxycarbonyl, Ethoxycarbonyl, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl oder Cyclohexyl,
in 2,3-Position verknüpftes 1,3-Propandiyl, Methylendioxy (-O-CH₂-O-) oder 1,2-Ethylendioxy (-O-CH₂-CH₂-O-), wobei diese Reste einfach oder mehrfach, gleichartig oder verschieden substituiert sein können durch Fluor, Chlor, Methyl, Ethyl, n-Propyl, i-Propyl und/oder Trifluormethyl;
R⁴ für Methyl, Ethyl, n-Propyl, i-Propyl, n-, i-, s- oder t-Butyl, Trifluormethyl, Trifluorethyl oder Cyclopropyl steht; und
X für Fluor oder Chlor steht.

3. Triazolopyrimidine wie in Anspruch 1 oder 2 definiert, wobei
R¹ und R² gemeinsam für eine aus den folgenden Gruppierungen
-CH(CF₃)-CH₂-CH₂-CH₂-, -CH(CF₃)-CH₂-CH₂-CH₂-CH₂-,
-CH(CH₃)-CH=CH-CH(CH₃)-, -CH(CH₃)-CH₂-CH₂-O-,
-CH(COOCH₃)-, -CH₂-CH(CH₃)-CH₂-CH(CH₃)-CH₂-,
-CH₂-CH(CH₃)-O-CH(CH₃)-CH₂-, -CH₂-CH(NH₂)-CH₂-CH₂-
CH₂-C-CH₂-CH(OH)-CH₂-CH₂-, -CH₂-CH(OH)-CH₂-CH₂-CH₂-
-CH₂-CH=C(C₂H₅)-CH₂-CH₂-, -CH₂-CH₂-C(CH₃)₂-CH₂-CH₂-
-CH₂-CH₂-CH₂-, -CH₂-CH₂-CH(CF₃)-CH₂-CH₂-
-CH₂-CH₂-CH(CH₃)-CH₂-CH₂-, -CH₂-CH₂-CH(CH₃)-CH₂-CH₂-
-CH₂-CH₂-CH(COCH₃)-CH₂-CH₂-, -CH₂-CH₂-CH(COOCH₃)-CH₂-CH₂-
-CH₂-CH₂-CH(NH-COCH₃)-CH₂-CH₂-, -CH₂-CH₂-CH(OH)-CH₂-CH₂-
-CH₂-CH₂-CH=C(CH₃)-CH₂-, -CH₂-CH₂-CH=CH-CH₂-
-CH₂-CH₂-CH₂-CH(CH₃)-, -CH₂-CH₂-CH₂-CH(CH₃)-CH₂-
-CH₂-CH₂-CH₂-CH₂-, -CH₂-CH₂-CH₂-CH₂-CH(CH₃)-
-CH₂-CH₂-CH₂-CH₂-CH₂-, -CH₂-CH₂-CHBr-CH₂-CH₂-
-CH₂-CH₂-CHF-CH₂-CH₂-, -CH₂-CH₂-N(CH₃)-CH₂-CH₂-
-CH₂-CH₂-O-CH₂-CH₂-, -CH₂-CH₂-S-CH₂-CH₂-
-CH₂-S-CH₂-CH₂-, -NH-CH₂-CH₂-CH₂-CH₂-
-O-CH₂-CH₂-CH₂-CH(CH₃)- und -O-CH₂-CH₂-CH₂-CH₂- ausgewählte
Gruppierung stehen, oder
für eine aus den folgenden Gruppierungen 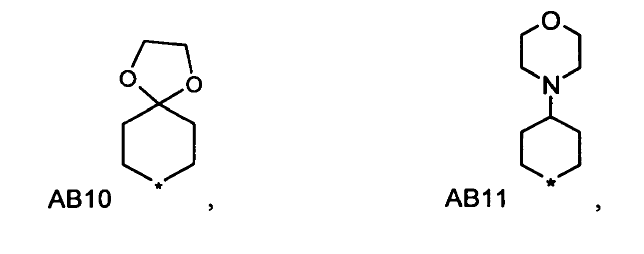 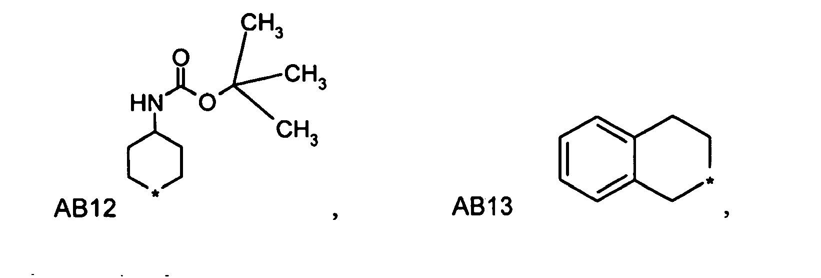 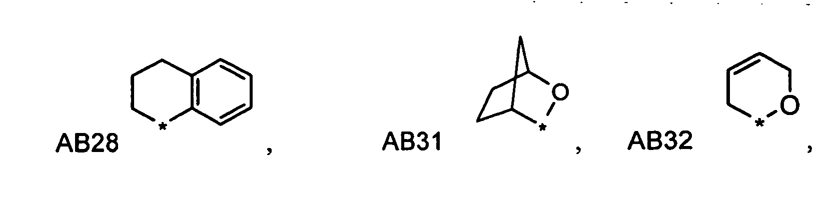 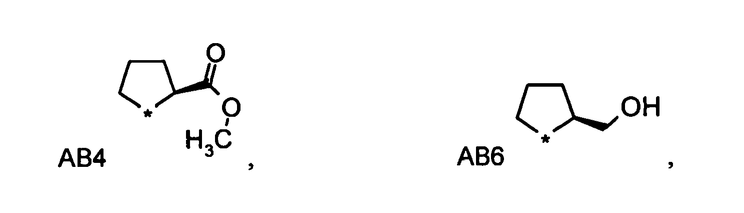 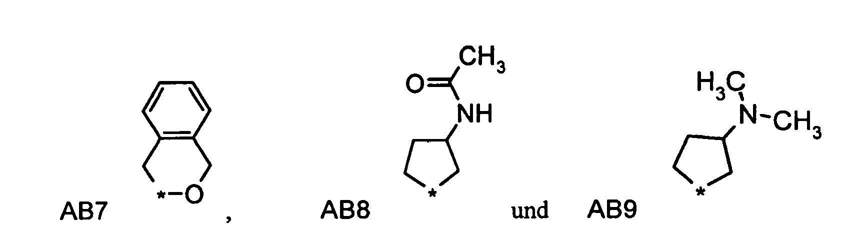 ausgewählte Gruppierung stehen, in denen * für das Stickstoffatom steht, an welches die Reste R¹, R² gebunden sind;
R² für Wasserstoff, Methyl, Ethyl, n-, i-Propyl, n-, i-, s- oder t-Butyl steht;
R³ für Phenyl steht, welches einfach bis dreifach, gleich oder verschieden durch Fluor und/oder Chlor in den Positionen 2, 4 und 6 substituiert ist; und
X für Chlor steht.

4. Triazolopyrimidine wie in irgendeinem der Ansprüche 1 bis 3 definiert, wobei R³ für 2,4-disubstituiertes, 2,6-disubstituiertes oder 2,4,6-trisubstituiertes Phenyl steht.

5. Triazolopyrimidine wie in irgendeinem der Ansprüche 1 bis 4 definiert, wobei R⁴ für Cyclopropyl steht.

6. Verfahren zur Herstellung von Triazolopyrimidinen der Formel (I) gemäß irgendeinem der Ansprüche 1 bis 5, **dadurch gekennzeichnet, dass** man
(a) Dihalogen-triazolopyrimidine der Formel 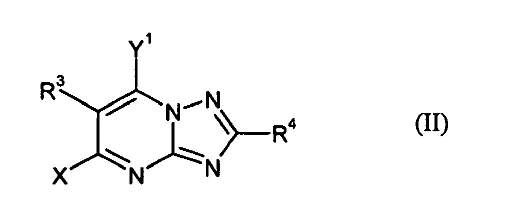 in welcher
R³, R⁴ und X die oben angegebenen Bedeutungen haben und
Y¹ für Halogen steht,
mit Aminen der Formel 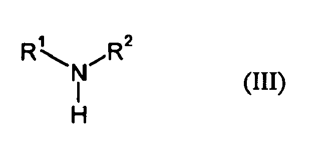 in welcher
R¹ und R² die oben angegebenen Bedeutungen haben,
gegebenenfalls in Gegenwart eines Verdünnungsmittels und gegebenenfalls in Gegenwart eines Säureakzeptors umsetzt, oder
(b) Triazolopyrimidine der Formel 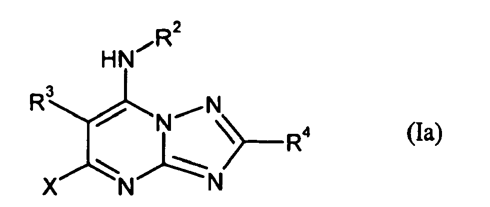 in-welcher
R², R³, R⁴ und X die oben angegebenen Bedeutungen haben,
mit Sulfensäurehalogeniden der Formel
Y²-S-R⁵ (IV),
in welcher
R⁵ die oben angegebenen Bedeutungen hat und
Y² für Halogen steht,
gegebenenfalls in Gegenwart eines Verdünnungsmittels und gegebenenfalls in Gegenwart eines Säureakzeptors umsetzt,
und gegebenenfalls an die so erhaltenen Verbindungen der Formel (I), in denen R¹ für Amino steht,
eine Säure addiert.

7. Mittel zur Bekämpfung von unerwünschten Mikroorganismen, **gekennzeichnet durch** einen Gehalt an mindestens einem Triazolopyrimidin der Formel (I) gemäß irgendeinem der Ansprüche 1 bis 5 bzw. an einem Säureadditions-Salz davon neben Streckmitteln und/oder oberflächenaktiven Stoffen.

8. Verwendung von Triazolopyrimidinen der Formel (I) gemäß irgendeinem der Ansprüche 1 bis 5 bzw. von deren Säureadditions-Salzen zur Bekämpfung von unerwünschten Mikroorganismen.

9. Verfahren zur Bekämpfung von unerwünschten Mikroorganismen, **dadurch gekennzeichnet, dass** man Triazolopyrimidine der Formel (I) gemäß irgendeinem der Ansprüche 1 bis 5 bzw. deren säureadditions-Salze auf die unerwünschten Mikroorganismen und/oder deren Lebensraum ausbringt.

10. Verfahren zur Herstellung von Mitteln zur Bekämpfung von unerwünschten Mikroorganismen, **dadurch gekennzeichnet, dass** man Triazolopyrimidine der Formel (I) gemäß irgendeinem der Ansprüche 1 bis 5 bzw. deren Säureadditions-Salze mit Streckmitteln und/oder oberflächenaktiven Stoffen vermischt.

11. Dihalogen-triazolopyrimidine der Formel 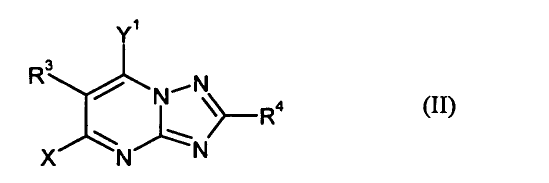 in welcher
R³, R⁴ und X die oben angegebenen Bedeutungen haben und
Y¹ für Halogen steht.

12. Verfahren zur Herstellung von Dihalogen-triazolopyrimidinen der Formel (II) gemäß Anspruch 11, **dadurch gekennzeichnet, dass** man
(c) Dihydroxy-triazolopyrimidine der Formel 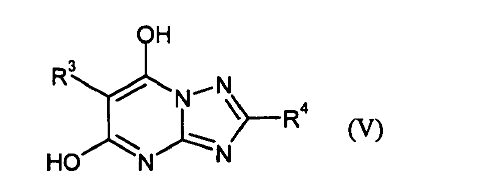 in welcher R³ und R⁴ die oben angegebenen Bedeutungen haben,
mit Halogenierungsmitteln, gegebenenfalls in Gegenwart eines
Verdünnungsmittels umsetzt.

13. Dihydroxy-triazolopyrimidine der Formel 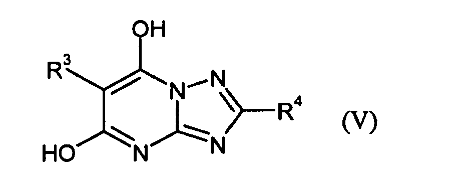 in welcher R³ und R⁴ die oben angegebenen Bedeutungen haben.

14. Verfahren zur Herstellung von Dihydroxy-triazolopyrimidinen der Formel (V) gemäß Anspruch 13, **dadurch gekennzeichnet, dass** man
(d) Arylmalonester der Formel 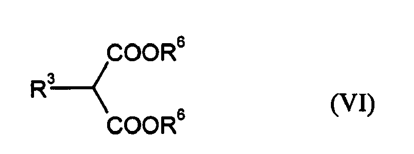 in welcher
R³ die oben angegebenen Bedeutungen hat und
R⁶ für Alkyl mit 1 bis 4 Kohlenstoffatomen steht,
mit Aminotriazolen der Formel 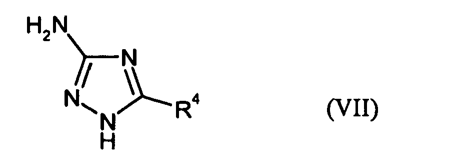 in welcher
R⁴ die oben angegebenen Bedeutungen hat,
gegebenenfalls in Gegenwart eines Verdünnungsmittels und gegebenenfalls in Gegenwart eines Säurebindemittels umsetzt.

15. Amine der Formel (IIIa) 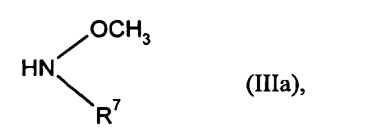 in welcher
R⁷ für Isobutyl, 2-Methoxyethyl oder für 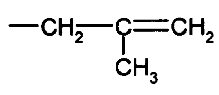 steht.

16. Verfahren zur Herstellung von Aminen der Formel (IIIa) gemäß Anspruch 15, **dadurch gekennzeichnet, dass** man
(e) in einer ersten Stufe N-Methoxycarbaminsäure-ethylester der Formel (VIII) 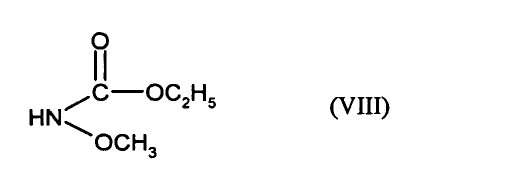 mit Halogenverbindungen der Formel (IX),
R⁷-X¹ (IX)
in welcher
R⁷ die oben angegebenen Bedeutungen hat und
X¹ für Brom oder Iod steht,
in Gegenwart einer Base und in Gegenwart eines Verdünnungsmittels umsetzt und die entstehenden Carbamate der Formel (X), 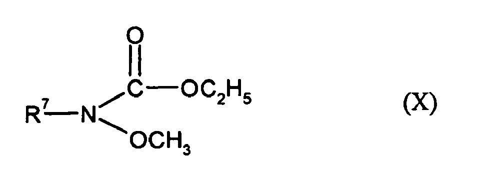 in welcher
R⁷ die oben angegebenen Bedeutungen hat,
in einer zweiten Stufe mit Kaliumhydroxid in Gegenwart von Ethanol und Wasser umsetzt.

17. Amine der Formel (IIIb), 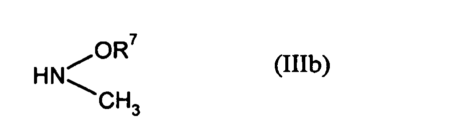 in welcher
R⁷ für Isobutyl, 2-Methoxyethyl oder für 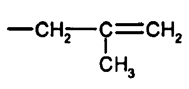 steht.

18. Verfahren zur Herstellung von Aminen der Formel (IIIb) gemäß Anspruch 17, **dadurch gekennzeichnet, dass** man
(f) in einer ersten Stufe N-Hydroxy-N-methyl-carbaminsäure-ethylester der Formel (XI) 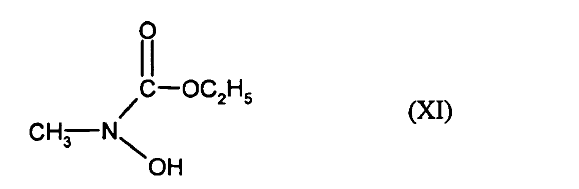 mit Halogenverbindungen der Formel (IX),
R⁷-X¹ (IX)
in welcher
R⁷ und X¹ die oben angegebenen Bedeutungen haben,
in Gegenwart einer Base und in Gegenwart eines Verdünnungsmittels umsetzt und die entstehenden Carbamate der Formel (XII), 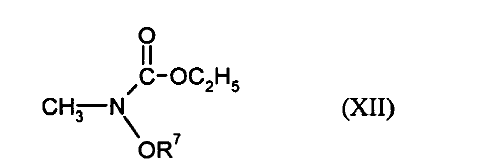 in welcher
R⁷ die oben angegebenen Bedeutungen hat,
in einer zweiten Stufe mit Kaliumhydroxid in Gegenwart von Ethanol und Wasser umsetzt.

19. Trifluorisopropylamine der Formel (IIIc), 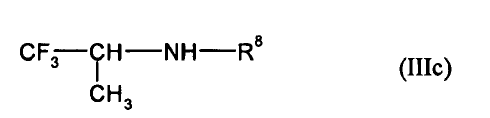 in welcher
R⁸ für Methyl, Ethyl oder Propyl steht.

20. Verfahren zur Herstellung von Trifluorisopropylaminen der Formel (IIIc) gemäß Anspruch 19, **dadurch gekennzeichnet, dass** man
(g) in einer ersten Stufe N-Trifluorisopropyl-carbaminsäure-ethylester der Formel (XIII) 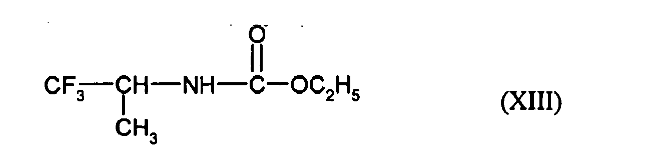 mit Halogenverbindungen der Formel (XIV),
R⁸-X¹ (XIV)
in welcher
R⁸ und X¹ die oben angegebenen Bedeutungen haben, in Gegenwart einer Base und in Gegenwart eines Verdünnungsmittels umsetzt und die entstehenden Carbamate der Formel (XV), 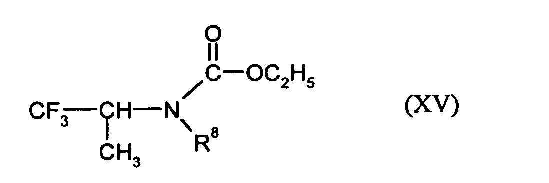 in welcher
R⁸ die oben angegebenen Bedeutungen hat,
in einer zweiten Stufe mit Kaliumhydroxid in Gegenwart von Ethanol und Wasser umsetzt.

21. Die Verbindung 3-Trifluor-methyl-3-amino-propen der Formel (III-4)

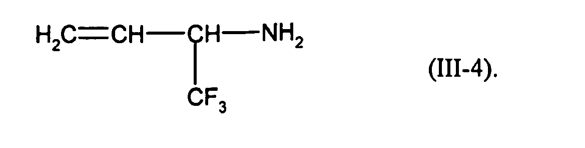

22. Verfahren zur Herstellung von 3-Trifluormethyl-3-amino-propen der Formel (III-4) gemäß Anspruch 21, **dadurch gekennzeichnet, dass** man
(h) das Carbamat der Formel (XVI) 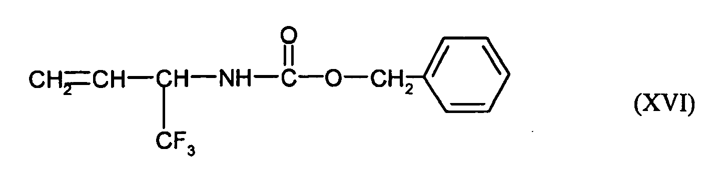 mit wässriger Salzsäure umsetzt.

23. Carbamate der Formel (X), 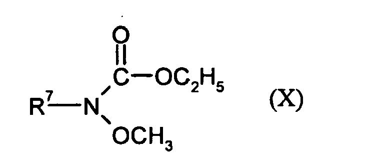 in welcher
R⁷ für Isobutyl, 2-Methoxyethyl oder für 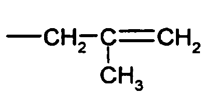 steht.

24. Carbamate der Formel (XII), 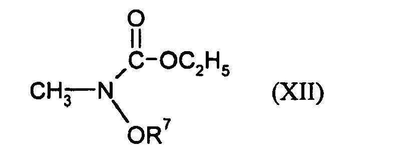 in welcher
R⁷ für Isobutyl, 2-Methoxyethyl oder für 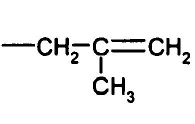 steht.

25. Carbamate der Formel (XIII), 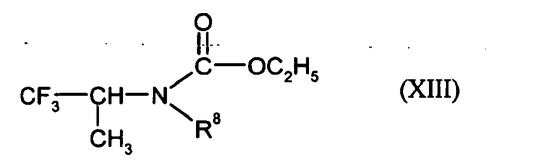 in welcher
R⁸ für Methyl, Ethyl oder Propyl steht.

## Claims

1. Triazolopyrimidines of the following formula 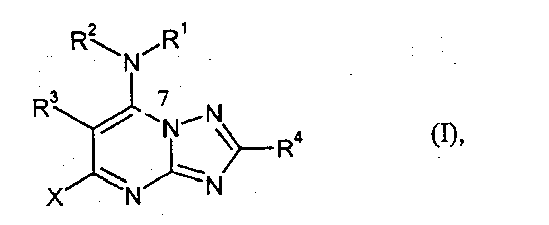 in which
R¹ represents amino or hydroxyl,
represents alkyl having 1 to 6 carbon atoms which is optionally substituted by halogen, cyano, hydroxyl, amino, phenyl, heterocyclyl, alkoxy having 1 to 4 carbon atoms, alkoxycarbonyl having 1 to 4 carbon atoms, alkylamino having 1 to 4 carbon atoms, dialkylamino having 2 to 8 carbon atoms, cycloalkyl having 3 to 6 carbon atoms, halogenocycloalkyl having 3 to 6 carbon atoms and 1 to 5 halogen atoms, alkylthio having 1 to 4 carbon atoms, oxo, hydroxyimino and/or alkoxyimino having 1 to 4 carbon atoms,
represents alkenyl having 2 to 6 carbon atoms which is optionally substituted by halogen, cycloalkyl, cyano, phenyl and/or heterocyclyl,
represents alkinyl having 2 to 6 carbon atoms which is optionally substituted by halogen, cycloalkyl, cyano, phenyl and/or heterocyclyl,
represents cycloalkyl having 3 to 7 carbon atoms which is optionally substituted by halogen, cycloalkyl, cyano, halogenoalkyl having 1 to 2 carbon atoms and 1 to 5 halogen atoms, phenyl and/or heterocyclyl,
represents alkoxy having 1 to 7 carbon atoms which is optionally substituted by halogen, cycloalkyl, cyano, phenyl and/or heterocyclyl,
represents alkenyloxy having 2 to 6 carbon atoms which is optionally substituted by halogen, cycloalkyl, cyano, phenyl and/or heterocyclyl,
represents alkinyloxy having 2 to 6 carbon atoms which is optionally substituted by halogen, cycloalkyl, cyano, phenyl and/or heterocyclyl,
represents cycloalkyloxy having 3 to 7 carbon atoms which is optionally substituted by halogen, cycloalkyl, cyano, phenyl and/or heterocyclyl,
represents alkylamino having 1 to 7 carbon atoms which is optionally substituted by halogen, cycloalkyl, cyano, phenyl and/or heterocyclyl,
represents dialkylamino having 1 to 7 carbon atoms in each of the alkyl radicals which is optionally substituted by halogen, cycloalkyl, cyano, phenyl and/or heterocyclyl,
represents alkenylamino having 2 to 6 carbon atoms which is optionally substituted by halogen, cycloalkyl, cyano, phenyl and/or heterocyclyl,
represents alkinylamino having 2 to 6 carbon atoms which is optionally substituted by halogen, cycloalkyl, cyano, phenyl and/or heterocyclyl,
represents cycloalkylamino having 3 to 7 carbon atoms which is optionally substituted by halogen, cycloalkyl, cyano, phenyl and/or heterocyclyl,
represents optionally halogen-, cyano-, phenyl- and/or heterocyclyl-substituted N-alkyl-N-alkenylamino having 1 to 6 carbon atoms in the alkyl moiety and 2 to 6 carbon atoms in the alkenyl moiety,
represents N-cycloalkyl-N-alkyl-amino having 3 to 7 carbon atoms in the cycloalkyl moiety and 1 to 7 carbon atoms in the alkyl moiety which is optionally substituted by halogen, cycloalkyl, cyano, phenyl and/or heterocyclyl,
represents alkylideneamino having 2 to 6 carbon atoms which is optionally substituted by halogen, cycloalkyl, cyano, phenyl and/or heterocyclyl,
represents phenyl which is optionally substituted by halogen, cycloalkyl, cyano, phenyl and/or heterocyclyl,
represents heterocyclyl having 5 or 6 ring members which is optionally substituted by halogen, alkyl, cycloalkyl, cyano, phenyl and/or heterocyclyl or
represents heterocyclyloxy having 5 or 6 ring members which is optionally substituted by halogen, alkyl, cycloalkyl, cyano, phenyl and/or heterocyclyl, or
represents a radical of the formula -SR⁵, in which
R⁵ represents alkyl having 1 to 6 carbon atoms which is optionally substituted by halogen, cycloalkyl, cyano, phenyl and/or heterocyclyl,
represents alkenyl having 2 to 6 carbon atoms which is optionally substituted by halogen, cycloalkyl, cyano, phenyl and/or heterocyclyl, represents alkinyl having 2 to 6 carbon atoms which is optionally substituted by halogen, cycloalkyl, cyano, phenyl and/or heterocyclyl or
represents cycloalkyl having 3 to 7 carbon atoms which is optionally substituted by halogen, cycloalkyl, cyano, phenyl and/or heterocyclyl, and
where
the heterocyclyl radicals mentioned above may be mono- to trisubstituted by identical or different substituents from the group consisting of halogen, hydroxyl, phenyl, 1,2-dioxyethylene, alkyl having 1 to 4 carbon atoms, halogenoalkyl having 1 or 2 carbon atoms and 1 to 5 halogen atoms, alkoxy having 1 to 4 carbon atoms, alkylthio having 1 to 4 carbon atoms, halogenoalkoxy having 1 or 2 carbon atoms and 1 to 5 halogen atoms, halogenoalkylthio having 1 or 2 carbon atoms and 1 to 5 halogen atoms, and
the heterocyclyl radicals mentioned above are saturated or partially unsaturated,
and the phenyl radicals mentioned above may be mono- to trisubstituted by identical or different substituents from the group consisting of
halogen, cyano, nitro, amino, hydroxyl, formyl, carboxyl, carbamoyl, thiocarbamoyl;
in each case straight-chain or branched alkyl, alkoxy, alkylthio, alkylsulphinyl or alkylsulphonyl having in each case 1 to 6 carbon atoms;
in each case straight-chain or branched alkenyl or alkenyloxy having
in each case 2 to 6 carbon atoms;
in each case straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms;
in each case straight-chain or branched halogenoalkenyl or halogenoalkenyloxy having in each case 2 to 6 carbon atoms and 1 to 13 identical or different halogen atoms;
in each case straight-chain or branched alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties;
cycloalkyl having 3 to 6 carbon atoms,
1,3-propanediyl, 1,4-butanediyl, methylenedioxy (-O-CH₂-O-) or 1,2-ethylenedioxy (-O-CH₂-CH₂-O-) which is attached in the 2,3-position, where these radicals may be mono- or polysubstituted by identical or different substituents from the group consisting of halogen, alkyl having 1 to 4 carbon atoms and/or halogenoalkyl having 1 to 4 carbon atoms and 1 to 9 identical or different halogen atoms,
R² represents hydrogen,
represents alkyl having 1 to 4 carbon atoms which is optionally substituted by halogen, cycloalkyl having 3 to 6 carbon atoms, alkoxy having 1 to 4 carbon atoms, alkylthio having 1 to 4 carbon atoms, oxo, hydroximino and/or alkoximino having 1 to 4 carbon atoms, represents alkenyl having 2 to 4 carbon atoms which is optionally substituted by halogen and/or cycloalkyl having 3 to 6 carbon atoms,
represents alkinyl having 2 to 4 carbon atoms which is optionally substituted by halogen and/or cycloalkyl having 3 to 6 carbon atoms or
represents cycloalkyl having 3 to 6 carbon atoms which is optionally substituted by halogen and/or cycloalkyl having 3 to 6 carbon atoms,
R¹ and R² represent, together with the nitrogen atom to which they are attached, a 3-to 6-membered heterocyclic ring which is saturated or partially saturated, which may in addition to the nitrogen atom already mentioned contain a further heteroatom from the series consisting of nitrogen, oxygen and sulphur, and which may be substituted from one to three times by identical or different substituents selected from
halogen, hydroxyl, cyano, morpholinyl, amino, a fused phenyl ring or a methylene or ethylene bridge, alkyl having 1 to 4 carbon atoms,
halogenoalkyl having 1 to 4 carbon atoms and 1 to 9 identical or different halogen atoms;
alkylcarbonylamino having 1 to 4 carbon atoms,
dialkylamino having 2 to 8 carbon atoms,
alkoxycarbonylamino having 1 to 4 carbon atoms,
di(alkoxycarbonyl)amino having 2 to 8 carbon atoms,
hydroxyalkyl having 1 to 4 carbon atoms,
alkoxycarbonyl having 1 to 4 carbon atoms and/or
alkylcarbonyl having 1 to 4 carbon atoms,
R³ represents phenyl which may be mono- to tetrasubstituted by identical or different substituents from the group consisting of
halogen, cyano, nitro, amino, hydroxyl, formyl, carboxyl, carbamoyl, thiocarbamoyl;
in each case straight-chain or branched alkyl, alkoxy, alkylthio, alkylsulphinyl or alkylsulphonyl having in each case 1 to 6 carbon atoms;
in each case straight-chain or branched alkenyl or alkenyloxy having in each case 2 to 6 carbon atoms;
in each case straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms;
in each case straight-chain or branched halogenoalkenyl or halogenoalkenyloxy having in each case 2 to 6 carbon atoms and 1 to 11 identical or different halogen atoms;
in each case straight-chain or branched alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties;
cycloalkyl having 3 to 6 carbon atoms,
1,3-propanediyl, 1,4-butanediyl, methylenedioxy (-O-CH₂-O-) or 1,2-ethylenedioxy (-O-CH₂-CH₂-O-) which is attached in the 2,3-position,
where these radicals may be mono- or polysubstituted by identical or different substituents from the group consisting of halogen, alkyl having 1 to 4 carbon atoms and/or halogenoalkyl having 1 to 4 carbon atoms and 1 to 9 identical or different halogen atoms,
R⁴ represents alkyl having 1 to 4 carbon atoms which is optionally substituted by 1 to 9 halogen atoms or represents cycloalkyl having 3 to 6 carbon atoms which is optionally substituted by 1 to 9 halogen atoms,
X represents fluorine, chlorine or bromine,
and also addition salts of those compounds of the formula (I) in which R¹ represents amino.

2. Triazolopyrimidines as defined in Claim 1, in which
R¹ represents hydroxyl, amino, methyl, ethyl, n-propyl, isopropyl, n-, iso-, s- or t-butyl, methoxymethyl, 2-methoxyethyl, methylthiomethyl, 2-methylthioethyl, hydroximinomethyl, methoximinomethyl, acetylmethyl, 2-hydroximinopropyl, 2-methoximinopropyl, allyl, 2-methylprop-2-enyl, propargyl, 2,2,2-trifluoroethyl, 1-(trifluoromethyl)ethyl, 3,3,3-trifluoropropyl, cyclopentyl, cyclohexyl,
represents cyclopropyloxy, cyclobutyloxy, cyclopentyloxy, cyclohexyloxy, difluoromethoxy, trifluoromethoxy, difluorochloromethoxy, trifluoroethoxy,
represents methylamino, ethylamino, n- or isopropylamino, n-, iso-, s- or t-butylamino, dimethylamino, diethylamino, trifluoroethylamino, cyclohexylmethylamino, 2-cyanoethylamino, allylamino, 1-cyclopropylethylamino, cyclopropylamino, cyclobutylamino, cyclopentylamino, cyclohexylamino, 1-methylethylideneamino, phenyl, benzyloxy, piperidinyl, morpholinyl, pyridylmethoxy, thiazolylmethoxy,
or
represents -S-R⁵, in which
R⁵ represents methyl, ethyl, n- or isopropyl, difluoromethyl, difluorochloromethyl, dichlorofluoromethyl or trifluoromethyl,
or
R¹ represents (2,2-dichlorocyclopropyl)methyl, (2-furyl)methyl, (2-tetrahydrofuryl)methyl, (2-tetrahydropyranyl)methyl, 1,2-dimethylpropyl, 1,3-dioxolan-2-ylmethyl, 1-cyclopropylethyl, 1-cyclopropylethylamino, 1-methylethylideneamino, 2,2,2-trifluoro-1-methylethyl, 2,4-dichlorobenzyloxy, 2,6-dichlorobenzyloxy, 2-butyl, 2-chlorobenzyloxy, 2-fluorocyclopropyl, 2-hexahydropyranyloxy, 2-methoxyethyl, 2-thienylmethyl, 2-tolyl, 2-trifluoromethylcyclohexyl, 3-(dimethylamino)propyl, 3,5-bis-trifluoromethylcyclohexyl, 3,5-dichlorobenzyloxy, 3-aminopropyl, 3-chlorobenzyloxy, 3-tolyl, 3-trifluoromethylbenzyloxy, 3-trifluoromethylcyclohexyl, 3,5-(bistrifluoromethyl)cyclohexyl, 2-trifluoromethylcyclohexyl, 4-trifluoromethylcyclohexyl, 4-chlorobenzyloxy, 4-fluorobenzyloxy, 4-fluorophenyl, 4-tolyl, 4-trifluoromethylbenzyloxy, 4-trifluoromethylcyclohexyl, allyl, allylamino, allyloxy, benzyloxy, -C(CH₃)₂-CF₃, -C(CH₃)₂-CH₂-COCH₃, -C₂H₅, -CH(CH₂OH)-COOCH₃, -CH(CH₃)-C(CH₃)₃-, -CH(CH₃)-CH(O-CH₃)₂-, -CH(CH₃)-CH=CH₂-, -CH(CH₃)-CH₂-CH(CH₃)₂-, -CH(CH₃)-CH₂-O-CH₃-, -CH(CH₃)-CH₂-OH, -CH(CH₃)-COOCH₃, -CH(CH₃)-COO-t-butyl, -CH₂-C(CH₃)=CH₂, -CH₂-C(CH₃)₃, -CH₂-CF₃, -CH₂-CH(OCH₃)₂, -CH₂-CH₂-CF₃, -CH₂-CH₂-Cl, -CH₂-CH₂-CN, -CH₂-CH₂-N(CH₃)₂, -CH₂-CH₂-N(CH₃)₂, -CH₂-CH₂-NH₂, -CH₂-CHF₂, -CH₂-CN, -CH₂-COOC₂H₅, -CH₂-COOC₂H₅, -CH₂-COOCH₃, -CH₃, cyclohexyl, cyclopentyl, cyclopropyl, cyclopropylmethyl, dimethylamino, isobutoxy, isobutyl, isopropylamino, n-butoxy, n-butyl, n-butylamino, -NH₂, -NH-CH₂-CF₂-CHF₂, -NH-CH₂-CF₃, -NH-CH₂-CH(CH₃)₂, -O-C₂H₅, -O-CH(CH₃)-CH₂-CH₃, -O-CH₃, -OH, O-isopropyl, propargyl, t-butoxy, t-butyl, t-butylamino, or represents a group selected from one of the following groups, 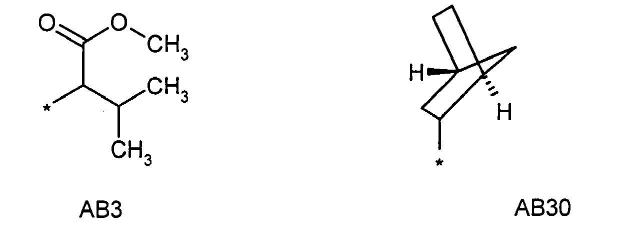 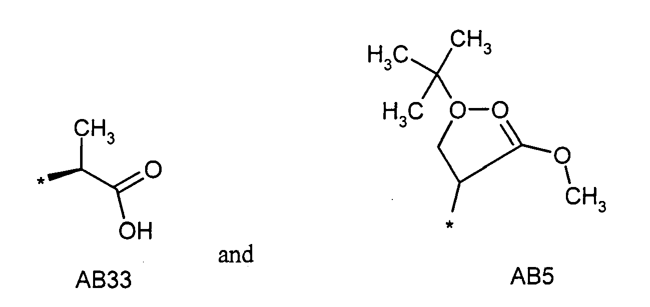 In which * marks in each case the bonding site,
where the aforementioned thiazolyl and pyridyl radicals may be mono- or disubstituted in the case of thiazolyl and mono- to trisubstituted in the case of pyridyl, in each case by identical or different fluorine, chlorine, bromine, methyl, ethyl, n- or isopropyl, n-, iso-, s- or t-butyl, methoxy, ethoxy, n- or isopropoxy, n-, iso-, s- or t-butoxy, methylthio, ethylthio, n- or isopropylthio, difluoromethoxy, trifluoromethoxy, difluorochloromethoxy, trifluoroethoxy, difluoromethylthio, difluorochloromethylthio, dichlorofluoromethylthio, trifluoromethylthio and/or phenyl, and
where the aforementioned phenyl and benzyloxy radicals may be substituted from one to three times in the phenyl moiety by identical or different substituents selected from fluorine, chlorine, bromine, cyano, nitro, amino, hydroxyl, formyl, carboxyl, carbamoyl, thiocarbamoyl, methyl, ethyl, n- or isopropyl, n-, iso-, s- or t-butyl, methoxy, ethoxy, n- or isopropoxy, methylthio, ethylthio, n- or isopropylthio, methylsulphinyl, ethylsulphinyl, methylsulphonyl or ethyl-sulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl, trifluoromethylsulphonyl, methylamino, ethylamino, n- or isopropylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl, ethoximinoethyl, cyclopropyl, cyclobutyl, cyclopentyl or cyclohexyl, or
1,3-propanediyl, methylenedioxy (-O-CH₂-O-), or 1,2-ethylenedioxy (-O-CH₂-CH₂-O-) which is attached in the 2,3-position, where these radicals may be mono- or polysubstituted by identical or different substituents from the group comprising fluorine, chlorine, methyl, ethyl, n-propyl, isopropyl and/or trifluoromethyl,
R² represents hydrogen, methyl, ethyl, n- or isopropyl, n-, iso-, s- or t-butyl, methoxymethyl, 2-methoxyethyl, methylthiomethyl, 2-methylthioethyl, hydroximinomethyl, methoximinomethyl, acetylmethyl, 2-hydroxyiminopropyl, 2-methoxyiminopropyl, allyl, propargyl, 2,2,2-trifluoroethyl, 1-(1,1,1-trifluoromethyl)ethyl, cyclopropylmethyl, cyclobutylmethyl, cyclopentylmethyl or cyclohexylmethyl,
or
R¹ and R² represent, together with the nitrogen atom to which they are attached, 1-pyrrolinyl, 3-pyrrolinyl, pyrrolidinyl, dihydropyridinyl, piperidinyl, pyrazolinyl, pyrazolidinyl, imidazolinyl, imidiazolidinyl, 1,2-diazinanyl, 1,3-diazinanyl, piperazinyl, oxazolinyl, oxazolidinyl, isoxazolyl, isoxazolidinyl, dihydrooxazinyl, morpholinyl, thiazolinyl, thiazolidinyl or thiomorpholinyl, the said heterocycles possibly being substituted by
fluorine, chlorine, bromine, cyano, nitro, amino, hydroxyl, formyl, carboxyl, carbamoyl, thiocarbamoyl, methyl, ethyl, n- or isopropyl, n-, iso-, s- or t-butyl, methoxy, ethoxy, n- or isopropoxy, methylthio, ethylthio, n- or isopropylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl, trifluoromethylsulphonyl, methylamino, ethylamino, n- or isopropylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl, ethoximinoethyl, cyclopropyl, cyclobutyl, cyclopentyl or cyclohexyl,
a fused phenyl ring or
a methanediyl or ethanediyl bridge,
R³ represents phenyl which may be mono- to trisubstituted by identical or different substituents from the group consisting of
fluorine, chlorine, bromine, cyano, nitro, formyl, methyl, ethyl, n- or isopropyl, n-, iso-, s- or t-butyl, allyl, propargyl, methoxy, ethoxy, n-or isopropoxy, methylthio, ethylthio, n- or isopropylthio, methylsulphinyl, ethylsulphinyl, methylsulphonyl, ethylsulphonyl, allyloxy, propargyloxy, trifluoromethyl, trifluoroethyl, difluoromethoxy, trifluoromethoxy, difluorochloromethoxy, trifluoroethoxy, difluoromethylthio, difluorochloromethylthio,trifluoromethylthio, trifluoromethylsulphinyl, trifluoromethylsulphonyl, trichloroethinyloxy, trifluoroethinyloxy, chloroallyloxy, iodopropargyloxy, methylamino, ethylamino, n- or iso-propylamino, dimethylamino, diethylamino, acetyl, propionyl, acetyloxy, methoxycarbonyl, ethoxycarbonyl, hydroximinomethyl, hydroximinoethyl, methoximinomethyl, ethoximinomethyl, methoximinoethyl, ethoximinoethyl, cyclopropyl, cyclobutyl, cyclopentyl and cyclohexyl,
1,3-propanediyl, methylenedioxy (-O-CH₂-O-) or 1,2-ethylenedioxy (-O-CH₂-CH₂-O-) which is attached in the 2,3-position, where these radicals may be mono- or polysubstituted by identical or different substituents from the group consisting of fluorine, chlorine, methyl, ethyl, n-propyl, isopropyl and trifluoromethyl,
R⁴ represents methyl, ethyl, n-propyl, isopropyl, n-, iso-, s- or t-butyl, trifluoromethyl, trifluoroethyl or cyclopropyl,
X represents fluorine or chlorine.

3. Triazolopyrimidines as defined in Claim 1 or 2, in which R¹ and R² together represent a group selected from one of the following groups:
-CH(CF₃)-CH₂-CH₂-CH₂-, -CH(CF₃)-CH₂-CH₂-CH₂-CH₂-,
-CH(CH₃)-CH=CH-CH(CH₃)-, -CH(CH₃)-CH₂-CH₂-O-,
-CH(COOCH₃)-, -CH₂-CH(CH₃)-CH₂-CH(CH₃)-CH₂-,
-CH₂-CH(CH₃)-O-CH(CH₃)-CH₂-, -CH₂-CH(NH₂)-CH₂-CH₂-
CH₂-C-CH₂-CH(OH)-CH₂-CH₂-, -CH₂-CH(OH)-CH₂-CH₂-CH₂-
-CH₂-CH=C(C₂H₅)-CH₂-CH₂-, -CH₂-CH₂-C(CH₃)₂-CH₂-CH₂-
-CH₂-CH₂-CH₂-, -CH₂-CH₂-CH(CF₃)-CH₂-CH₂-
-CH₂-CH₂-CH(CH₃)-CH₂-CH₂-, -CH₂-CH₂-CH(CH₃)-CH₂-CH₂-
-CH₂-CH₂-CH(COCH₃)-CH₂-CH₂-, -CH₂-CH₂-CH(COOCH₃)-CH₂-CH₂-
-CH₂-CH₂-CH(NH-COCH₃)-CH₂-CH₂-, -CH₂-CH₂-CH(OH)-CH₂-CH₂-
-CH₂-CH₂-CH=C(CH₃)-CH₂-, -CH₂-CH₂-CH=CH-CH₂-
-CH₂-CH₂-CH₂-CH(CH₃)-, -CH₂-CH₂-CH₂-CH(CH₃)-CH₂-
-CH₂-CH₂-CH₂-CH₂-, -CH₂-CH₂-CH₂-CH₂-CH(CH₃)-
-CH₂-CH₂-CH₂-CH₂-CH₂-, -CH₂-CH₂-CHBr-CH₂-CH₂-
-CH₂-CH₂-CHF-CH₂-CH₂-, -CH₂-CH₂-N(CH₃)-CH₂-CH₂-
-CH₂-CH₂-O-CH₂-CH₂-, -CH₂-CH₂-S-CH₂-CH₂-
-CH₂-S-CH₂-CH₂-, -NH-CH₂-CH₂-CH₂-CH₂-
-O-_{CH2}-CH₂-CH₂-CH(CH₃)- and -O-CH₂-CH₂-CH₂-CH₂-,
or
represent a group selected from one of the following groups: 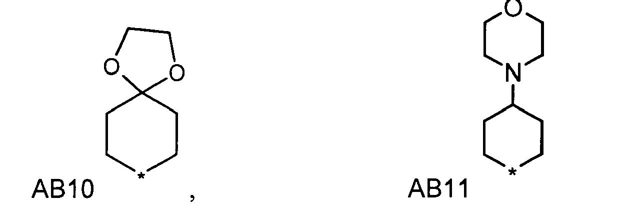 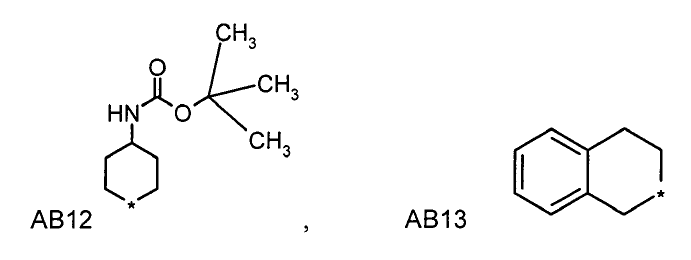 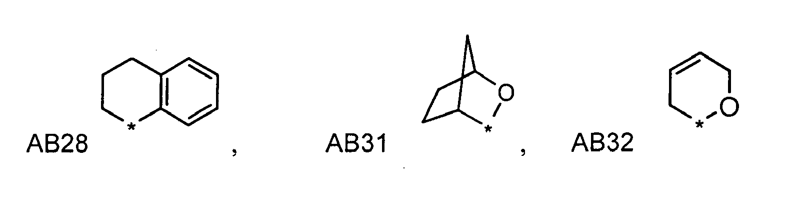 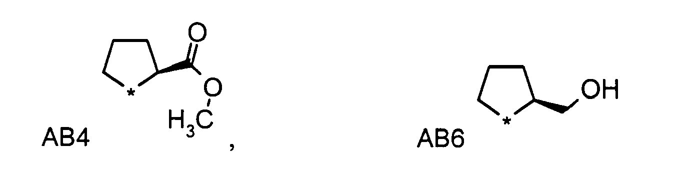 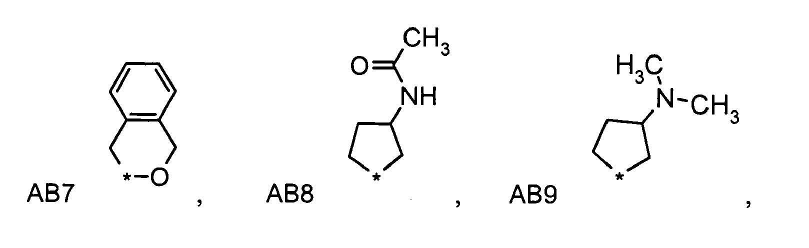 in which * represents the nitrogen atom to which the radicals R¹, R² are attached
R² represents hydrogen, methyl, ethyl, n-, isopropyl, n-, iso-, s- or t-butyl;
R³ represents phenyl which is mono- to trisubstituted by identical or different fluorine and/or chlorine substituents in positions 2, 4 and 6; and
X represents chlorine.

4. Triazolopyrimidines as defined in any of Claims 1 to 3, in which R³ represents 2,4-disubstituted, 2,6-disubstituted or 2,4,6-trisubstituted phenyl.

5. Triazolopyrimidines as defined in any of Claims 1 to 4, in which R⁴ represents cyclopropyl.

6. Process for preparing triazolopyrimidines of the formula (I) according to any of Claims 1 to 5, **characterized in that**
a) dihalogeno-triazolopyrimidines of the formula 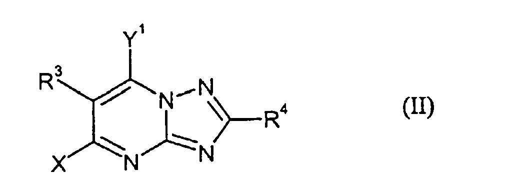 in which
R³, R⁴ and X have the meanings given above and
Y¹ represents halogen
are reacted with amines of the formula 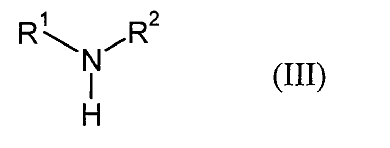 in which
R¹ and R² have the meanings given above,
if appropriate in the presence of a diluent and if appropriate in the presence of an acid acceptor,
or
b) triazolopyrimidines of the formula 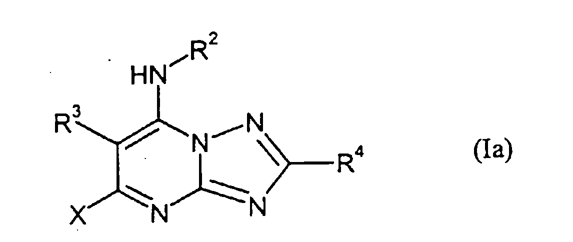 in which
R², R³, R⁴ and X have the meanings given above
are reacted with sulphenyl halides of the formula
Y²-S-R⁵ (IV)
in which
R⁵ has the meanings given above and
Y² represents halogen
if appropriate in the presence of a diluent and if appropriate in the presence of an acid acceptor,
and, if appropriate, an acid is added onto the resulting compounds of the formula (I) in which
R¹ represents amino.

7. Compositions for controlling unwanted microorganisms, **characterized in that** they comprise at least one triazolopyrimidine of the formula (I) according to any one of Claims 1 to 5 or an acid addition salt thereof, in addition to extenders and/or surfactants.

8. Use of triazolopyrimidines of the formula (I) according to any one of Claims 1 to 5 or of acid addition salts thereof for controlling unwanted microorganisms.

9. Method for controlling unwanted microorganisms, **characterized in that** triazolopyrimidines of the formula (I) according to any one of Claims 1 to 5 or acid addition salts thereof are applied to the unwanted microorganisms and/or their habitat.

10. Process for preparing compositions for controlling unwanted microorganisms, **characterized in that** triazolopyrimidines of the formula (I) according to any one of Claims 1 to 5 or acid addition salts thereof are mixed with extenders and/or surfactants.

11. Dihalogeno-triazolopyrimidines of the formula 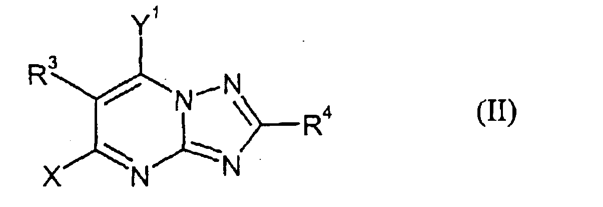 in which
R³, R⁴ and X have the meanings given above and
Y¹ represents halogen.

12. Process for preparing dihalogeno-triazolopyrimidines of the formula (II) according to Claim 11, **characterized in that**
c) dihydroxy-triazolopyrimidines of the formula 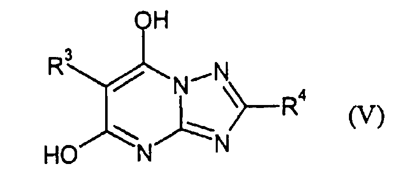 in which R³ and R⁴ have the meanings given above,
are reacted with halogenating agents, if appropriate in the presence of a diluent.

13. Dihydroxy-triazolopyrimidines of the formula 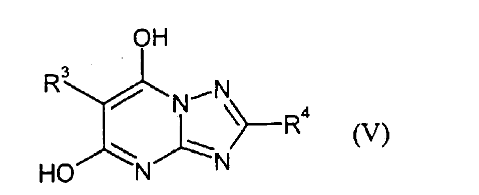 in which R³ and R⁴ have the meanings given above.

14. Process for preparing dihydroxy-triazolopyrimidines of the formula (V) according to Claim 13, **characterized in that**
d) arylmalonic esters of the formula 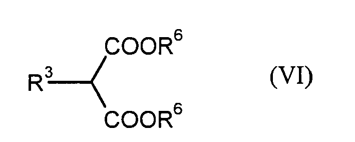 in which
R³ has the meanings given above and
R⁶ represents alkyl having 1 to 4 carbon atoms
are reacted with aminotriazoles of the formula 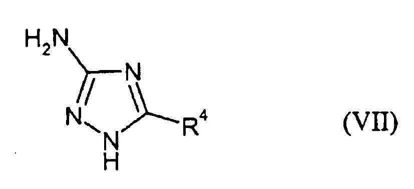 in which
R⁴ has the meanings given above,
if appropriate in the presence of a diluent and if appropriate in the presence of an acid binder.

15. Amines of the formula (IIIa), 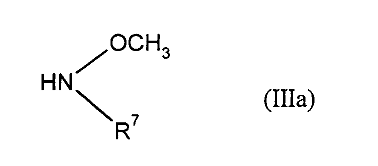 in which
R⁷ represents isobutyl, 2-methoxyethyl or represents

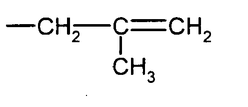

16. Process for preparing amines of the formula (IIIa) according to Claim 15,
**characterized in that**
e) in a first stage ethyl N-methoxycarbamate of the formula (VIII) 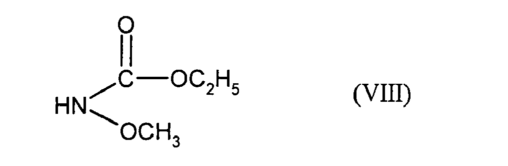 is reacted with halogen compounds of the formula (IX),
R⁷-X¹ (IX)
in which
R⁷ has the meanings given above and
X¹ represents bromine or iodine,
in the presence of a base and in the presence of a diluent and the resulting carbamates of the formula (X), 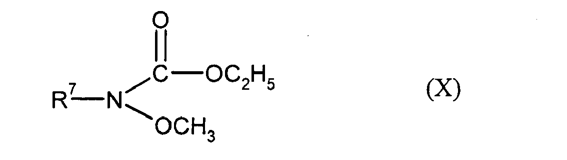 in which
R⁷ has the meanings given above,
in a second stage are reacted with potassium hydroxide in the presence of ethanol and water.

17. Amines of the formula (IIIb), 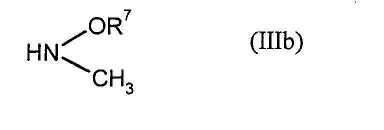 in which R⁷ represents isobutyl, 2-methoxyethyl or represents

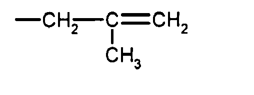

18. Process for preparing amines of the formula (IIIb) according to Claim 17,
**characterized in that**
f) in a first stage ethyl N-hydroxy-N-methylcarbamate of the formula (XI) 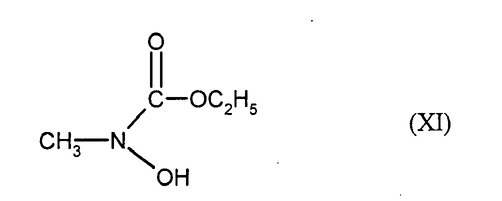 is reacted with halogen compounds of the formula (IX),
R⁷-X¹ (IX)
in which
R⁷ and X¹ have the meanings given above,
in the presence of a base and in the presence of a diluent and the resulting carbamates of the formula (XII), 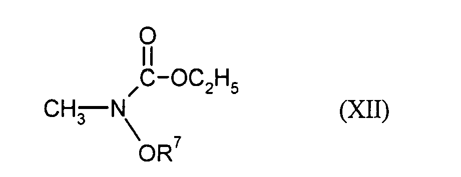 in which
R⁷ has the meanings given above,
in a second stage are reacted with potassium hydroxide in the presence of ethanol and water.

19. Trifluoroisopropylamines of the formula (IIIc), 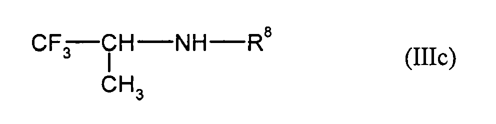 in which
R⁸ represents methyl, ethyl or propyl.

20. Process for preparing trifluoroisopropylamines of the formula (IIIc) according to Claim 19, **characterized in that**
g) in a first stage ethyl N-trifluoroisopropylcarbamate of the formula (XIII) 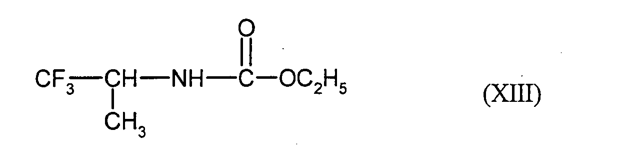 is reacted with halogen compounds of the formula (XIV),
R⁸-X¹ (XIV)
in which
R⁸ and X¹ have the meanings given above, in the presence of a base and in the presence of a diluent and the resulting carbamates of the formula (XV), 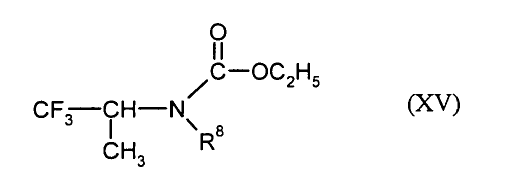 in which
R⁸ has the meanings given above,
in a second stage are reacted with potassium hydroxide in the presence of ethanol and water.

21. The compond 3-trifluoromethyl-3-aminopropene of the formula (III-4)

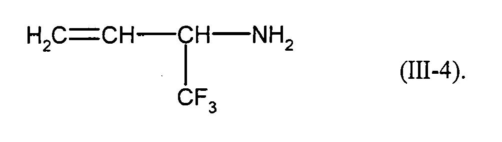

22. Process for preparing 3-trifluoromethyl-3-aminopropene of the formula (III-4) according to Claim 21, **characterized in that**
h) the carbamate of the formula (XVI) 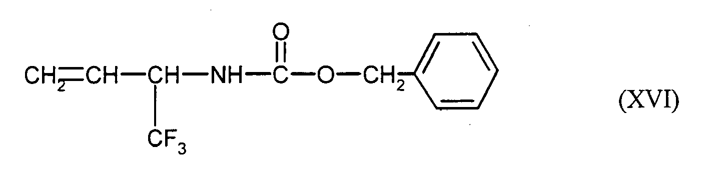 is reacted with aqueous hydrochloric acid.

23. Carbamates of the formula (X), 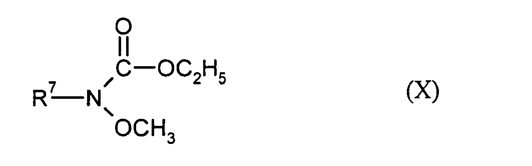 in which
R⁷ represents isobutyl, 2-methoxyethyl or represents

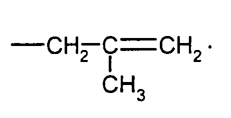

24. Carbamates of the formula (XII), 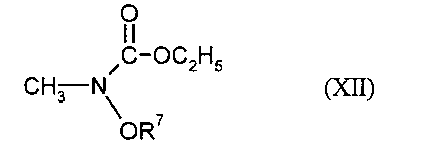 in which
R⁷ represents isobutyl, 2-methoxyethyl or represents

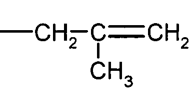

25. Carbamates of the formula (XIII), 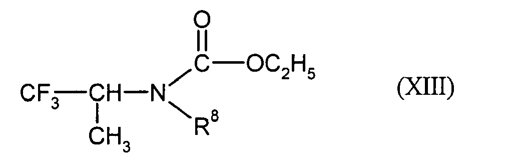 in which
R⁸ represents methyl, ethyl or propyl.

## Revendications

1. Triazolopyrimidines de formule suivante 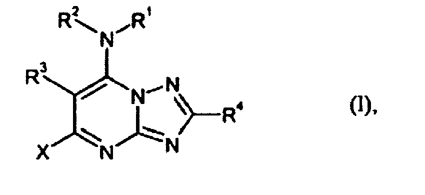 dans laquelle
R¹ est un groupe amino ou hydroxy, un reste alkyle ayant 1 à 6 atomes de carbone, éventuellement substitué par un radical halogéno, cyano, hydroxy, amino, phényle, hétérocyclyle, alkoxy ayant 1 à 4 atomes de carbone, alkoxycarbonyle ayant 1 à 4 atomes de carbone, alkylamino ayant 1 à 4 atomes de carbone, dialkylamino ayant 2 à 8 atomes de carbone, cycloalkyle ayant 3 à 6 atomes de carbone, halogénocycloalkyle ayant 3 à 6 atomes de carbone et 1 à 5 atomes d'halogènes, alkylthio ayant 1 à 4 atomes de carbone, oxo, hydroxyimino et/ou alkoximino ayant 1 à 4 atomes de carbone,
un reste alcényle ayant 2 à 6 atomes de carbone, éventuellement substitué par un radical halogéno, cycloalkyle, cyano, phényle et/ou hétérocyclyle,
un reste alcynyle ayant 2 à 6 atomes de carbone, éventuellement substitué par un radical halogéno, cycloalkyle, cyano, phényle et/ou hétérocyclyle,
un reste cycloalkyle ayant 3 à 7 atomes de carbone, éventuellement substitué par un radical halogéno, cycloalkyle, cyano, halogénalkyle ayant 1 ou 2 atomes de carbone et 1 à 5 atomes d'halogènes, phényle et/ou hétérocyclyle,
un reste alkoxy ayant 1 à 7 atomes de carbone, éventuellement substitué par un radical halogéno, cycloalkyle, cyano, phényle et/ou hétérocyclyle,
un reste alcényloxy ayant 2 à 6 atomes de carbone, éventuellement substitué par un radical halogéno, cycloalkyle, cyano, phényle et/ou hétérocyclyle,
un reste alcynyloxy ayant 2 à 6 atomes de carbone, éventuellement substitué par un radical halogéno, cycloalkyle, cyano, phényle et/ou hétérocyclyle,
un reste cycloalkyloxy ayant 3 à 7 atomes de carbone, éventuellement substitué par un radical halogéno, cycloalkyle, cyano, phényle et/ou hétérocyclyle,
un reste alkylamino ayant 1 à 7 atomes de carbone, éventuellement substitué par un radical halogéno, cycloalkyle, cyano, phényle et/ou hétérocyclyle,
un reste dialkylamino ayant 1 à 7 atomes de carbone dans chacun des restes alkyle, éventuellement substitué par un radical halogéno, cycloalkyle, cyano, phényle et/ou hétérocyclyle,
un reste alcénylamino ayant 2 à 6 atomes de carbone, éventuellement substitué par un radical halogéno, cycloalkyle, cyano, phényle et/ou hétérocyclyle,
un reste alcynylamino ayant 2 à 6 atomes de carbone, éventuellement substitué par un radical halogéno, cycloalkyle, cyano, phényle et/ou hétérocyclyle,
un reste cycloalkylamino ayant 3 à 7 atomes de carbone, éventuellement substitué par un radical halogéno, cycloalkyle, cyano, phényle et/ou hétérocyclyle,
un reste N-alkyl-N-alcénylamino ayant 1 à 6 atomes de carbone dans la partie alkyle et 2 à 6 atomes de carbone dans la partie alcényle, éventuellement substitué par un radical halogéno, cyano, phényle et/ou hétérocyclyle,
un reste N-cycloalkyl-N-alkylamino ayant 3 à 7 atomes de carbone dans la partie cycloalkyle et 1 à 7 atomes de carbone dans la partie alkyle, éventuellement substitué par un radical halogéno, cycloalkyle, cyano, phényle et/ou hétérocyclyle,
un reste alkylidène-amino ayant 2 à 6 atomes de carbone, éventuellement substitué par un radical halogéno, cycloalkyle, cyano, phényle et/ou hétérocyclyle,
un reste phényle, éventuellement substitué par un radical halogéno, cycloalkyle, cyano, phényle et/ou hétérocyclyle,
un reste hétérocyclyle pentagonal ou hexagonal, éventuellement substitué par un radical halogéno, alkyle, cycloalkyle, cyano, phényle et/ou hétérocyclyle, un reste hétérocyclyloxy pentagonal ou hexagonal, éventuellement substitué par un radical halogéno, alkyle, cycloalkyle, cyano, phényle et/ou hétérocyclyle, un reste de formule -SR⁵, dans laquelle
R⁵ représente un reste alkyle ayant 1 à 6 atomes de carbone, éventuellement substitué par un radical halogéno, cycloalkyle, cyano, phényle et/ou hétérocyclyle,
un reste alcényle ayant 2 à 6 atomes de carbone, éventuellement substitué par un radical halogéno, cycloalkyle, cyano, phényle et/ou hétérocyclyle,
un reste alcynyle ayant 2 à 6 atomes de carbone, éventuellement substitué par un radical halogéno, cycloalkyle, cyano, phényle et/ou hétérocyclyle, ou
un reste cycloalkyle ayant 3 à 7 atomes de carbone, éventuellement substitué par un radical halogéno, cycloalkyle, cyano, phényle et/ou
hétérocyclyle,
et
les restes hétérocyclyle mentionnés ci-dessus peuvent être substitués une à trois fois identiques ou différentes par un radical halogéno, hydroxy, phényle, 1,2-dioxyéthylène, alkyle ayant 1 à 4 atomes de carbone, halogénalkyle ayant 1 ou 2 atomes de carbone et 1 à 5 atomes d'halogènes, alkoxy ayant 1 à 4 atomes de carbone, alkylthio ayant 1 à 4 atomes de carbone, halogénalkoxy ayant 1 ou 2 atomes de carbone et 1 à 5 atomes d'halogènes, halogénalkyltio ayant 1 ou 2 atomes de carbone et 1 à 5 atomes d'halogènes, et
les restes hétérocyclyle mentionnés ci-dessus sont saturés ou partiellement insaturés,
les restes phényle mentionnés ci-dessus peuvent être substitués une à trois fois identiques ou différentes par
un halogène, un groupe cyano, nitro, amino, hydroxy, formyle, carboxy, carbamoyle, thiocarbamoyle,
un reste alkyle, alkoxy, alkylthio, alkylsulfinyle ou alkylsulfonyle ayant chacun 1 à 6 atomes de carbone et chacun étant linéaire ou ramifié,
un reste alcényle ou alcényloxy ayant chacun 2 à 6 atomes de carbone et chacun étant linéaire ou ramifié,
un reste halogénalkyle, halogénalkoxy, halogénalkylthio, halogénalkylsulfinyle ou halogénalkylsulfonyle ayant chacun 1 à 6 atomes de carbone et 1 à 13 atomes d'halogènes identiques ou différents et chacun étant linéaire ou ramifié,
un reste halogénalcényle ou halogénalcényloxy ayant chacun 2 à 6 atomes de carbone et 1 à 13 atomes d'halogènes identiques ou différents et chacun étant linéaire ou ramifié,
un reste alkylamino, dialkylamino, alkylcarbonyle, alkylcarbonyloxy, alkoxycarbonyle, alkylsulfonyloxy, hydroximinoalkyle ou alkoximinoalkyle ayant chacun 1 à 6 atomes de carbone dans les parties alkyle individuelles et chacun étant linéaire ou ramifié, un reste cycloalkyle ayant 3 à 6 atomes de carbone,
un reste, lié en positions 2,3, 1,3-propanediyle, 1,4-butanediyle, méthylènedioxy(-O-CH₂-O-), 1,2-éthylènedioxy(-O-CH₂-CH₂-O-), ces restes pouvant être substitués une ou plusieurs fois identiques ou différentes par un radical halogéno, alkyle ayant 1 à 4 atomes de carbone et/ou halogénalkyle ayant 1 à 4 atomes de carbone et 1 à 9 atomes d'halogènes identiques ou différents,
R² représente l'hydrogène,
un reste alkyle ayant 1 à 4 atomes de carbone, éventuellement substitué par un radical halogéno, cycloalkyle ayant 3 à 6 atomes de carbone, alkoxy ayant 1 à 4 atomes de carbone, alkylthio ayant 1 à 4 atomes de carbone, oxo, hydroximino et/ou alkoximino ayant 1 à 4 atomes de carbone,
un reste alcényle ayant 2 à 4 atomes de carbone, éventuellement substitué par un radical halogéno et/ou cycloalkyle ayant 3 à 6 atomes de carbone,
un reste alcynyle ayant 2 à 4 atomes de carbone, éventuellement substitué par un radical halogéno et/ou cycloalkyle ayant 3 à 6 atomes de carbone, ou bien
un reste cycloalkyle ayant 3 à 6 atomes de carbone, éventuellement substitué par un radical halogéno et/ou cycloalkyle ayant 3 à 6 atomes de carbone ; ou bien
R¹ et R² forment, conjointement avec l'atome d'azote auquel ils sont liés, un noyau hétérocyclique trigonal à hexagonal qui est saturé ou partiellement saturé, qui peut contenir, en plus de l'atome d'azote déjà mentionné, encore un autre hétéroatome de la série azote, oxygène et soufre et qui peut être substitué une à trois fois identiques ou différentes par un halogène, un groupe hydroxy, cyano, morpholinyle, amino, un noyau phényle condensé, un pont méthylène ou éthylène, un reste alkyle ayant 1 à 4 atomes de carbone,
un reste halogénalkyle ayant 1 à 4 atomes de carbone et 1 à 9 atomes d'halogènes identiques ou différents,
un reste alkylcarbonylamino ayant 1 à 4 atomes de carbone, dialkylamino ayant 2 à 8 atomes de carbone,
un reste alkoxycarbonylamino ayant 1 à 4 atomes de carbone,
un reste di(alkoxycarbonyl)amino ayant 2 à 8 atomes de carbone,
un reste hydroxyalkyle ayant 1 à 4 atomes de carbone,
un reste alkoxycarbonyle ayant 1 à 4 atomes de carbone, et/ou
un reste alkylcarbonyle ayant 1 à 4 atomes de carbone ;
R³ est un reste phényle qui peut être substitué 1 à 4 fois identiques ou différentes par
un halogène, un groupe cyano, nitro, amino, hydroxy, formyle, carboxy, carbamoyle, thiocarbamoyle,
un reste alkyle, alkoxy, alkylthio, alkylsulfinyle ou alkylsulfonyle ayant chacun 1 à 6 atomes de carbone et chacun étant linéaire ou ramifié,
un reste alcényle ou alcényloxy ayant chacun 2 à 6 atomes de carbone et chacun étant linéaire ou ramifié,
un reste halogénalkyle, halogénalkoxy, halogénalkylthio, halogénalkylsulfinyle ou halogénalkylsulfonyle ayant chacun 1 à 6 atomes de carbone et 1 à 13 atomes d'halogènes identiques ou différents et chacun étant linéaire ou ramifié, un reste halogénalcényle ou halogénalcényloxy ayant chacun 2 à 6 atomes de carbone et 1 à 11 atomes d'halogènes identiques ou différents et chacun étant linéaire ou ramifié,
un reste alkylamino, dialkylamino, alkylcarbonyle, alkylcarbonyloxy, alkoxycarbonyle, alkylsulfonyloxy, hydroximinoalkyle ou alkoximinoalkyle ayant chacun 1 à 6 atomes de carbone dans les parties alkyle individuelles et chacun étant linéaire ou ramifié,
un reste cycloalkyle ayant 3 à 6 atomes de carbone,
un reste, lié en positions 2,3, 1,3-propanediyle, 1,4-butanediyle, méthylènedioxy(-O-CH₂-O-), 1,2-éthylènedioxy(-O-CH₂-CH₂-O-), ces restes pouvant être substitués une ou plusieurs fois identiques
ou différentes par un radical halogéno, alkyle ayant 1 à 4 atomes de carbone et/ou halogénalkyle ayant 1 à 4 atomes de carbone et 1 à 9 atomes d'halogènes identiques ou différents ;
R⁴ représente un reste alkyle ayant 1 à 4 atomes de carbone éventuellement substitué par 1 à 9 atomes d'halogènes ou un reste cycloalkyle ayant 3 à 6 atomes de carbone éventuellement substitué par 1 à 9 atomes d'halogènes ; et
X représente le fluor, le chlore ou le brome,
ainsi que des sels d'addition d'acides de ceux des composés de formule (I), dans lesquels R¹ est un groupe amino.

2. Triazolopyrimidines telles que définies dans la revendication 1, dans lesquelles
R¹ est un groupe hydroxy, amino, un reste méthyle, éthyle, n-propyle, isopropyle, n-butyle, isobutyle, sec.-butyle, tertiobutyle, méthoxyméthyle, 2-méthoxyéthyle, méthylthiométhyle, 2-méthylthioéthyle, hydroximinométhyle, méthoximinométhyle, acétylméthyle, 2-hydroximinopropyle, 2-méthoximinopropyle, allyle, 2-méthyl-prop2-ényle, propargyle, 2,2,2-trifluoréthyle, 1-(trifluorométhyl)-éthyle, 3,3,3-trifluoropropyle, cyclopentyle, cyclohexyle,
un reste cyclopropyloxy, cyclobutyloxy, cyclopentyloxy, cyclohexyloxy, difluorométhoxy, trifluorométhoxy, difluorochlorométhoxy, trifluoréthoxy,
un reste méthylamino, éthylamino, n-propylamino, isopropylamino, n-butylamino, isobutylamino, sec.-butylamino, tertiobutylamino, diméthylamino, diéthylamino, trifluoréthylamino, cyclohexylméthylamino, 2-cyanéthylamino, allylamino, 1-cyclopropyléthylamino, cyclopropylamino, cyclobutylamino, cyclopentylamino, cyclohexylamino, 1-méthyléthylidène-amino, phényle, benzyloxy, pipéridinyle, morpholinyle, pyridylméthoxy, thiazolylméthoxy,
ou bien
un groupe -S-R⁵, dans lequel
R⁵ est un reste méthyle, éthyle, n-propyle, isopropyle, difluorométhyle, difluorochlorométhyle, dichlorofluorométhyle ou trifluorométhyle,
ou bien
R¹ est un reste (2,2-dichlorocyclopropyl)méthyle, (2-furyl)méthyle, (2-tétrahydrofuryl)méthyle, (2-tétrahydropyrannyl)méthyle, 1,2-diméthylpropyle, 1,3-dioxolanne-2-ylméthyle, 1-cyclopropyléthyle, 1-cyclopropyléthylamino, 1-méthyléthylidène-amino, 2,2,2-trifluoro-1-méthyléthyle, 2,4-dichlorobenzyloxy, 2,6-dichlorobenzyloxy, 2-butyle, 2-chlorobenzyloxy, 2-fluorocyclopropyle, 2-hexahydropyrannyloxy, 2-méthoxyéthyle, 2-thiénylméthyle, 2-tolyle, 2-trifluorométhylcyclohexyle, 3-(diméthylamino)-propyle, 3,5-bis-trifluorométhylcyclohexyle, 3,5-dichlorobenzyloxy, 3-aminopropyle, 3-chlorobenzyloxy, 3-tolyle, 3-trifluorométhylbenzyloxy, 3-trifluorométhylcyclohexyle, 3,5-(bis-trifluorométhyl)-cyclohexyle, 2-trifluorométhyl-cyclohexyle, 4-trifluorométhyl-cyclohexyle, 4-chlorobenzyloxy, 4-fluorobenzyloxy, 4-fluorophényle, 4-tolyle, 4-trifluorométhylbenzyloxy, 4-trifluorométhylcyclohexyle, allyle, allylamino, allyloxy, benzyloxy, -C(CH₃)₂-CF₃, -C(CH₃)₂-CH₂-COCH₃, -C₂H₅, -CH(CH₂OH)-COOCH₃, -CH(CH₃)-C(CH₃)₃-, -CH(CH₃)-CH(O-CH₃)₂-, -CH(CH₃)-CH=CH₂-, -CH(CH₃)-CH₂-CH(CH₃)₂-, -CH(CH₃)-CH₂-O-, CH₃-, -CH(CH₃)-CH₂-OH, -CH(CH₃)-COOCH₃, -CH(CH₃)-COO-tertiobutyle, -CH₂-C(CH₃)=CH₂, -CH₂-C(CH₃)₃, -CH₂-CF₃, -CH₂-CH(OCH₃)₂, -CH₂-CH₂-CF₃, -CH₂-CH₂-Cl, -CH₂-CH₂-CN, -CH₂-CH₂-N(CH₃)₂, -CH₂-CH₂-N(CH₃)₂, -CH₂-CH₂-NH₂, -CH₂-CHF₂, -CH₂-CN, CH₂-COOC₂H₅, -CH₂-COOC₂H₅, -CH₂-COOCH₃, -CH₃, cyclohexyle, cyclopentyle, cyclopropyle, cyclopropylméthyle, diméthylamino, isobutoxy, isobutyle, isopropylamino, n-butoxy, n-butyle, n-butylamino, -NH₂, -NH-CF₂-CF₂-CHF₂, -NH-CH₂-CF₃, -NH-CH₂-CH(CH₃)₂, -O-C₂H₅, -O-CH(CH₃)-CH₂-CH₃, -O-CH₃, -OH, O-isopropyle, propargyle, tertiobutoxy, tertiobutyle, tertiobutylamino ou un groupement choisi parmi les groupements suivants 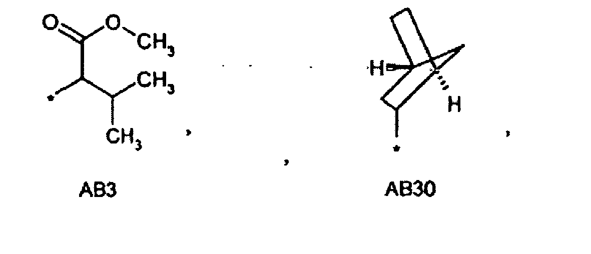 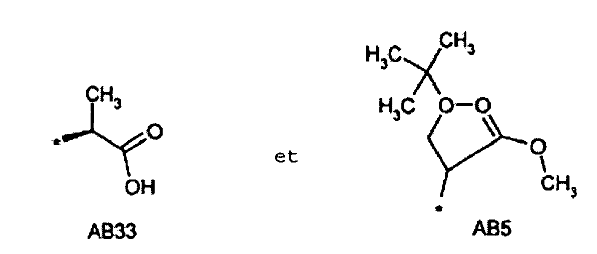 où * marque dans chaque cas la position de liaison,
les restes thiazolyle et pyridyle mentionnés ci-dessus pouvant être substitués une ou deux fois dans le cas de thiazolyle et une à trois fois dans le cas de pyridyle, dans chaque cas de façon identique ou différente par un radical fluoro, chloro, bromo, méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthylthio, éthylthio, n-propylthio, isopropylthio, difluorométhoxy, trifluorométhoxy, difluorochlorométhoxy, trifluoréthoxy, difluorométhylthio, difluorochlorométhylthio, dichlorofluorométhylthio, trifluorométhylthio, et/ou phényle, et
les restes phényle et benzyloxy mentionnés ci-dessus pouvant être substitués dans la partie phényle une à trois fois identiques ou différentes par
un radical fluoro, chloro, bromo, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle, éthoximinoéthyle, cyclopropyle, cyclobutyle, cyclopentyle ou cyclohexyle, ou bien
un reste, lié en positions 2,3, 1,3-propanediyle, méthylènedioxy(-O-CH₂-O-) ou 1,2-éthylènedioxy(-O-CH₂-CH₂-O-), ces restes pouvant être substitués une ou plusieurs fois identiques ou différentes par un radical fluoro, chloro, méthyle, éthyle, n-propyle, isopropyle et/ou trifluorométhyle ;
R² représente l'hydrogène, un reste méthyle, éthyle, n-propyle, isopropyle, n-butyle, isobutyle, sec.-butyle, tertiobutyle, méthoxyméthyle, 2-méthoxyéthyle, méthylthiométhyle, 2-méthylthioéthyle, hydroximinométhyle, méthoximinométhyle, acétylméthyle, 2-hydroxyiminopropyle, 2-méthoxyimino-propyle, allyle, propargyle, 2,2,2-trifluoréthyle, 1-(1,1,1-trifluorométhyl)éthyle, cyclopropylméthyle, cyclobutylméthyle, cyclopentylméthyle
ou cyclohexylméthyle ;
ou bien
R¹ et R² forment, conjointement avec l'atome d'azote auquel ils sont liés, un hétérocycle 1-pyrrolinyle, 3-pyrrolinyle, pyrrolidinyle, dihydropyridinyle, pipéridinyle, pyrazolinyle, pyrazolidinyle, imidazolinyle, imidiazolidinyle, 1,2-diazinane-yle, 1,3-diazinaneyle, pipérazinyle, oxazolinyle, oxazolidinyle, isoxazolyle, isoxazolidinyle, dihydro-oxazinyle, morpholinyle, thiazolinyle, thiazolidinyle ou thiomorpholinyle, les hétérocycles mentionnés pouvant être substitués par
un radical fluoro, chloro, bromo, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhythio, trifluorométhylsulfinyle, trifluorométhylsulfonyle, méthylamino, éthylamino, n-propylamino, isopropylamino, diméthylamino, diéthylamino, acétyle, propionyle, acétyloxa, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle, éthoximinoéthyle, cyclopropyle, cyclobutyle, cyclopentyle ou cyclohexyle, un noyau phényle condensé ou un pont méthanediyle ou éthanediyle ;
R³ est un reste phényle qui peut être substitué une à trois fois identiques ou différentes par
un radical fluoro, chloro, bromo, cyano, nitro, formyle, méthyle, éthyle, n-propyle, isopropyle, n-butyle, isobutyle, sec.-butyle, tertiobutyle, allyle, propargyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, allyloxy, propargyloxy, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, trichloréthynyloxy, trifluoréthynyloxy, chlorallyloxy, iodopropargyloxy, méthylamino, éthylamino, n-propylamino, isopropylamino, diméthylamino, diéthylamino, acétyle, propionyle, acétyloxy, méthoxycarbonyle, éthoxycarbonyle, hydroximinométhyle, hydroximinoéthyle, méthoximinométhyle, éthoximinométhyle, méthoximinoéthyle, éthoximinoéthyle, cyclopropyle, cyclobutyle, cyclopentyle
ou cyclohexyle,
un reste, lié en positions 2,3, 1,3-propanediyle, méthylènedioxy(-O-CH₂-O-) ou 1,2-éthylènedioxy(-O-CH₂-CH₂-O-), ces restes pouvant être substitués une ou plusieurs fois identiques ou différentes par un radical fluoro, chloro, méthyle, éthyle, n-propyle, isopropyle et/ou trifluorométhyle ;
R⁴ représente un reste méthyle, éthyle, n-propyle, isopropyle, n-butyle, isobutyle, sec.-butyle, tertiobutyle, trifluorométhyle, trifluoréthyle ou cyclopropyle ; et
X représente le fluor ou le chlore.

3. Triazolopyrimidines telles que définies dans la revendication 1 ou 2, dans lesquelles
R¹ et R² forment ensemble un groupement choisi parmi les groupements suivants -CH(CF₃)-CH₂-CH₂-CH₂-, CH(CF₃)-CH₂-CH₂-CH₂-CH₂, -CH(CH₃)-CH=CH-CH(CH₃)-, -CH(CH₃)-CH₂-CH₂-O-, -CH(COOCH₃)-, -CH₂-CH(CH₃)-CH₂-CH(CH₃)-CH₂-, -CH₂-CH(CH₃)-O-CH(CH₃)-CH₂-, -CH₂-CH(NH₂)-CH₂-CH₂-CH₂-C-CH₂-CH(OH)-CH₂-CH₂, -CH₂-CH(OH)CH₂-CH₂-CH₂CH₂-CH=C(C₂H₅) -CH₂-CH₂-, -CH₂-CH₂-C(CH₃)₂-CH₂-CH₂-CH₂-CH₂-CH₂-, -CH₂-CH₂-CH(CF₃)-CH₂-CH₂-CH₂-CH₂-CH(CH₃)-CH₂-CH₂-, -CH₂-CH₂-CH(CH₃) -CH₂-CH₂-CH₂-CH₂-CH(COCH₃) -CH₂-CH₂, -CH₂-CH₂-CH(COOCH₃) -CH₂-CH₂-CH₂-CH₂-CH(NH-COCH₃) -CH₂-CH₂-, -CH₂-CH₂-CH(OH)-CH₂-CH₂-CH₂-CH₂-CH=C(CH₃)-CH₂-, -CH₂-CH₂-CH=CH-CH₂-CH₂-CH₂-CH₂-CH(CH₃)-, -CH₂-CH₂-CH₂-CH(CH₃)-CH₂-CH₂-CH₂-CH₂-CH₂-, -CH₂-CH₂-CH₂-CH₂-CH(CH₃)-CH₂-CH₂-CH₂-CH₂-CH₂-, -CH₂-CH₂-CHBr-CH₂-CH₂-CH₂-CH₂-CHF-CH₂-CH₂-, -CH₂-CH₂-N(CH₃)-CH₂-CH₂-CH₂-CH₂-O-CH₂-CH₂-, CH₂-CH₂-S-CH₂-CH₂-CH₂-S-CH₂-CH₂-, -NH-CH₂-CH₂-CH₂-CH₂-O-CH₂-CH₂-CH₂-CH(CH₃)- et -O-CH₂-CH₂-CH₂-CH₂-, ou bien
un groupement choisi parmi les groupements suivants 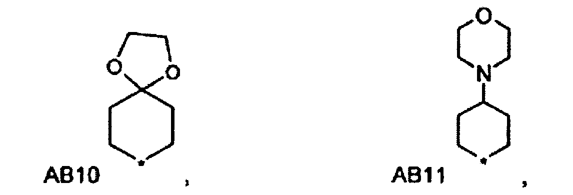 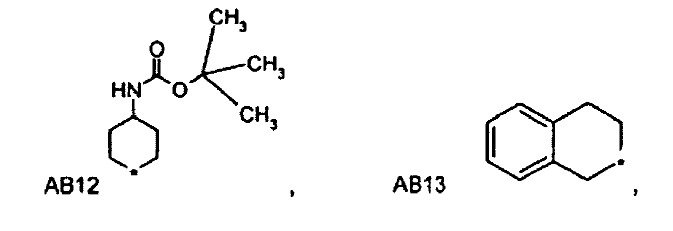 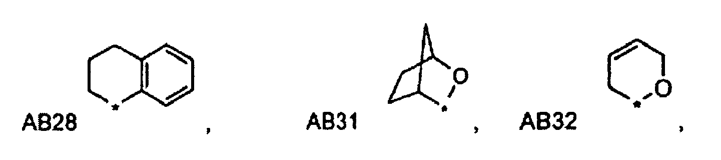 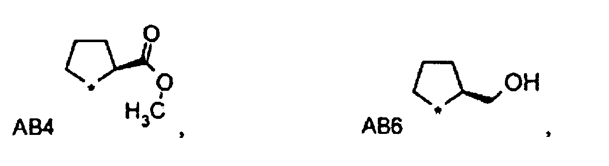 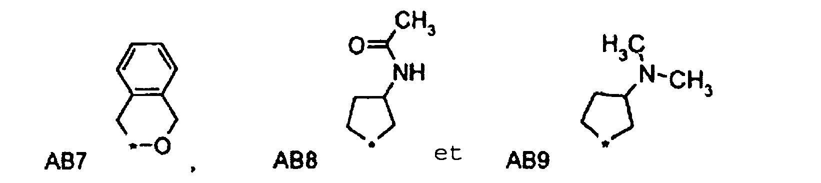 dans lesquels * indique l'atome d'azote auquel les restes R¹, R² sont liés ;
R² représente l'hydrogène, un reste méthyle, éthyle, n-propyle, isopropyle, n-butyle, isobutyle, sec.-butyle ou tertiobutyle,
R³ représente un reste phényle, qui est substitué une à trois fois identiques ou différentes par du fluor et/ou du chlore dans les positions 2, 4 et 6 ; et
X représente le chlore.

4. Triazolopyrimidines telles que définies dans l'une quelconque des revendications 1 à 3, dans lesquelles
R³ représente un reste phényle disubstitué en positions 2,4, disubstitué en positions 2,6 ou trisubstitué en positions 2,4,6.

5. Triazolopyrimidines telles que définies dans l'une quelconque des revendications 1 à 4, dans lesquelles R⁴ est un reste cyclopropyle.

6. Procédé de production de triazolopyrimidines de formule (I) suivant l'une quelconque des revendications 1 à 5, **caractérisé en ce que** :
(a) on fait réagir des dihalogéno-triazolopyrimidines de formule 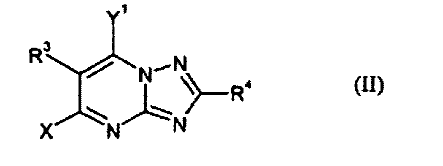 dans laquelle
R³, R⁴ et X ont les définitions indiquées ci-dessus et
Y¹ représente un halogène,
avec des amines de formule 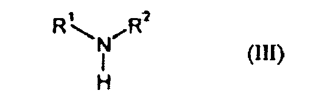 dans laquelle
R¹ et R² ont les définitions indiquées ci-dessus,
éventuellement en présence d'un diluant et, le cas échéant, en présence d'un accepteur d'acide,
ou bien
(b) on fait réagir des triazolopyrimidines de formule 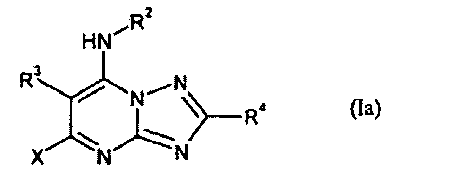 dans laquelle
R², R³, R⁴ et X ont les définitions indiquées ci-dessus,
avec des halogénures d'acides sulféniques de formule
Y²-S-R⁵ (IV),
dans laquelle
R⁵ a les définitions indiquées ci-dessus et
Y² représente un halogène,
éventuellement en présence d'un diluant et, le cas échéant, en présence d'un accepteur d'acide,
et on additionne éventuellement un acide sur les composés de formule (I) ainsi obtenus dans lesquels R¹ est un groupe amino.

7. Compositions destinées à combattre des micro-organismes indésirables, **caractérisées par** une teneur en au moins une triazolopyrimidine de formule (I) suivant l'une quelconque des revendications 1 à 5 ou d'un sel d'addition d'acide de cette triazolopyrimidine, à côté de diluants et/ou d'agents tensioactifs.

8. Utilisation de triazolopyrimidines de formule (I) suivant l'une quelconque des revendications 1 à 5 ou de leurs sels d'addition d'acides pour combattre des micro-organismes indésirables.

9. Procédé de lutte contre des micro-organismes indésirables, **caractérisé en ce qu'**on épand des triazolopyrimidines de formule (I) suivant l'une quelconque des revendications 1 à 5 ou leurs sels d'addition d'acides sur les micro-organismes indésirables et/ou sur leur milieu.

10. Procédé de préparation de compositions destinées à combattre des micro-organismes indésirables, **caractérisé en ce qu'**on mélange des triazolopyrimidines de formule (I) suivant l'une quelconque des revendications 1 à 5 ou leurs sels d'addition d'acides avec des diluants et/ou des agents tensioactifs.

11. Dihalogéno-triazolopyrimidines de formule 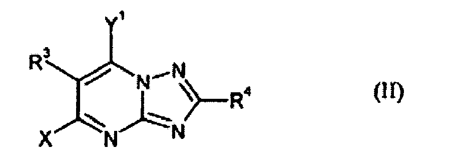 dans laquelle
R³, R⁴ et X ont les définitions indiquées ci-dessus et
Y¹ représente un halogène

12. Procédé de production de dihalogéno-triazolopyrimidines de formule (II) suivant la revendication 11,
**caractérisé en que** :
(c) on fait réagir des dihydroxy-triazolopyrimidines de formule 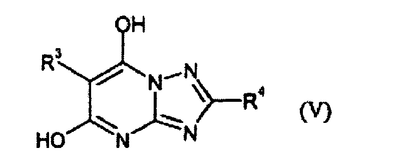 dans laquelle R³ et R⁴ ont les définitions indiquées ci-dessus,
avec des agents d'halogénation, éventuellement en présence d'un diluant.

13. Dihydroxy-triazolopyrimidines de formule 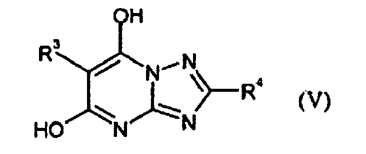 dans laquelle R³ et R⁴ ont les définitions indiquées ci-dessus.

14. Procédé de production de dihydroxy-triazolopyrimidines de formule (V) suivant la revendication 13,
**caractérisé en que** :
(d) on fait réagir des esters arylmaloniques de formule 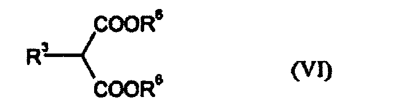 dans laquelle
R³ a les définitions indiquées ci-dessus et
R⁶ est un reste alkyle ayant 1 à 4 atomes de carbone, avec des aminotriazoles de formule 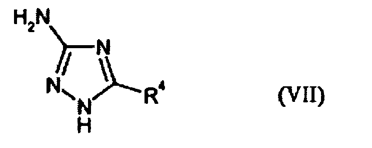 dans laquelle
R⁴ a les définitions indiquées ci-dessus,
éventuellement en présence d'un diluant et, le cas échéant, en présence d'un accepteur d'acide.

15. Amines de formule (IIIa) 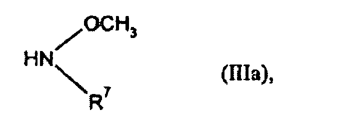 dans laquelle
R⁷ est un reste isobutyle, 2-méthoxyéthyle ou

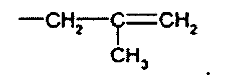

16. Procédé de production d'amines de formule (IIIa) suivant la revendication 15, **caractérisé en que** :
(e) on fait réagir dans une première étape des esters éthyliques d'acide N-méthoxycarbamique de formule (VIII) 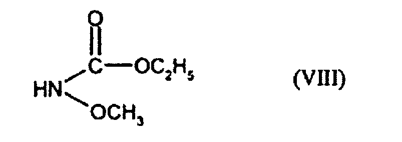 avec des composés halogénés de formule (IX),
R⁷-X¹ (IX)
dans laquelle
R⁷ a les définitions indiquées ci-dessus et
X¹ représente le brome ou l'iode,
en présence d'une base et en présence d'un diluant et on fait réagir dans une seconde étape les carbamates obtenus de formule (X), 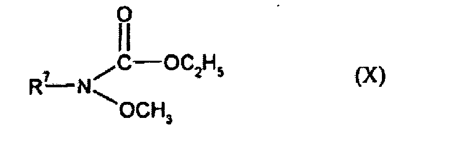 dans laquelle
R⁷ a les définitions indiquées ci-dessus
avec l'hydroxyde de potassium en présence d'éthanol et d'eau.

17. Amines de formule (IIIb) 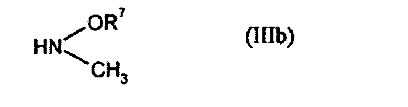 dans laquelle
R⁷ représente un reste isobutyle, 2-méthoxyéthyle ou un groupe

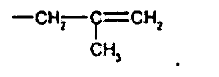

18. Procédé de production d'amines de formule (IIIb) suivant la revendication 17, **caractérisé en que** :
(f) on fait réagir dans une première étape des esters éthyliques d'acide N-hydroxy-N-méthyl-carbamique de formule (XI) 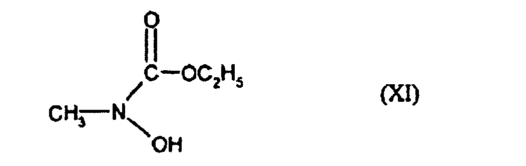 avec des composés halogénés de formule (IX),
R⁷-X¹ (IX)
dans laquelle
R⁷ et X¹ ont les définitions indiquées ci-dessus
en présence d'une base et en présence d'un diluant et on fait réagir dans une seconde étape les carbamates produits de formule (XII), 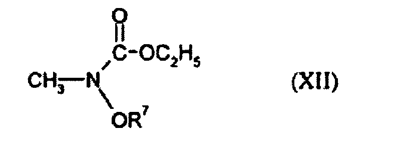 dans laquelle
R⁷ a les définitions indiquées ci-dessus
avec l'hydroxyde de potassium en présence d'éthanol et d'eau.

19. Trifluorisopropylamines de formule (IIIc) 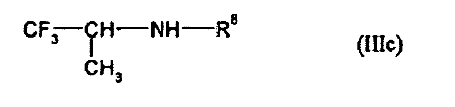 dans laquelle
R⁸ est un reste méthyle, éthyle ou propyle.

20. Procédé de production de trifluorisopropylamines de formule (IIIc) suivant la revendication 19, **caractérisé en ce que**
(g) on fait réagir dans une première étape des esters éthyliques d'acide N-trifluorisopropyl-carbamique de formule (XIII) 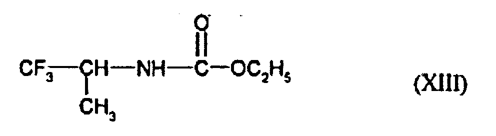 avec des composés halogénés de formule (XIV)
R⁸-X¹ (XIV)
dans laquelle
R⁸ et X¹ ont les définitions indiquées ci-dessus, en présence d'une base et en présence d'un diluant et on fait réagir dans une seconde étape les carbamates produits de formule (XV) 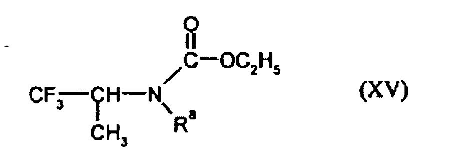 dans laquelle
R⁸ a les définitions indiquées ci-dessus,
avec l'hydroxyde de potassium en présence d'éthanol et d'eau.

21. Le 3-trifluorométhyl-3-aminopropène de formule (III-4)

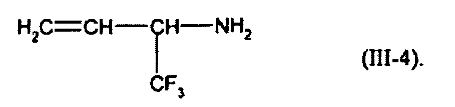

22. Procédé de production du 3-trifluorométhyl-3-aminopropène de formule (III-4) suivant la revendication 21, **caractérisé en ce que**
(h) on fait réagir le carbamate de formule (XVI) 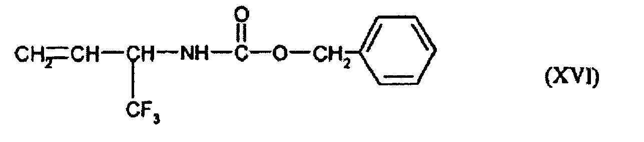 avec de l'acide chlorhydrique aqueux.

23. Carbamates de formule (X), 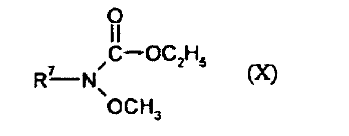 dans laquelle
R⁷ est un reste isobutyle, 2-méthoxyéthyle ou un groupe

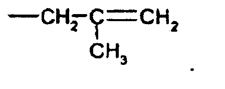

24. Carbamates de formule (XII), 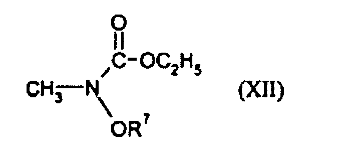 dans laquelle
R⁷ est un reste isobutyle, 2-méthoxyéthyle ou un groupe

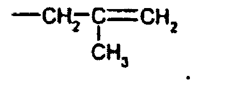

25. Carbamates de formule (XIII), 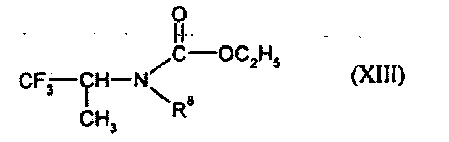 dans laquelle
R⁸ est un reste méthyle, éthyle ou propyle.
